(19)

Europäisches
Patentamt
European
Patent Office
Office européen
des brevets

(11) **EP 2 314 318 A1**

(12) **EUROPEAN PATENT APPLICATION**

(43) Date of publication:
**27.04.2011 Bulletin 2011/17**

(21) Application number: **10182916.6**

(22) Date of filing: **31.01.2002**

(51) Int Cl.:
*A61K 45/00* (2006.01)    *A61K 45/06* (2006.01)
*A61K 39/395* (2006.01)    *A61P 35/00* (2006.01)
*A61P 35/02* (2006.01)

(84) Designated Contracting States:
**AT BE CH CY DE DK ES FI FR GB GR IE IT LI LU
MC NL PT SE TR**

(30) Priority: **31.01.2001 US 772938
16.05.2001 US 855717
05.11.2001 US 985646
09.11.2001 US 331187 P**

(62) Document number(s) of the earlier application(s) in
accordance with Art. 76 EPC:
**02709219.6 / 1 372 724**

(71) Applicant: **Biogen Idec Inc.
Cambridge MA 02142 (US)**

(72) Inventors:
• **Hariharan, Kandasamy
San Diego, CA 92129 (US)**

• **Hanna, Nabil
Rancho Sante Fe, CA 92067 (US)**

(74) Representative: **Adams, Harvey Vaughan John et
al
Mathys & Squire LLP
120 Holborn
London
EC1N 2SQ (GB)**

Remarks:
•Claims filed after the date of receipt of the divisional
application (Rule 68(4) EPC).
•This application was filed on 30-09-2010 as a
divisional application to the application mentioned
under INID code 62.

(54) **CD80 antibody for use in combination with chemotherapeutics to treat B cell malignancies**

(57)    A combination antibody therapy for treating B
cell malignancies using an immunoregulatory antibody,
especially an anti-B7, anti-CD23, or anti-CD40L antibody
and a B cell depleting antibody, especially anti-CD19,
anti-CD20, anti-CD22 or anti-CD37 antibody is provided.
Preferably, the combination therapy will comprise anti-
B7 and anti-CD20 antibody administration.

EP 2 314 318 A1

**Description**

**Cross Reference to Related Applications**

This application claims priority from US Serial No. 09/772,938 filed January 31,2001, US Serial No. 09/855,717 filed May 16,2001, US Serial No. 09/985,646 filed November 5, 2001 and U.S. Provisional Application No. 60/331,187 filed November 9, 2001 each of which is incorporated in its entirety herein by reference.

**Field of the Invention**

The invention relates to a synergistic combination antibody therapy for treatment of neoplasms, especially B cell lymphomas and leukemias. In preferred embodiments this synergistic antibody combination comprises an antibody that modulates or regulates the immune system, e.g., by modulating B cell/T cell interactions and/or B cell activity, differentiation or proliferation (e.g., anti-B7, anti-CD40, anti-CD23 or anti-CD40L ) and, optionally, at least one antibody having substantial B cell depleting activity (e.g., an anti-CD19, CD20, CD22 or CD37 antibody) and In other preferred embodiments the invention may comprise synergistic combinations of two immunomodulating antibodies such as anti-CD40L and anti-B7.

**Background of Invention**

The immune system of vertebrates (for example, primates, which include humans, apes, monkeys, etc.) consists of a number of organs and cell types which have evolved to: accurately and specifically recognize foreign microorganisms ("antigen") which invade the vertebrate-host; specifically bind to such foreign microorganisms; and, eliminate/destroy such foreign microorganisms. Lymphocytes, as well as other types of cells, are critical to the immune system and to the elimination and destruction of foreign microorganisms. Lymphocytes are produced in the thymus, spleen and bone marrow (adult) and represent about 30% of the total white blood cells present in the circulatory system of humans (adult). There are two major sub-populations of lymphocytes: T cells and B cells. T cells are responsible for cell mediated immunity, while B cells are responsible for antibody production (humoral immunity). However, T cells and B cells can be considered interdependent -- in a typical immune response, T cells are activated when the T cell receptor binds to fragments of an antigen that are bound to major histocompatability complex ("MHC") glycoproteins on the surface of an antigen presenting cell; such activation causes release of biological mediators ("interleukins" or "cytokines") which, in essence, stimulate B cells to differentiate and produce antibody ("immunoglobulins") against the antigen.

Each B cell within the host expresses a different antibody on its surface-- thus one B cell will express antibody specific for one antigen, while another B cell will express antibody specific for a different antigen. Accordingly, B cells are quite diverse, and this diversity is critical to the immune system. In humans, each B cell can produce an enormous number of antibody molecules (i.e., about $10^7$ to $10^8$). Such antibody production most typically ceases (or substantially decreases) when the foreign antigen has been neutralized. Occasionally, however, proliferation of a particular B cell will continue unabated; such proliferation can result in a cancer referred to as "B cell lymphoma."

Non-Hodgkin's lymphoma is one type of lymphoma that is characterized by the malignant growth of B lymphocytes. According to the American Cancer Society, an estimated 54,000 new cases will be diagnosed, 65% of which will be classified as intermediate- or high-grade lymphoma. Patients diagnosed with intermediate-grade lymphoma have an average survival rate of two to five years, and patients diagnosed with high-grade lymphoma survive an average of six months to two years after diagnosis.

Conventional therapies have included chemotherapy and radiation, possibly accompanied by either autologous or allogeneic bone marrow or stem cell transplantation if a suitable donor is available, and if the bone marrow contains too many tumor cells upon harvesting. While patients often respond to conventional therapies, they usually relapse within several months.

It is known that B cell malignancies, e.g., B cell lymphomas and leukemias may be successfully treated using antibodies specific to B cell antigens that possess B cell depleting activity. Examples of B cell antibodies that have been reported to possess actual or potential application for the treatment of B cell malignancies include antibodies specific to CD20, CD19, CD22, CD37 and CD40.

Also, the use anti-CD37 antibodies having B cell depleting activity have been well reported to possess potential for treatment of B cell lymphoma. See e.g., Presr et al., J. Clin. Oncol. 7(8): 1027-1038 (August 1989); Grossbard et al., Blood 8(4): 863-876 (August 15,1992).

CD20 is a cell surface antigen expressed on more than 90% of B-cell lymphomas, which does not shed or modulate in the neoplastic cells (McLaughlin et al., J. Clan. Oncol. 16: 2825-2833 (1998b)). The CD20 antigen is a non-glycosylated, 35 kDa B-cell membrane protein involved in intracellular signaling, B-cell differentiation and calcium channel mobilization (Clark et al., Adv. Cancer Res. 52: 81-149 (1989); Tedder et al., Immunology Today 15: 450-454 (1994)).

The antigen appears as an early marker of the human B-cell lineage, and is ubiquitously expressed at various antigen densities on both normal and malignant B-cell populations. However, the antigen is absent on fully, mature B-cells (e.g., plasma cells), early B-cell populations and stem cells, making it a suitable target for antibody mediated therapy.

Anti-CD20 antibodies have been prepared for use both in research and therapeutics. One anti-CD20 antibody is the monoclonal B1 antibody (U.S. Patent No. 5,843,398). Anti-CD20 antibodies have also been prepared in the form of radionuclides for treating B-cell lymphoma (e.g., $^{131}$I-labeled anti-CD20 antibody), as well as a $^{89}$Sr-labeled form for the palliation of bone pain caused by prostate and breast cancer metastasises (Endo, Gan To Kagaku Ryoho 26: 744-748 (1999)).

A murine monoclonal antibody, 1F5, (an anti-CD20 antibody) was reportedly administered by continuous intravenous infusion to B-cell lymphoma patients. However, extremely high levels 2 grams) of 1F5 were reportedly required to deplete circulating tumor cells, and the results were described as "transient" (Press et al., Blood 69: 584-591(1987)). A potential problem with using monoclonal antibodies in therapeutics is non-human monoclonal antibodies (e.g., murine monoclonal antibodies) typically lack human effector functionality, e.g., they are unable to, inter alia, mediate complement dependent lysis or lyse human target cells through antibody-dependent cellular toxicity or Fc-receptor mediated phagocytosis. Furthermore, non-human monoclonal antibodies can be recognized by the human host as a foreign protein; therefore, repeated injections of such foreign antibodies can lead to the induction of immune responses leading to harmful hypersensitivity reactions. For murine-based monoclonal antibodies, this is often referred to as a Human Anti-Mouse Antibody response, or "HAMA" response. Additionally, these "foreign" antibodies can be attacked by the immune system of the host such that they are, in effect, neutralized before they reach their target site.

RITUXAN® (also known as Rituximab, MabThera®, IDEC-C2B8 and C2B8) was the first FDA-approved monoclonal antibody and was developed at IDEC Pharmaceuticals (see U.S. Patent Nos. 5,843,439; 5,776,456 and 5,736,137) for treatment of human B-cell lymphoma (Reff et al., Blood 83: 435-445 (1994)). RITUXAN® is a chimeric, anti-CD20 monoclonal (MAb) which is growth inhibitory and reportedly sensitizes certain lymphoma cell lines for apoptosis by chemotherapeutic agents in vitro (Demidem et al., Cancer Biotherapy & Radiopharmaceuticals 12: 177- (1997)). RITUXAN® also demonstrates anti-tumor activity when tested in vivo using murine xenograft animal models. RITUXAN® efficiently binds human complement, has strong FcR binding, and can efficiently kill human lymphocytes in vitro via both complement dependent (CDC) and antibody-dependent (ADCC) mechanisms (Reff et al., Blood 83: 435-445 (1994)). In macaques, the antibody selectively depletes normal B-cells from blood and lymph nodes.

RITUXAN® has been recommended for treatment of patients with low-grade or follicular B-cell non-Hodgkin's lymphoma (McLaughlin et al., Oncology (Huntingt) 12: 1763-1777 (1998a); Maloney et al., Oncology 12: 63-76 (1998); Leget et al., Curr. Opin. Oncol. 10:548-551(1998)). In Europe, RITUXAN® has been approved for therapy of relapsed stage III/IV follicular lymphoma (White et al., Pharm. Sci. Technol. Today 2: 95-101 (1999)) and is reportedly effective against follicular center cell lymphoma (FCC) (Nguyen et al., Eur. J. Haematol 62: 76-82 (1999)). Other disorders treated with RITUXAN® include follicular centre cell lymphoma (FCC), mantle cell lymphoma (MCL), diffuse large cell lymphoma (DLCL), and small lymphocytic lymphoma/chronic lymphocytic leukemia (SLL/CLL) (Nguyen et al., 1999)). Patients with refractory or incurable NHL reportedly have responded to a combination of RITUXAN® and CHOP (e.g., cyclophosphamide, vincristine, prednisone and doxorubicin) therapies (Ohnishi et al., Gan To Kagaku Ryoho 25: 2223-8 (1998)). RITUXAN® has exhibited minimal toxicity and significant therapeutic activity in low-grade non-Hodgkin's lymphomas (NHL) in phase I and II clinical studies (Berinstein et al., Ann. Oncol. 9: 995-1001 (1998)).

RITUXAN®, which was used alone to treat B-cell NHL at weekly doses of typically 375 mg/M$^2$ for four weeks with relapsed or refractory low-grade or follicular NHL, was well tolerated and had significant clinical activity (Piro et al., Ann. Oncol. 10: 655-61 (1999); Nguyen et al., (1999); and Coiffier et al., Blood 92: 1927-1932 (1998)). However, up to 500 mg/M$^2$ of four weekly doses have also been administered during trials using the antibody (Maloney et al., Blood 90: 2188-2195 (1997)). RITUXAN® also has been combined with chemotherapeutics, such as CHOP (e.g., cyclophosphamide, doxorubicin, vincristine and prednisone), to treat patients with low-grade or follicular B-cell non-Hodgkin's lymphoma (Czuczman et al., J. Clin. Oncol. 17: 268-76 (1999); and McLaughlin et al., (1998a)).

Still further, the use of anti-B7 antibodies for treatment of B cell lymphoma was mentioned in a patent assigned to IDEC Pharmaceuticals Corporation (US Patent No. 6,113,198). However, the focus of the patent was the use thereof for treating diseases which immunosuppression is therapeutically beneficial. Examples included allergic, autoimmune and transplant indications.

CD40 is expressed on the cell surface of mature B-cells, as well as on leukemic and lymphocytic B-cells, and on Hodgkin's and Reed-Sternberg (RS) cells of Hodgkin's Disease (HD) (Valle et al., Eur. J.Immunol. 19: 1463-1467 (1989); and Gruss et al., Leuk. Lymphoma 24: 393-422 (1997)). CD40 is a B-cell receptor leading to activation and survival of normal and malignant B-cells, such as non-Hodgkin's follicular lymphoma (Johnson et al., Blood 82: 1848-1857 (1993); and Metkar et al., Cancer Immunol. Immunother. 47: 104 (1998)). Signaling through the CD40 receptor protects immature B-cells and B-cell lymphomas from IgM- or Fas-induced apoptosis (Wang et al., J. Immunology 155: 3722-3725 (1995)). Similarly, mantel cell lymphoma cells have a high level of CD40, and the addition of exogenous CD40L enhanced their survival and rescued them from fludarabin-induced apoptosis (Clodi et al., Brit. J. Haematol. 103: 217-219 (1998)).

In contrast, others have reported that CD40 stimulation may inhibit neoplastic B-cell growth both *in vitro* (Funakoshi et αl., Blood 83: 2787-2794 (1994)) and *in vivo* (Murphy et αl., Blood 86:1946-1953 (1995)).

Anti-CD40 antibodies (see U.S. Patent Nos. 5,874,082 and 5,667,165) administered to mice increased the survival of mice with human B-cell lymphomas (Funakoshi *et αl.,* (1994); and Tutt et αl., J. Immunol. 161: 3176-3185 (1998)). Methods of treating neoplasms, including B-cell lymphomas and EBV-induced lymphomas using anti-CD40 antibodies mimicking the effect of CD40L and thereby delivering a death signal, are described in U.S. Patent No. 5,674,492 (1997), which is herein incorporated by reference in its entirety. CD40 signaling has also been associated with a synergistic interaction with CD20 (Ledbetter et al., Circ. Shock 44: 67-72 (1994)). Additional references describing preparation and use of anti-CD40 antibodies include U.S. Patent Nos. 5,874,085 (1999), 5,874,082 (1999), 5,801,227 (1998), 5,674,492 (1997) and 5,667,165 (1997), which are incorporated herein by reference in their entirety.

A CD40 ligand, gp39 (also called CD40 ligand, CD40L or CD154), is expressed on activated, but not resting, CD4[+] Th cells (Spiggs et al., Exp. Med 176: 1543-1550 (1992); Lane el αl., Eur. J. Immunol. 22: 2573-2578 (1992); and Roy et al., J. Immunol. 151: 1-14 (1993)). Both CD40 and CD40L have been cloned and characterized (Stamenkovi et αl., EMBO J. 8: 1403-1410 (1989); Armitage et αl., Nature 357: 80-82 (1992); Lederman et αl., J. Exp. Med 175: 1091-1101 (1992); and Hollenbaugh et αl., EMBO J. 11: 4313-4321 (1992)). Human CD40L is also described in U.S. Patent No. 5,945,513. Cells transfected with the CD40L gene and expressing the CD40L protein on their surface can trigger B-cell proliferation, and together with other stimulatory signals, can induce antibody production (Armitage *et al.,* (1992); and U.S. Patent No. 5,945,513). CD40L may play an important role in the cell contact-dependent interaction of tumor B-cells (CD40[+]) within the neoplastic follicles or Reed-Sternberg cells (CD40[+]) in Hodgkin's Disease areas (Carbone et αl., Am. J. Pathol. 147: 912-922 (1995)). Anti-CD40L monoclonal antibodies reportedly have been effectively used to inhibit the induction of murine AIDS (MAIDS) in LP-BM5-infected mice (Green et αl., Virology 241: 260-268 (1998)). However, the mechanism of CD40L-CD40 signaling leading to survival versus cell death responses of malignant B-cells is unclear. For example, in follicular lymphoma cells, down-regulation of a apoptosis inducing TRAIL molecule (APO-2L) (Ribeiro et al., British J. Hαemαtol. 103: 684-689 (1998)) and over expression of BCL-2, and in the case of B-CLL, down-regulation of CD95 (Fas/APO-1) (Laytragoon-Lewin et αl., Eur. J Haematol. 61: 266-271 (1998)) have been proposed as mechanisms of survival. In contrast, evidence exists in follicular lymphoma, that CD40 activation leads to up-regulation ofTNF (Worin et al., Internαtionαl Immunol. 6:1883-1890 (1994)) CD95 molecules (Plumas et al., Blood 91:2875-2885(1998)).

Anti-CD40 antibodies have also been prepared to prevent or treat antibody-mediated diseases, such as allergies and autoimmune disorders as described in U.S. Patent No. 5,874,082 (1999). Anti-CD40 antibodies reportedly have been effectively combined with anti-CD20 antibodies yielding an additive effect in inhibiting growth of non-Hodgkin's B-cell lymphomas in cell culture (Benoit et αl., Immunophαrmαcology 35: 129-139 (1996)). *In vivo* studies in mice purportedly demonstrated that anti-CD20 antibodies were more efficacious than anti-CD40 antibodies administered individually in promoting the survival of mice bearing some, but not all, lymphoma lines (Funakoshi et αl., J.Immunother. Emphαsis Tumor Immunol. 19: 93-101 (1996)). Anti-CD19 antibodies are reportedly also effective *in vivo* in the treatment of two syngeneic mouse B-cell lymphomas, BCL1 and A31 (Tutt *et al.* (1998)). Antibodies to CD40L have also been described for use to treat disorders associated with B-cell activation (European Patent No. 555,880 (1993)). Anti-CD40L antibodies include monoclonal antibodies 3E4, 2H5, 2H8, 4D9-8, 4D9-9, 24-31, 24-43, 89-76 and 89-79, as described in U.S. Patent No. 5,7474,037 (1998), and anti-CD40L antibodies described in U.S. Patent No. 5,876,718 (1999) used to treat graft-versus-host-disease.

The synthesis of monoclonal antibodies against CD22 and their use in therapeutic regimens has also been reported. CD22 is a B-cell-specific molecule involved in B-cell adhesion that may function in homotypic or heterotypic interactions (Stamenkovic al, Nαture 344:74 (1990); Wilson et al, J. Exp. Med. 173:137 (1991); Stamenkovic al, Cell 66:1133 (1991)). The CD22 protein is expressed in the cytoplasm of progenitor B and pre-B-cells (Dorken et al, J. Immunol. 136:4470 (1986); Dorken et al, "Expression of cytoplasmic CD22 in B-cell ontogeny. In Leukocyte Typing III, White Cell Differentiation Antigens. McMichael et al, eds., Oxford University Press, Oxford, p. 474 (1987); Schwarting et al, Blood 65:974 (1985); Mason et al, Blood 69:836 (1987)), but is found only on the surface of mature B-cells, being present at the same time as surface IgD (Dorken et al, J. Immunol. 136:4470 (1986)). CD22 expression increases following activation and disappears with further differentiation (Wilson et al, J. Exp. Med. 173:137 (1991); Dorken et al, J. Immunol. 136:4470 (1986)). In lymphoid tissues, CD22 is expressed by follicular mantle and marginal zone B-cells but only weakly by germinal center B-cells (Dorken et al, J. Immunol. 136:4470 (1986); Ling et al, "B-cell and plasma antigens: new and previously defined clusters" In Leukocyte Typing III. White Cell Differentiation Antigens, McMichael et al, eds., Oxford University Press, Oxford, p. 302 (1987)). However, in situ hybridization reveals the strongest expression of CD22 mRNA within the germinal center and weaker expression within the mantle zone (Wilson et al, J. Exp. Med 173:137 (1991)). CD22 is speculated to be involved in the regulation of B-cell activation since the binding of CD22 mAb to B-cells *in vitro* has been found to augment both the increase in intracellular free calcium and the proliferation induced after cross-linking of surface Ig (Pezzutto et al, J. Immunol. 138:98 (1987); Pezzutto et al, J. Immunol 140:1791 (1988)). Other studies have determined, however, that the augmentation of anti-Ig induced proliferation is modest (Dorken et al, J. Immunol. 136:4470 (1986)). CD22 is constitutively phosphorylated, but the level of phosphorylation is augmented after treatment of cells with PMA (Boue et al, J. Immunol. 140:192(1988)). Furthermore, a soluble form of CD22 inhibits the CD3-mediated activation of human T-cells, suggesting CD22 may be important in T-cell - B-cell interactions (Stamenkovic et al, Cell 66:1133 (1991)).

Ligands that specifically bind the CD22 receptor have been reported to have potential application in the treatment of various diseases, especially B-cell lymphomas and autoimmune diseases. In particular, the use of labeled and non-labeled anti-CD22 antibodies for treatment of such diseases has been reported.

For example, Tedder et al, U.S. patent 5,484,892, that purportedly bind CD22 with high affinity and block the interaction of CD22 with other ligands. These monoclonal antibodies are disclosed to be useful in treating autoimmune diseases such as glomerulonephritis, Goodpasture's syndrome, necrotizing vasculitis, lymphadenitis, periarteritis nodosa, systemic lupus erythematosis, arthritis, thrombocytopenia purpura, agranulocytosis, autoimmune hemolytic anemias, and for inhibiting immune reactions against foreign antigens such as fetal antigens during pregnancy, myasthenia gravis, insulin-resistant diabetes, Graves' disease and allergic responses.

Also, Leung et al, U. S. Patent 5,789,557, disclose chimeric and humanized anti-CD22 monoclonal antibodies produced by CDR grafting and the use thereof in conjugated and unconjugated form for therapy and diagnosis of B-cell lymphomas and leukemias. The reference discloses especially such antibodies conjugated to cytotoxic agents, such as chemotherapeutic drugs, toxins, heavy metals and radionuclides. (See U.S. Patent 5,789,554, issued August 4, 1998, to Leung et al, and assigned to Immunomedics.)

Further, PCT applications WO 98/42378, WO 00/20864, and WO 98/41641 describe monoclonal antibodies, conjugates and fragments specific to CD22 and therapeutic use thereof, especially for treating B-cell related diseases.

Also, the use of anti-CD22 antibodies for treatment of autoimmune diseases and cancer has been suggested. *See,* e.g., U.S. Patent 5,443,953, issued August 22,1995 to Hansen et al and assigned to Immunomedics Inc. that purports to describe anti-CD22 immunoconjugates for diagnosis and therapy, especially for treatment of viral and bacterial infectious diseases, cardiovascular disease, autoimmune diseases, and cancer, and U.S. Patent 5,484,892, issued January 16, 1998 to Tedder et al and assigned to Dana-Farber Cancer institute, Inc. that purports to describe various monoclonal antibodies directed against CD22, for treatment of diseases wherein retardation or blocking of CD22 adhesive function is therapeutically beneficial, particularly autoimmune diseases.) These references suggest that an anti-CD22 antibody of fragment may be directly or indirectly conjugated to a desired effector moiety, e.g., a label that may be detected, such as an enzyme, fluorophore, radionuclide, electron transfer agent during an in *vitro* immunoassay or *in vivo* imaging, or a therapeutic effector moiety, e.g., a toxin, drug or radioisotope.

Further, an anti-human CD22 monoclonal antibody of the IgG1 isotype is commercially available from Leinco Technologies, and reportedly is useful for treatment of B-cell lymphomas and leukemias, including hairy cell leukemia. (Campana, D. et al, J. Immunol. 134:1524 (1985)). Still further, Dorken et al, J. Immunol. 150:4719 (1993) and Engel et al, J. Immunol. 150:4519 (1993) both describe monoclonal antibodies specific to CD22.

Also, the use of anti-CD 19 antibodies and fragments thereof for treating lymphoma has been reported in the literature. For example, U. S. Patent 5,686,072, issued November 11,1997, to Uhr et al, and assigned to the University of Texas, discloses the use of anti-CD 19 and anti-CD22 antibodies and immunotoxins for treatment of leukemia lymphomas. This patent is incorporated by reference in its entirety herein.

Further, the use of anti-CD 19 antibodies for classifying the status and prognosis of leukemias has been reported.

Thus, based on the foregoing, it is clear that numerous individual antibodies have been reported to possess therapeutic potential for the treatment of neoplastic disorders. Notwithstanding this fact, it is an object of the present invention to provide novel antibody regimens for treatment of various malignancies including lymphomas and leukemias.

## Brief Description and Objects of the Invention

Toward that end, it is an object of the invention to provide a novel improved antibody therapy for treatment of various neoplastic disorders including B cell malignancies such as Hodgkin's and non-Hodgkin's lymphoma of any grade.

More specifically, it is an object of the invention to provide a novel antibody regimen for treatment of a neoplastic disorder involving the administration of at least one immunoregulatory or immunomodulatory antibody and, optionally, at least one B cell depleting antibody.

Even more specifically, it is an object of the invention to provide a novel antibody therapy for treatment of neoplastic disorders that involves the administration of at least one at least one immunomodulatory or immunoregulatory antibody preferably selected from the group consisting of anti-B7 antibodies, anti-CD23 antibodies, anti-CD40 antibodies, anti-CD40L antibodies and anti-CD4 antibodies and, optionally, at least one B cell depleting antibody preferably selected from the group consisting of anti-CD20 antibodies, anti-CD19 antibodies, anti-CD22 antibodies and anti-CD37 antibodies. In a particularly preferred embodiment the treatment or therapy will comprise the administration of a therapeutically effective amount of an anti-B7 antibody in combination with the administration of a therapeutically effective amount of an anti-CD20 antibody.

In other embodiments it is a particular object of the present invention to provide a treatment or prophylaxis for a neoplastic disorder comprising administering a therapeutically effective amount of an antibody to CD40L in combination with a therapeutically effective amount of an antibody to B7. Preferably this antibody combination will be administered for treatment of a B cell malignancy such as non-Hodgkin's lymphoma or chronic lymphocyte leukemia (CLL) and even more preferably will comprise those B7 antibodies disclosed in US Patent 6,113,898 and those anti-CD40L antibodies disclosed in US Patent 6,001,358.

Accordingly, an important aspect of the present invention encompasses a method of treating a neoplastic disorder in a mammal comprising the steps of:

administering a therapeutically effective amount of a first immunoregulatory antibody to said mammal; and administering a therapeutically effective amount of a second immunoregulatory antibody or a B cell depleting antibody to said mammal wherein said first and second immunoregulatory antibodies bind to different antigens and the first immunoreglatory antibody and the second immunoregulatory antibody or B cell depleting antibody may be administered in any order or concurrently.

It is another object of the invention to provide novel compositions, articles of manufacture and/or kits for treatment of neoplastic disorders including B cell malignancies, in B cell lymphomas and leukemias, wherein the kits or articles of manufacture include at least one immunoregulatory or immunomodulatory antibody and, optionally, at least one B cell depleting antibody. Preferably, the immunoregulatory or immunomodulatory antibody will comprise at least one anti-CD23 antibody, anti-CD40 antibody, anti-CD40L antibody or anti-B7 antibody and the B cell depleting antibody will be specific to CD20, CD19, CD22 or CD37. Most preferably, the kit or article of manufacture will comprise an anti-CD40L or anti-B7 antibody or combination thereof and, optionally, an anti-CD20 antibody. Additionally the article of manufacture will include an insert, instructions or labeled containers indicating that the contents thereof are useful in the treatment of a neoplastic disorder.

Another object of the invention is to provide a combination therapy for the treatment of a B-cell lymphoma or a B-cell leukemia comprising an anti-CD40L antibody or antibody fragment or CD40L antagonist and at least one of the following (a) a chemotherapeutic agent or a combination of chemotherapeutic agents, (b) radiotherapy, (c) an anti-CD20 antibody or fragment thereof, (d) an anti-CD40 antibody or fragment thereof, (e) an anti-CD 19 antibody or fragment thereof; (f) an anti-CD22 antibody or fragment thereof, (g) cytokines (h) an anti-B7 antibody or fragment thereof, where antibodies may be conjugated with a toxin or a radiolabel, or may be engineered with human constant regions as to elicit human antibody effector mechanisms, i.e. resulting in apoptosis or death of targeted cells.

Other objects, features and advantages of the present invention will be apparent to those skilled in the art from a consideration of the following detailed description of preferred exemplary embodiments thereof.

## Brief Description of the Figures

Fig. 1. Sensitivity-of B-lymphoma cells to adriamycin after 4 hour exposure.

Fig. 2. (Panel A) Anti CD40L (IDEC-131) overrides CD40L mediated resistance to killing by ADM of B-lymphoma cells. (Panel B) Effect of RITUXAN® on normal and sCD40L pre-treated DHL-4 cells.

Fig. 3. (Panel A) Blocking of CD40L mediated cell survival ofB-CLL by anti-CD40L antibody (IDEC-131). (Panel B) Blocking of CD40L mediated survival of B-CLL by Rituxan®.

Fig. 4. FACS analysis comprising HLA-DR expression in CD19[+] CLL cells cultured with sCD40L and not cultured with sCD40L.

Fig. 5 is a graphical representation showing the binding activity of two different lots of IDEC-114 to membrane bound CD80 cells were determined by flow cytometry using CHO cells expressing the CD80 molecule.

Fig. 6 is a graphical representation showing the ADCC activity of IDEC-114 and rituximab on SB or SKW cells.

Fig. 7 is a graphical representation showing the ADCC activity of IDEC-114, rituximab and a combination thereof on activated host cells obtained from two donors: A and B.

Fig. 8A is a graphical representation showing the CDC activity of IDEC-114 on CD80-expressing CHO cells.

Fig. 8B is a graphical representation showing the CDC activity of IDEC-114 and rituximab on CD80-expressing SKW cells.

Fig. 8C is a graphical representation showing the CDC activity of IDEC-114 and rituximab on CD80-expressing Daudi cells.

Fig. 9A is a graphical representation showing the antitumor response of SKW/SCID mice to IDEC-114.

Fig. 9B is a graphical representation showing the antitumor response of SKW/SCID mice to rituximab.

Fig. 10 is a graphical representation showing the antitumor response of SKW/SCID mice to IDEC-114 in combination with rituximab.

## Detailed Description of the Invention

While the present invention may be embodied in many different forms, disclosed herein are specific illustrative embodiments thereof that exemplify the principles of the invention. It should be emphasized that the present invention is not limited to the specific embodiments illustrated.

The present invention provides novel antibody regimens that involve the administration of at least one immunoregulatory or immunomodulatory antibody (the terms may be used interchangeably for the purposes of the instant disclosure), e.g., an anti-B7 antibody, anti-CD23 antibody, anti-CD40 antibody or anti-CD40L antibody and, optionally, at least one B cell depleting antibody, e.g., an anti-CD20, anti-CD 19, anti-CD22 or anti-CD37 antibody having substantial B cell depleting activity.

Such combinations will afford synergistic results based on the different mechanisms by which the antibodies elicit a therapeutic benefit. In particular, it is theorized that the complementary mechanisms of action will yield a more durable and potent clinical response as two or more immunoregulatory antibodies or an immunoregulatory antibody and a B cell depleting antibody can attack any neoplastic cells in concert. For example, in some embodiments the B cell depleting antibody will deplete activated B cells which may be resistant to the action of immunoregulatory or immunomodulatory antibodies such as anti-B7 or anti-CD40L antibodies. Such activated B cells can otherwise serve as effective antigen presenting cells for T cells as well as antibody producing cells. In the context of B cell malignancies, such activated B cells may include malignant cells which unless eradicated by give rise to new cancer cells and tumors.

Accordingly, one preferred embodiment of the present invention comprises a method for treating a patient suffering from a neoplastic disorder comprising administering therapeutically effective amounts of a combination of immunregulatory antibodies or an immunoregulatory antibody in conjunction with a B cell depleting antibody. In particularly preferred embodiments the combination of immunoregulatory antibodies will comprise an antibody or immunoreactive fragment thereof directed to CD40L and an antibody or immunoreactive fragment thereof directed to B7. Those skilled in the art will appreciate that the two immunoregulatory antibodies may be administered in any order or concomitantly and that what constitutes an therapeutically effective amount may easily be discerned using well known techniques. Moreover, it is within the purview of the instant invention to administer the combination of immunoregulatory antibodies with adjunct therapies such as B cell depleting antibodies, chemotherapy or radioimmunotherapy.

"B Cell Depleting Antibody" as used herein is an antibody or fragment that upon administration, results in demonstrable B cell depletion. Typically, such antibody will bind to a B cell antigen or B cell marker expressed on the surface of a B cell. Preferably, such antibody, after administration, typically within about several days or less, will result in a depletion of cell number by about 50% or more. In a preferred embodiment, the B cell depleting antibody will be RITUXAN® (a chimeric anti-CD20 antibody) or one having substantially the same or at least 20-50% the cell depleting activity of RITUXAN®.

A "B cell surface marker" or "B cell target" or "B cell antigen" herein is an antigen expressed on the surface of a B cell which can be targeted with an agonist or antagonist which binds thereto. Exemplary B cell surface markers include the CD 10, CD19, CD20, CD21, CD22, CD23, CD24, CD37, CD53, CD72, CD73, CD74, CDw75, CDw76, CD77, CDw78, CD79a, CD79b, CD80 (B7.1), CD8 1, CD82, CD83, CDw84, CD85 and CD86 (B7.2) leukocyte surface markers: The B cell surface marker of particular interest is preferentially expressed on B cells compared to other non-B cell tissues of a mammal and may be expressed on both precursor B cells and mature B cells. In one embodiment, the marker is one, like CD20 or CD19, which is found on B cells throughout differentiation of the lineage from the stem cell stage up to a point just prior to terminal differentiation into plasma cells. The preferred B cell surface markers herein are CD19 and CD20. The preferred B cell surface markers herein are CD 19, CD20, CD22, CD23, CD80 and CD86.

As used herein "immunoregulatory antibody" or "immunomodulatory antibody" refers to an antibody that elicits an effect on the immune system by a mechanism different from B cell depletion, e.g., by CDL and/or ADCC activity and may be an agonist. Examples of such include antibodies that inhibit T cell immunity, B cell immunity, e.g. by inducing tolerance (anti-CD40L, anti-CD40) or other immunosuppressant antibodies, e.g., those that inhibit B7 cell signaling (anti-B7.1, anti-B7.2, anti-CD4, anti-CD23, etc.). In some instances, the immunoregulatory antibody may possess the ability to potentiate apoptosis. Also, an antibody that is normally a B cell depleting antibody can be engineered to become immunoregulatory by substantiating human constant regions as to take advantage of different effector mechanisms.

Prior to discussing the invention, the following additional definitions are provided:

The term "antibody" as used herein is intended to include immunoglobulins and fragments thereof which are specifically reactive to the designated protein or peptide thereof. An antibody can include human antibodies, primatized antibodies, chimeric antibodies, bispecific antibodies, humanized antibodies, antibodies fused to other proteins or radiolabels, and antibody fragments, Moreover, the term "antibody" herein is used in the broadest sense and specifically covers intact monoclonal antibodies, polyclonal antibodies, multispecific antibodies (e.g. bispecific antibodies) formed from at least two intact antibodies, and antibody fragments so long as they exhibit the desired biological activity. Unless specifically noted otherwise or dictated by the context of use, the term "antibody" is to be construed broadly for the purposes of the instant application and claims and is explicitly held to encompass all variants, fragments or immunoreactive constructs thereof that provide the desired regulatory or depleting effects as described herein.

"Antibody fragments" comprise a portion of an intact antibody, preferably comprising the antigen-binding or variable region thereof. Examples of antibody fragments include Fab, Fab', F(ab')2, and Fv fragments; diabodies; linear antibodies; single-chain antibody molecules; domain deleted antibodies; and multispecific antibodies formed from antibody fragments. Antibody fragments may be isolated using conventional techniques. For example, $F(ab^1)_2$ fragments can be generated by treating antibodies with pepsin. The resulting $F(ab^1)_2$ fragment can be treated to reduce disulfide bridges to produce $Fay^1$ fragments.

"Native antibodies" are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies among the heavy chains of different immunoglobulin isotypes. Each heavy and light chain also has regularly spaced intrachain disulfide bridges. Each heavy chain has at one end a variable domain (VH) followed by a number of constant domains. Each light chain has a variable domain at one end (VL) and a constant domain at its other end; the constant domain of the light chain is aligned with the first constant domain of the heavy chain, and the light-chain variable domain is aligned with the variable domain of the heavy chain. Particular amino acid residues are believed to form an interface between the light chain and heavy chain variable domains.

The term "variable" refers to the fact that certain portions of the variable domains differ extensively in sequence among antibodies and are used in the binding and specificity of each particular antibody for its particular antigen. However, the variability is not evenly distributed throughout the variable domains of antibodies. It is concentrated in three segments called hypervariable regions both in the light chain and the heavy chain variable domains. The more highly conserved portions of variable domains are called the framework regions (FRs). The variable domains of native heavy and light chains each comprise four FRs, largely adopting a 13-sheet configuration, connected by three hypervariable regions, which form loops connecting, and in some cases forming part of, the B -sheet structure. The hypervariable regions in each chain are held together in close proximity by the FRs and, with the hypervariable regions from the other chain, contribute to the formation of the antigen-binding site of antibodies (see Kabat et $\alpha l$.,Sequences of Proteins of Immu-nologic$\alpha$l Interest, 5th Ed. Public Health Service, National Institutes of Health, Bethesda, MD. (1991)). The constant domains are not involved directly in binding an antibody to an antigen, but exhibit various effector functions, such as participation of the antibody in antibody dependent cellular cytotoxicity (ADCC).

Papain digestion of antibodies produces two identical antigen-binding fragments, called "Fab" fragments, each with a single antigen-binding site, and a residual "Fc" fragment, whose name reflects its ability to crystallize readily. Pepsin treatment yields an F(ab')2 fragment that has two antigen-binding sites and is still capable of cross-linking antigen.

"Fv" is the minimum antibody fragment which contains a complete antigen-recognition and antigen-binding site. This region consists of a dimer of one heavy chain and one light chain variable domain in tight, non-covalent association. It is in this configuration that the three hypervariable regions of each variable domain interact to define an antigen-binding site on the surface of the VH-VL dimer. Collectively, the six hypervariable regions confer antigen-binding specificity to the antibody. However, even a single variable domain (or half of an Fv comprising only three hypervariable regions specific for an antigen) has the ability to recognize and bind antigen, although at a lower affinity than the entire binding site.

The Fab fragment also contains the constant domain of the light chain and the first constant domain ($C_HI$) of the heavy chain. Fab' fragments differ from Fab fragments by the addition of a few residues at the carboxy terminus of the heavy chain $C_HI$ domain including one or more cysteines from the antibody hinge region. Fab'-SH is the designation herein for Fab' in which the cysteine residue(s) of the constant domains bear at least one free thiol group. F(ab')Z antibody fragments originally were produced as pairs of Fab' fragments which have hinge cysteines between them. Other chemical couplings of antibody fragments are also known.

The "light chains" of antibodies (immunoglobulins) from any vertebrate species can be assigned to one of two clearly distinct types, called kappa and lambda, based on the amino acid sequences of their constant domains.

Depending on the amino acid sequence of the constant domain of their heavy chains, antibodies can be assigned to different classes. There are five major classes of intact antibodies: IgA, IgD, IgE, IgG, and IgM, and several of these may be further divided into subclasses (isotypes), e.g., IgG1 IgG2, IgG3, IgG4, IgA, and IgA2. The heavy-chain constant domains that correspond to the different classes of antibodies are called alpha, delta, epsilon, gamma and mu, respectively. Preferably, the heavy-chain constant domains will complete the gamma-1, gamma-2, gamma-3 and gamma-4 constant region. Preferably, these constant domains will also comprise modifications to enhance antibody stability such as the P and E modification disclosed in U.S. Patent No. 6,011,138 incorporated by reference in its entirety herein. The subunit structures and three dimensional configurations of different classes of immunoglobulins are well known.

"Single-chain Fv" or "scFv" antibody fragments comprise the VH and VL domains of antibody, wherein these domains are present in a single polypeptide chain. Preferably, the Fv polypeptide further comprises a polypeptide linker between the VH and VL domains which enables the scFv to form the desired structure for antigen binding. For a review of scfv see Pluckthun in The Pharmacology of Monoclonal Antibodies, vol. 113, Rosenburg and Moore eds., Springer-Verlag, New York, pp. 269-315(1994).

The term "diabodies" refers to small antibody fragments with two antigen-binding sites, which fragments comprise a heavy-chain variable domain (VH) connected to a light chain variable domain (VL) in the same polypeptide chain (VH - VL). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al., Proc. Natl. Acad Sci. USA, 90:6444-6448 (1993).

The term "monoclonal antibody" as used herein refers to an antibody obtained from a population of substantially homogeneous antibodies, *i.e.,* the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the antigen. In addition to their specificity, the monoclonal antibodies are advantageous in that they are synthesized by the hybridoma culture, uncontaminated by other immunoglobulins.

By "humanized antibody" is meant an antibody derived from a non-human antibody, typically a murine antibody, that retains or substantially retains the antigen-binding properties of the parent antibody, but which is less immunogenic in humans. This may be achieved by various methods, including (a) grafting the entire non-human variable domains onto human constant regions to generate chimeric antibodies; (b) grafting only the non-human complementarity determining regions (CDRs) into human framework and constant regions with or without retention of critical framework residues; and (c) transplanting the entire non-human variable domains, but "cloaking" them with a human-like section by replacement of surface residues. Such methods are disclosed in Morrison et al., Proc. Natl. Acad Sci. 81: 6851-5 (1984); Morrison et al., Adv. Immunol. 44: 65-92 (1988); Verhoeyen et al., Science 239:1534-1536 (1988); Padlan, Molec. Immun. 28:489-498 (1991); and Padlan, Molec. Immun. 31: 169-217 (1994), all of which are hereby incorporated by reference in their entirety. Humanized anti-CD40L antibodies can be prepared as described in U.S.P.N. 6,001,358 filed November 7, 1995 also incorporated herein by reference in its entirety.

By "human antibody" is meant an antibody containing entirely human light and heavy chain as well as constant regions, produced by any of the known standard methods.

By "primatized antibody" is meant a recombinant antibody which has been engineered to contain the variable heavy and light domains of a monkey (or other primate) antibody, in particular, a cynomolgus monkey antibody, and which contains human constant domain sequences, preferably the human immunoglobulin gamma 1 or gamma 4 constant domain (or PE variant). The preparation of such antibodies is described in Newman et al., Biotechnology, 10:1458-1460 (1992); also in commonly assigned 08/379,072, 08/487,550, or 08/746,361, all of which are incorporated by reference in their entirety herein. These antibodies have been reported to exhibit a high degree of homology to human antibodies, *i.e.,* 85-98%, display human effector functions, have reduced immunogenicity, and may exhibit high affinity to human antigens.

By "antibody fragment" is meant an fragment of an antibody such as Fab, F(ab')$_2$, Fab' and scFv.

By "chimeric antibody" is meant an antibody containing sequences derived from two different antibodies, which typically are of different species. Most typically, chimeric antibodies comprise human and murine antibody fragments, and generally human constant and murine variable regions.

The "CD20" antigen is a -35 kDa, non-glycosylated phosphoprotein found on the surface of greater than 90% of B cells from peripheral blood or lymphoid organs. CD20 is expressed during early pre-B cell development and remains until plasma cell differentiation. CD20 is present on both normal B cells as well as malignant B cells. Other names for CD20 in the literature include "B-lymphocyte-restricted antigen" and "Bp35". The CD20 antigen is described in Clark et al. PNAS (USA) 82:1766(1985).

The "CD 19" antigen refers to a -90kDa antigen identified, for example, by the HD237-CD 19 or B4 antibody (Kiesel et al. Leukemia Research 11, 12: 1119 (1987)). Like CD20, CD19 is found on cells throughout differentiation of the lineage from the stem cell stage up to a point just prior to terminal differentiation into plasma cells. Binding of an antagonist to CD 19 may cause internalization of the CD 19 antigen.

The "CD22" antigen refers to an antigen expressed on B cells, also known as "BL-CAM" and "LybB" that is involved in B cell signaling and an adhesion. (See Nitschke et al., Curr. Biol. 7:133 (1997); Stamenkovic et al., Nature 345:74 (1990)). This antigen is a membrane immunoglobulin-associated antigen that is tyrosine phosphorylated when membrane Ig is ligated. (Engel et al., J. Etyp. Med 181(4):1521 1586 (1995)). The gene encoding this antigen has been cloned, and its 1g domains characterized.

The B7 antigen includes the B7.1 (CD80), B7.2 (CD86) and B7.3 antigen, which are transmembrane antigens expressed on B cells. Antibodies which specifically bind B7 antigens, including human B7.1 and B7.2 antigens are known in the art. Preferred B7 antibodies comprise the primatized® B7 antibodies disclosed by Anderson et al. in U.S. Patent No. 6,113,898, assigned to IDEC Pharmaceuticals Corporation, as well as human and humanized B7 antibodies.

CD23 refers to the low affinity receptor for IgE expressed by B and other cells. In the present invention, CD23 will preferably be human CD23 antigen. CD23 antibodies are also known in the art. Most preferably, in the present invention, the CD23 antibody will be a human or chimeric anti-human CD23 antibody comprising human IgG1 or IgG3 constant domains.

A B cell "antagonist" is a molecule which, upon binding to a B cell surface marker, destroys or depletes B cells in a mammal and/or interferes with one or more B cell functions, e.g. by reducing or preventing a humoral response elicited by the B cell. The antagonist preferably is able to deplete B cells (i.e. reduce circulating B cell levels) in a mammal treated therewith. Such depletion may be achieved via various mechanisms such antibody-dependent cell-mediated cytotoxicity (ADCC) and/or complement dependent cytotoxicity (CDC), inhibition of B cell proliferation and/or induction of B cell death (e.g. via apoptosis). Antagonists included within the scope of the present invention include antibodies, synthetic or native sequence peptides and small molecule antagonists which bind to the B cell marker, optionally conjugated with or fused to a cytotoxic agent.

A CD40L antagonist is a molecule that specifically binds CD40L and preferably antagonizes the interaction of CD40L and CD40. Examples thereof include antibodies and antibody fragments that specifically bind CD40L, soluble CD40, soluble CD40 fusion proteins, and small molecules that bind CD40L. The preferred antagonist according to the invention comprises an antibody or antibody fragment specific to CD40.

"Antibody-dependent cell-mediated cytotoxicity" and "ADCC" refer to a cell mediated reaction in which nonspecific cytotoxic cells that express Fc receptors (FcRs) (e.g. Natural Killer (NK) cells, neutrophils, and macrophages) recognize bound antibody on a target cell and subsequently cause lysis of the target cell. The primary cells for mediating ADCC, NK cells, express FcγRIII only, whereas monocytes express FcγRI, FcγRII and FcγRIII. FcR expression on hematopoietic cells in summarized is Table 3 on page 464 of Ravetch and Kinet, Annu, Rev. Immunol 9:457-92 (1991). To assess ADCC activity of a molecule of interest, an *in vitro* ADCC assay, such as that described in US Patent No. 5,500,362 or 5,821,337 may be performed. Useful effector cells for such assays include peripheral blood mononuclear cells (PBMC) and Natural Killer (NK) cells. Alternatively, or additionally, ADCC activity of the molecule of interest may be *assessed in vivo, e.g.,* in a animal model such as that disclosed in Clynes et αl. PNAS (USA) 95:652-656 (1998).

"Human effector cells" are leukocytes which express one or more FcRs and perform effector functions. Preferably, the cells express at least FcγRIII and perform ADCC effector function. Examples of human leukocytes which mediate ADCC include peripheral blood mononuclear cells (PBMC), natural killer (NK) cells, monocytes, cytotoxic T cells and neutrophils; with PBMCs and NK cells being preferred. The effector cells may be isolated from a native source thereof, *e.g.* from blood or PBMCs as described herein.

The terms "Fc receptor" or "FcR" are used to describe a receptor that binds to the Fc region of an antibody. The preferred FcR is a native sequence human FcR. Moreover, a preferred FcR is one which binds an IgG antibody (a gamma receptor) and includes receptors of the FcγRI, FcγRII, and FcγRII subclasses, including allelic variants and alternatively spliced forms of these receptors. FcγRII receptors include FcγRIIA (an "activating receptor") and FcγRUB (an "inhibiting receptor"), which have similar amino acid sequences that differ primarily in the cytoplasmic domains thereof. Activating receptor FcγRIIA contains an immunoreceptor tyrosine-based activation motif (ITAM) in its cytoplasmic domain. Inhibiting receptor FcγRIIB contains an immunoreceptor tyrosine-based inhibition motif (ITIM) in its cytoplasmic domain. (see review M. in Daeon, Annu. Rev. Immunol. 15:203-234 (1997)). FcRs are reviewed in Ravetch and Kinet, Annu. Rev. Immunol. 9:457-92 (1991); Capel et al., Immunomethods 4:25-34 (1994); and de Haas et αl., J. Lαb. Clin. Med. 126:330-41 (1995). Other FcRs, including those to be identified in the future, are encompassed by the term "FcR" herein. The term also includes the neonatal receptor, FcRn, which is responsible for the transfer of maternal IgGs to the fetus (Guyer et αl., J. Immunol. 117:587 (1976) and Kim et al., J. Immunol. 24:249 (1994)).

"Complement dependent cytotoxicity" or "CDC" refers to the ability of a molecule to lyse a target in the presence of complement. The complement activation pathway is initiated by the binding of the first component of the complement system (Clq) to a molecule (e.g. an antibody) complexed with a cognate antigen. To assess complement activation, a CDC assay, e.g. as described in Gazzano-Santoro et al., J. Immunol. Methods 202:163 (1996), may be performed.

"Growth inhibitory" antagonists are those which prevent or reduce proliferation of a cell expressing an antigen to which the antagonist binds. For example, the antagonist may prevent or reduce proliferation of B cells in vitro and/or in vivo.

Antagonists which "induce apoptosis" are those which induce programmed cell death, e.g. of a B cell, as determined by binding of annexin V, fragmentation of DNA, cell shrinkage, dilation of endoplasmic reticulum, cell fragmentation, and/or formation of membrane vesicles (called apoptotic bodies).

The term "hypervariable region" when used herein refers to the amino acidresidues of an antibody which are responsible for antigen-binding. The hypervariable region comprises amino acid residues from a "complementarity determining region" or "CDR" (e.g. residues 24-34 (L1), 50-56 (L2) and 89-97 (L3) in the light chain variable domain and 31-35 (H1), 50-65 (H2) and 95-102 (H3) in the heavy chain variable domain; Kabat et αl., Sequences of Proteins of Immunologicαl Interest, 5th Ed. Public Health Service, National Institutes of Health, Bethesda, MD. (1991)) and/or those residues from a "hypervariable loop" (e.g. residues 26-32 (L1), 50-52 (L2) and 91-96 (L3) in the light chain variable domain and 26-32 (H1), 53-55 (H2) and 96-101 (H3) in the heavy chain variable domain; Chothia and Lesk.1. Mol. Biol. 196:901-917 (1987)). "Framework" or "FR" residues are those variable domain residues other than the hypervariable region residues as herein defined.

An antagonist "which binds" an antigen of interest, e.g. a B cell surface marker, is one capable of binding that antigen with sufficient affinity such that the antagonist is useful as a therapeutic agent for targeting a cell, i.e. a B cell, expressing the antigen.

An "anti-CD20 antibody" herein is an antibody that specifically binds CD20 antigen, preferably human CD20, having measurable B cell depleting activity, preferably having at least about 10% the B cell depleting activity of RITUXAN® (see U.S. Patent No. 5,736,137, incorporated by reference herein in its entirety).

As previously alluded to, the terms "rituximab" or "RITUXAN®" herein refer to the genetically engineered chimeric murine/human monoclonal antibody directed against the CD20 antigen and designated "C2B8" in US Patent No. 5,736,137 expressly incorporated herein by reference. The antibody is an IgGI kappa immunoglobulin containing murine light and heavy chain variable region sequences and human constant region sequences. Rituximab has a binding affinity for the CD20 antigen of approximately 8.0 nM.

An "anti-CD22 antibody" herein is an antibody that specifically binds CD22 antigen, preferably human CD22, having measurable B cell depleting activity, preferably having at least about 10% the B cell depleting activity of RITUXAN®.

An "anti-CD 19 antibody" herein is an antibody that specifically binds CD 19 antigen, preferably human CD19, having measurable B cell depleting activity, preferably having at least about 10% the B cell depleting activity of RITUXAN®.

An "anti-CD37 antibody" herein is an antibody that specifically binds CD37 antigen, preferably human CD37, having measurable B cell depleting activity, preferably having at least about 10% the B cell depleting activity of RITUXAN®.

An "anti-B7 antibody" herein is an antibody that specifically binds B7.1, B7.2 or B7.3, most preferably human B7.3, that inhibits B7/CD28 interactions and, which more does not substantially inhibit B7/CTLA-4 interactions, and even more preferably, the particular antibodies described in U.S. Patent 6,113,898, incorporated by reference in its entirety herein. IDEC- 114 (IDEC Pharmaceuticals, San Diego CA) is a anti-87 antibody presently in phase II clincal trials and is compatible with preferred embodiments of the instant invention. It has recently been shown that these antibodies promote apoptosis. Therefore, they are well suited for anti-neoplastic applications. Other examples of antibodies that bind B7 antigen include the B7 antibody reported U.S. Patent 5,885,577, issued to Linsley et al, the anti-B7 antibody reported in U.S. Patent 5,869,050, issued in DeBoer et al, assigned to Chiron Corporation.

An "anti-CD40L antibody" is an antibody that specifically binds CD40L (also known as CD154, gp39, TBAM), preferably one having agonistic activity. A preferred anti-Cd40L antibody is one having the specificity of a humanized antibody disclosed in U.S. Patent No. 6,011,358 (assigned to IDEC Pharmaceuticals Corporation), incorporated by reference in its entirety herein. IDEC-131 (IDEC Pharmaceutical, San Diego CA) is an anti-CD40L antibody presently in phase II clincal trials and is compatible with preferred embodiments of the instant invention.

An "anti-CD4 antibody" is one that specifically binds CD4, preferably human CD4, more preferably a primatized or humanized anti-CD4 antibody.

An "anti-CD40 antibody" is an antibody that specifically binds CD40, preferably human CD40, such as those disclosed in U.S. Patent 5,874,085, 5,874,082, 5,801,227, 5,674,442, snf 5,667,165, all of which are incorporated by reference herein.

Preferably, both the B cell depleting antibody and the immunoregulatory antibody will contain human constant domains. Suitable antibodies may include IgG1, IgG2, IgG3 and IgG4 isotypes.

Specific examples of antibodies which bind the CD20 antigen include: "Rituximab" ("RITUXANS®") (US Patent No. 5,736,137, expressly incorporated herein by reference); yttrium-[90]-labeled 2B8 murine antibody "Y2B8" (US Patent No.5,736,B7, expressly incorporated herein by reference); murine IgG2a "B1" optionally labeled with $^{131}$I,«$^{131}$I B1" antibody (BEXXAR™) (US Patent No. 5,595,721, expressly incorporated herein by reference); murine monoclonal antibody "1F5" (Press et αl. Blood 69(2):584-591 (1987); and "chimeric 2H7" antibody (US Patent No. 5,677,180, expressly incorporated herein by reference).

Specific examples of antibodies which bind CD22 include Lymphocide™ reported by Immunomedics, now in clinical trials for non-Hodgkin's lymphoma.

Specific examples of antibodies that bind CD23 are well known and preferably include the primatized® antibodies specific to human CD23 reported by Reff et al., in U.S. Patent 6,011,138, issued on July 4, 1999, co-assigned to IDEC Pharmaceuticals Corp. and Seikakagu Corporation of Japan; those reported by Bonnefoy *et al.,* No. 96 12741; Rector et αl. J. Immunol. 55:481-488 (1985); Flores-Rumeo et αl. Science 241:1038-1046 (1993); Sherr et αl. J. Immunol., 142:481-489 (1989); and Pene et αl., PNAS, USA 85:6820-6824 (1988). IDEC-152 (IDEC Pharmaceuticals, San Diego CA) is an anti-CD23 antibody presently in phase II clincal trials and is compatible with preferred embodiments of the instant invention. Such antibodies are reportedly useful for treatment of allergy, autoimmune diseases, and inflammatory diseases.

An "isolated" antagonist is one which has been identified and separated and/or recovered from a component of its natural environment. Contaminant components of its natural environment are materials which would interfere with diagnostic or therapeutic uses for the antagonist, and may include enzymes, hormones, and other proteinaceous or nonproteinaceous solutes. In preferred embodiments, the antagonist will be purified (1) to greater than 95% by eight of antagonist as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, preferably, silver stain. Isolated antagonist includes the antagonist *in situ* within recombinant cells since at least one component of the antagonist's natural environment will not be present. Ordinarily, however, isolated antagonist will be prepared by at least one purification step.

"Mammal" for purposes of treatment refers to any animal classified as a mammal, including humans, domestic and farm animals, and zoo, sports, or pet animals, such as dogs, horses, cats, cows, etc. Preferably, the mammal is human.

"Treatment" refers to both therapeutic treatment and prophylactic or preventative measures. Those in need of treatment include those already with the disease or disorder as well as those in which the disease or disorder is to be prevented. Hence, the mammal may have been diagnosed as having the disease or disorder or may be predisposed or susceptible to the disease.

As discussed in detail above, the present invention provides compounds, compositions, kits and methods for the treatment of neoplastic disorders in a mammalian subject in need of treatment thereof. Preferably, the subject is a human. The neoplastic disorder (e.g., cancers and malignancies) may comprise solid tumors such as melanomas, gliomas, sarcomas, and carcinomas as well as myeloid or hematologic malignancies such as lymphomas and leukemias. In general the disclosed invention may be used to prophylactically or therapeutically treat any neoplasm comprising an antigenic marker that allows for the targeting of the cancerous cells by the modified antibody. Exemplary cancers that may be treated include, but are not limited to, prostate, colon, skin, breast, ovarian, lung and pancreatic. In preferred embodiments selected antibody combinations of the instant invention may be used to diagnose or treat colon cancers or other gastric carcinomas. More particularly, the antibodies of the instant invention may be used to treat Kaposi's sarcoma, CNS neoplasms (capillary hemangioblastomas, meningiomas and cerebral metastases), melanoma, gastrointestinal and renal sarcomas, rhabdomyosarcoma, glioblastoma (preferably glioblastoma multiforme), leiomyosarcoma, retinoblastoma, papillary cystadenocarcinoma of the ovary, Wilm's tumor or small cell lung carcinoma. It will be appreciated that appropriate antibody combinations may be derived for tumor associated antigens related to each of the forgoing neoplasms without undue experimentation in view of the instant disclosure.

Exemplary hematologic malignancies that are amenable to treatment with the disclosed invention include Hodgkins and non-Hodgkins lymphoma as well as leukemias, including ALL-L3 (Burkitt's type leukemia), chronic lymphocytic leukemia (CLL) and monocytic cell leukemias. It will be appreciated that the compounds and methods of the present invention are particularly effective in treating a variety of B-cell lymphomas, including low grade/ follicular non-Hodgkin's lymphoma (NHL), cell lymphoma (FCC), mantle cell lymphoma (MCL), diffuse large cell lymphoma (DLCL), small lymphocytic (SL) NHL, intermediate grade/ follicular NHL, intermediate grade diffuse NHL, high grade immunoblastic NHL, high grade lymphoblastic NHL, high grade small non-cleaved cell NHL, bulky disease NHL and Waldenstrom's Macroglobulinemia. It should be clear to those of skill in the art that these lymphomas and lukemias will often have different names due to changing systems of classification, and that patients having hematologic malignancies classified under different names may also benefit from the combined therapeutic regimens of the present invention. In addition to the aforementioned neoplastic disorders, it will be appreciated that the disclosed invention may advantageously be used to treat additional malignancies bearing compatible tumor associated antigens.

In preferred embodiments the neoplastic disorder will comprise a B cell malignancy. According to the present invention this includes any B cell malignancy, e.g., B cell lymphomas and leukemias. Preferred examples include Hodgkin's disease (all forms, e.g., relapsed Hodgkin's disease, resistant Hodgkin's disease) non-Hodgkin's lymphomas (low grade, intermediate grade, high grade, and other types). Examples include small lymphocytic/B cell chronic lymphocytic leukemia (SLL/B-CLL), lymhoplasmacytoid lymphoma (LPL), mantle cell lymphoma (MCL), follicular lymphoma (FL), diffuse large cell lymphoma (DLCL), Burkitt's lymphoma (BL), AIDS- related lymphomas, monocytic B cell lymphoma, angioimmunoblastic lymphoadenopathy, small lymphocytic, follicular, diffuse large cell, diffuse small cleaved cell, large cell immunoblastic lymphoblastoma, small, non-cleaved, Burkitt's and non-Burkitt's, follicular, predominantly large cell; follicular, predominantly small cleaved cell; and follicular, mixed small cleaved and large cell lymphomas. See, Gaidono et al., "Lymphomas", IN CANCER: PRINCIPLES & PRACTICE OF ONCOLOGY, Vol. 2: 2131-2145 (DeVita et al., eds., 5th ed. 1997).

Other types of lymphoma classifications include immunocytomal Waldenstrom's MALT oetype/monocytoid B cell, mantle cell lymphoma B-CLL/SLL, diffuse large B-cell lymphoma, follicular lymphoma, and precursor B-LBL.

As noted, B cell malignancies further include especially leukemias such as ALL-L3 (Burkitt's type leukemia), chronic lymphocytic leukemia (CLL), chronic leukocytic leukemia, acute myelogenous leukemia, acute lymphoblastic leukemia, chronic lymphocytic leukemia, chronic myelogenous leukemia, lymphoblastic leukemia, lymphocytic leukemia, monocytic leukemia, myelogenous leukemia, and promyelocytic leukemia and monocytic cell leukemias.

The expression "therapeutically effective amount" refers to an amount of the antagonist which is effective for preventing, ameliorating or treating the B cell malignancy disease in question.

The term "immunosuppressive agent" as used herein for adjunct therapy refers to substances that act to suppress or mask the immune system of the mammal being treated herein. This would include substances that suppress cytokine production, downregulate or suppress self-antigen expression, or mask the MHC antigens. Examples of such agents include 2-amino-6-aryl-5-substituted pyrimidines (see U.S. Pat. No. 4,665,077, the disclosure of which is incorporated herein by reference), azathioprine; cyclophosphamide; bromocryptine; danazol; dapsone; glutaraldehyde (which masks the MHC antigens, as described in U.S. Pat. No. 4,120,649); anti-idiotypic antibodies for MHC antigens and MHC fragments; cyclosporin A; steroids such as glucocorticosteroids, e.g., prednisone, methylprednisolone, and dexamethasone; cytokine or cytokine receptor antagonists including anti-interferon-a, β- or δ-antibodies, anti-tumor necrosis factor-a antibodies, anti-tumor necrosis factor-β antibodies, anti-interleukin-2 antibodies and anti-IL-2 receptor antibodies; anti-LFA- 1 antibodies, including anti-CD1 1a and anti-CD18 antibodies; anti-L3T4 antibodies; heterologous anti-lymphocyte globulin; pan-T antibodies, preferably anti-CD3 or anti-CD4/CD4a antibodies; soluble peptide containing a LFA-3 binding domain (WO 90/08187 published 7/26/90), streptolanase; TGF-β; streptodomase; RNA or DNA from the host; FK506; RS-61443; deoxyspergualin; rapamycin; T-cell receptor (Cohen et al., US. Pat. No. 5,114,721); T-cell receptor fragments (Offner et al., Science, 251: 430-432 (1991); WO 90/11294; laneway, Nature, 341: 482 (1989); and WO 91/01133); and T cell receptor antibodies (EP 340,109) such as T10B9.

As used herein, "a cytotoxin or cytotoxic agent" means any agent that is detrimental to the growth and proliferation of cells and may act to reduce, inhibit or distroy a malignancy when exposed thereto. Exemplary cytotoxins include, but are not limited to, radionuclides, biotoxins, cytostatic or cytotoxic therapeutic agents, prodrugs, immunologically active ligands and biological response modifiers such as cytokines. As will be discussed in more detail below, radionuclide cytotoxins are particularly preferred for use in the instant invention. However, any cytotoxin that acts to retard or slow the growth of malignant cells or to eliminate malignant cells and may be associated with the modified antibodies disclosed herein is within the purview of the present invention.

It will be appreciated that, in previous studies, anti-tumor antibodies labeled with isotopes have been used successfully to destroy cells in solid tumors as well as lymphomas/leukemias in animal models, and in some cases in humans. The radionuclides act by producing ionizing radiation which causes multiple strand breaks in nuclear DNA, leading to cell death. The isotopes used to produce therapeutic conjugates typically produce high energy α-, γ- or β- particles which have a therapeutically effective path length. Such radionuclides kill cells to which they are in close proximity, for example neoplastic cells to which the conjugate has attached or has entered. They generally have little or no effect on non-localized cells. Radionuclides are essentially non-immunogenic.

With respect to the use of radiolabeled conjugates in conjunction with the present invention, the antibodies may be directly labeled (such as through iodination) or may be labeled indirectly through the use of a chelating agent. As used herein, the phrases "indirect labeling" and "indirect labeling approach" both mean that a chelating agent is covalently attached to an antibody and at least one radionuclide is associated with the chelating agent. Such chelating agents are typically referred to as bifunctional chelating agents as they bind both the polypeptide and the radioisotope. Particularly preferred chelating agents comprise 1-isothiocycmatobenzyl-3-methyldiothelene triaminepentaacetic acid ("MX-DTPA") and cyclohexyl diethylenetriamine pentaacetic acid ("CHX-DTPA") derivatives. Other chelating agents comprise P-DOTA and EDTA derivatives. Particularly preferred radionuclides for indirect labeling include $^{111}$In and [90]Y.

As used herein, the phrases "direct labeling" and "direct labeling approach" both mean that a radionuclide is covalently attached directly to an antibody (typically via an amino acid residue). More specifically, these linking technologies include random labeling and site-directed labeling. In the latter case, the labeling is directed at specific sites on the dimer or tetramer, such as the N-linked sugar residues present only on the Fc portion of the conjugates. Further, various direct labeling techniques and protocols are compatible with the instant invention. For example, Technetium-99m labelled antibodies may be prepared by ligand exchange processes, by reducing pertechnate ($TcO_4$-) with stannous ion solution, chelating the reduced technetium onto a Sephadex column and applying the antibodies to this column, or by batch labelling techniques, e.g. by incubating pertechnate, a reducing agent such as $SnCl_2$, a buffer solution such as a sodium-potassium phthalate-solution, and the antibodies. In any event, preferred radionuclides for directly labeling antibodies are well known in the art and a particularly preferred radionuclide for direct labeling is $^{131}$I, covalently attached via tyrosine residues. Antibodies according to the invention may be derived, for example, with radioactive sodium or potassium iodide and a chemical oxidising agent, such as sodium hypochlorite, chloramine T or the like, or an enzymatic oxidising agent, such as lactocroxidase, glucose oxidase and glucose. However, for the purposes of the present invention, the indirect labeling approach is particularly preferred.

Patents relating to chelators and chelator conjugates are known in the art. For instance, U.S. Patent No. 4,831,175 of Gansow is directed to polysubstituted diethylenetrimninepentaacetic acid chelates and protein conjugates containing the same, and methods for their preparation. U.S. Patent Nos. 5,099,069, 5,246,692, 5,286,850, 5,434,287 and 5,124,471 of Gansow also relate to polysubstituted DTPA chelates. These patents are incorporated herein in their entirety. Other examples of compatible metal chelators are ethylenediaminetetraacetic acid (EDTA), diethylenetriaminepentaacetic acid (DPTA), 1,4,8,11-tetraazatctradecane, 1,4,8,11-tetraazatetradecane-1,4,8,11- tetraacetic acid, 1-oxa-4,7,12,15-tetraazaheptadecane-4,7,12,15-tetraacetic acid, or the like. Cyclohexyl-DTDA or CHX-DTPA is particularly preferred and is exemplified extensively below. Still other compatible chelators, including those yet to be discovered, may easily be discerned by a skilled artisan and are clearly within the scope of the present invention.

Compatible chelators, including the specific bifunctional chelator used to facilitate chelation in co-pending application Serial Nos. 08/475,813, 08/475,815 and 08/478,967, are preferably selected to provide high affinity for trivalent metals, exhibit increased tumor-to-non-tumor ratios and decreased bone uptake as well as greater in vivo retention of radionuclide at target sites, i.e., B-cell lymphoma tumor sites. However, other bifunctional chelators that may or may not possess all of these characteristics are known in the art and may also be beneficial in tumor therapy.

It will also be appreciated that, in accordance with the teachings herein, antibodies may be conjugated to different radiolabels for diagnostic and therapeutic purposes. To this end the aforementioned co-pending applications, herein incorporated by reference in their entirety, disclose radiolabeled therapeutic conjugates for diagnostic "imaging" of tumors before administration of therapeutic antibody. "In2B8" conjugate comprises a murine monoclonal antibody, 2B8, specific to human CD20 antigen, that is attached to $^{111}$In via a bifunctional chelator, i.e., MX-DTPA (diethylenetriaminepentaacetic acid), which comprises a 1: mixture of 1-isothiocyanatobenzyl-3-methyl-DTPA and 1-methyl-3-isothiocyanatobenzyl-DTPA. $^{111}$In is particularly preferred as a diagnostic radionuclide because between about 1 to about 10 mCi can be safely administered without detectable toxicity; and the imaging data is generally predictive of subsequent $^{90}$Y-labeled antibody distribution. Most imaging studies utilize 5 mCi $^{111}$In-labeled antibody, because this dose is both safe and has increased imaging efficiency compared with lower doses, with optimal imaging occurring at three to six days after antibody administration. See, for example, Murray, J. Nut. Med. 26: 3328 (1985) and Carraguillo et al., J Nuc. Med. 26: 67 (1985).

As indicated above, a variety of radionuclides are applicable to the present invention and those skilled in the art are credited with the ability to readily determine which radionuclide is most appropriate under various circumstances. For example, $^{131}$I is a well known radionuclide used for targeted immunotherapy. However, the clinical usefulness of $^{131}$I can be limited by several factors including: eight-day physical half-life; dehalogenation of iodinated antibody both in the blood and at tumor sites; and emission characteristics (e.g., large gamma component) which can be suboptimal for localized dose deposition in tumor. With the advent of superior chelating agents, the opportunity for attaching metal chelating groups to proteins has increased the opportunities to utilize other radionuclides such as $^{111}$In and $^{90}$Y. $^{90}$Y provides several benefits for utilization in radioimmunotherapeutic applications: the 64 hour half-life of $^{90}$Y is long enough to allow antibody accumulation by tumor and, unlike e.g., $^{131}$I, $^{90}$Y is a pure beta emitter of high energy with no accompanying gamma irradiation in its decay, with a range in tissue of 100 to 1,000 cell diameters. Furthermore, the minimal amount of penetrating radiation allows for outpatient administration of $^{90}$Y-labeled antibodies. Additionally, internalization of labeled antibody is not required for cell killing, and the local emission of ionizing radiation should be lethal for adjacent tumor cells lacking the target antigen.

Effective single treatment dosages (i.e., therapeutically effective amounts) of $^{90}$Y-labeled modified antibodies range from between about 5 and about 75 mCi, more preferably between about 10 and about 40 mCi. Effective single treatment non-marrow ablative dosages of $^{131}$I-labeled antibodies range from between about 5 and about 70 mCi, more preferably between about 5 and about 40 mCi. Effective single treatment ablative dosages (i.e., may require autologous bone marrow transplantation) of $^{131}$I-labeled antibodies range from between about 30 and about 600 mCi, more preferably between about 50 and less than about 500 mCi. In conjunction with a chimeric antibody, owing to the longer circulating half life vis-á-vis murine antibodies, an effective single treatment non-marrow ablative dosages of iodine-131 labeled chimeric antibodies range from between about 5 and about 40 mCi, more preferably less than about 30 mCi. Imaging criteria for, e.g., the $^{111}$In label, are typically less than about 5 mCi.

While a great deal of clinical experience has been gained with $^{131}$I and $^{90}$Y, other radiolabels are known in the art and have been used for similar purposes. Still other radioisotopes are used for imaging. For example, additional radioisotopes which are compatible with the scope of the instant invention include, but are not limited to, $^{123}$I, $^{125}$I, $^{32}$P, $^{51}$Co, $^{64}$Cu, $^{61}$Cu, $^{17}$Br, $^{81}$Rb, $^{81}$Kr, $^{87}$Sr, $^{113}$In, $^{121}$Cs, $^{129}$Cs, $^{132}$I, $^{197}$Hg, $^{203}$Pb, $^{206}$Bi, $^{177}$Lu, $^{186}$Re, $^{212}$Pb, $^{212}$Bi, $^{47}$Sc, $^{105}$Rh, $^{109}$Pd, $^{153}$Sm $^{188}$Re, $^{199}$Au, $^{225}$Ac $^{211}$At, and $^{213}$Bi. In this respect alpha, gamma and beta emitters are all compatible with in the instant invention. Further, in view of the instant disclosure it is submitted that one skilled in the art could readily determine which radionuclides are compatible with a selected course of treatment without undue experimentation. To this end, additional radionuclides which have already been used in clinical diagnosis include $^{125}$I, $^{123}$I, $^{99}$Tc, $^{43}$K, $^{52}$Fe $^{67}$Ga, $^{68}$Ga, as well as $^{111}$In. Antibodies have also been labeled with a variety of radionuclides for potential use in targeted immunotherapy Peirersz et al. Immunol. Cell Biol. 65: 111-125 (1987). These radionuclides include $^{188}$Re and $^{186}$Re as well as $^{199}$Au and $^{67}$Cu to a lesser extent. U.S. Patent No. 5,460,785 provides additional data regarding such radioisotopes and is incorporated herein by reference.

In addition to radionuclides, the modified antibodies of the present invention may be conjugated to, or associated with, any one of a number of biological response modifiers, pharmaceutical agents, toxins or immunologically active ligands. Those skilled in the art will appreciate that these non-radioactive conjugates may be assembled using a variety of techniques depending on the selected cytotoxin. For example, conjugates with biotin are prepared e.g. by reacting the modified antibodies with an activated ester of biotin such as the biotin N-hydroxysuccinimide ester. Similarly, conjugates with a fluorescent marker may be prepared in the presence of a coupling agent, e.g. those listed above, or by reaction with an isothiocyanate, preferably fluorescein-isothiocyanate. Conjugates of the chimeric antibodies of the invention with cytostatic/cytotoxic substances and metal chelates are prepared in an analogous manner.

Preferred agents for use in the present invention are cytotoxic drugs, particularly those which are used for cancer therapy. Such drugs include, in general, cytostatic agents, alkylating agents, antimetabolites, anti-proliferative agents, tubulin binding agents, hormones and hormone antagonists, and the like. Exemplary cytostatics that are compatible with the present invention include alkylating substances, such as mechlorethamine, triethylenephosphoramide, cyclophosphamide, ifosfamide, chlorambucil, busulfan, melphalan or triaziquone, also nitrosourea compounds, such as carmustine, lomustine, or semustine. Other preferred classes of cytotoxic agents include, for example, the anthracycline family of drugs, the vinca drugs, the mitomycins, the bleomycins, the cytotoxic nucleosides, the pteridine family of drugs, diynenes, and the podophyllotoxins. Particularly useful members of those classes include, for example, adriamycin, carminomycin, daunorubicin (daunomycin), doxorubicin, aminopterin, methotrexate, methopterin, mithramycin, streptonigrin, dichloromethotrexate, mitomycin C, actinomycin-D, porfiromycin, 5-fluorouracil, floxuridine, ftorafur, 6-mercaptopurine, cytarabine, cytosine arabinoside, podophyllotoxin, or podophyllotoxin derivatives such as etoposide or etoposide phosphate, melphalan, vinblastine, vincristine, leurosidine, vindesine, leurosine and the like. Still other cytotoxins that are compatible with the teachings herein include taxol, taxane, cytochalasin B, gramicidin D, ethidium bromide, emetine, tenoposide, colchicin, dihydroxy anthracin dione, mitoxantrone, procaine, tetracaine, lidocaine, propranolol, and puromycin and analogs or homologs thereof. Hormones and hormone antagonists, such as corticosteroids, e.g. prednisone, progestins, e.g. hydroxyprogesterone or medroprogesterone, estrogens, e.g. diethylstilbestrol, antiestrogens, e.g. tamoxifen, androgens, e.g. testosterone, and aromatase inhibitors, e.g. aminogluthetimide are also compatible with the teachings herein. As noted previously, one skilled in the art may make chemical modifications to the desired compound in order to make reactions of that compound more convenient for purposes of preparing conjugates of the invention.

One example of particularly preferred cytotoxins comprise members or derivatives of the enediyne family of anti-tumor antibiotics, including calicheamicin, esperamicins or dynemicins. These toxins are extremely potent and act by cleaving nuclear DNA, leading to cell death. Unlike protein toxins which can be cleaved in vivo to give many inactive but immunogenic polypeptide fragments, toxins such as calicheamicin, esperamicins and other enediynes are small molecules which are essentially non-immunogenic. These non-peptide toxins are chemically-linked to the dimers or tetramers by techniques which have been previously used to label monoclonal antibodies and other molecules. These linking technologies include site-specific linkage via the N-linked sugar residues present only on the Fc portion of the conjugates. Such site-directed linking methods have the advantage of reducing the possible effects of linkage on the binding properties of the conjugate.

As previously alluded to, compatible cytotoxins may comprise a prodrug. As used herein, the term "prodrug" refers to a precursor or derivative form of a pharmaceutically active substance that is less cytotoxic to tumor cells compared to the parent drug and is capable of being enzymatically activated or converted into the more active parent form. Prodrugs compatible with the invention include, but are not limited to, phosphate-containing prodrugs, thiophosphate-containing prodrugs, sulfate containing prodrugs, peptide containing prodrugs, β-lactam-containing prodrugs, optionally substituted phenoxyacetainide-containing prodrugs or optionally substituted phenylacetamide-containing prodrugs, 5-fluorocytosine and other 5-fluorouridine prodrugs that can be converted to the more active cytotoxic free drug. Further examples of cytotoxic drugs that can be derivatized into a prodrug form for use in the present invention comprise those chemotherapeutic agents described above.

Among other cytotoxins, it will be appreciated that the antibody can also be associated with a biotoxin such as ricin subunit A, abrin, diptheria toxin, botulinum, cyanginosins, saxitoxin, shigatoxin, tetanus, tetrodotoxin, trichothecene, verrucologen or a toxic enzyme. Preferably, such constructs will be made using genetic engineering techniques that allow for direct expression of the antibody-toxin construct. Other biological response modifiers that may be associated with the modified antibodies of the present invention comprise cytokines such as lymphokines and interferons. Moreover, as indicated above, similar constructs may also be used to associate immunologically active ligands (e.g. antibodies or fragments thereof) with the modified antibodies of the present invention. Preferably, these immunologically active ligands would be directed to antigens on the surface of immunoactive effector cells. In these cases, the constructs could be used to bring effector cells, such as T cells or NK cells, in close proximity to the neoplastic cells bearing a tumor associated antigen thereby provoking the desired immune response. In view of the instant disclosure it is submitted that one skilled in the art could readily form such constructs using conventional techniques.

A "chemotherapeutic agent" is a chemical compound useful in the treatment of cancer. Examples of chemotherapeutic agents include alkylating agents such as thiotepa and cyclosphosphamide (CYTOXAN™); alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethylenethiophosphaoramide and trimethylolomelamime nitrogen mustards such as chiorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nittosureas such as carmustine, chlorozotoein, fotemustine, lomustine, nimustine, ranimustine; antibiotics such as aclacinomysins, actinomycin, authramycin, azaserine, bleomycins, cactinomycin, calicheamicin, carabicin, carminomyein, carzinophifin, chromomycins, dactinomycin, daunorubicin, detorubicin, 6-diazo-5-oxo-L-norleucine, doxorubicin, epirubicin, esonibicin, idarubicin, inarcellomycin, mitomycins, mycophenolic acid, nogalamycin, olivomycins, peplomycin, potfiromycin, puromycin, quelamycin, rodorubicin, streptonigrin, streptozocin, tubercidili, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguamne; pyrimidine analogs such as ancitabine, azacitidine, 6-azauridine, carmofur, cytarabine, dideoxyuridine, doxifluridine, enocitabine, floxuridine, 5-FU; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aidophosphamide glycoside; aminolevulinic acid; amsacrine; bestrabucil; bisantrene; edatraxate; defofamine; demecolcine; diaziquone; elfornithine; elliptinium acetate; etoglucid; gallium nitrate; hydroxyurea; lentinan; lonidamine; mitoguazone; mitoxantrone; mopidamol; nitracrine; pentostatin; phenamet; pirarubicin; podophyllinic acid; 2-ethylhydrazide; procarbazine; PSK® razoxane; sizofiran; spirogermanium; tenuazonic acid; triaziquone; 2, 2',2"-trichlorotriethylamine; urethan; vindesine; dacarbazine; mannomustine; mitobronitol; mitolactol; pipobroman; gacytosine; arabinoside ("Ara-C"); cyclophosphamide; thiotepa; taxoids, e.g. paclitaxel (TAXOL®, Bristol-Myers Squibb Oncology, Princeton, NJ) and doxetaxel (Taxotere, Rhone-Poulenc Rorer, Antony, France); chlorambucil; gemcitabine; 6-thioguanine; mercaptopurine; methotrexate; platinum analogs such as cisplatin and carboplatin; vinblastine; platinum; etoposide (VP-16); ifosfamide; mitomycin C; mitoxantrone; vincristine; vinorelbine; navelbine; novantrone; teniposide; daunomycin; aminopterin; xeloda; ibandronate; CPT11; topoisomerase inhibitor RFS 2000; difluoromethylomithine (DMFO); retinoic acid; esperamicins; capecitabine; and pharmaceutically acceptable salts, acids or derivatives of any of the above. Also included in this definition are anti-hormonal agents that act to regulate or inhibit hormone action on tumors such as anti-estrogens including for example tamoxifen, raloxifene, aromatase inhibiting 4(5)-imidazoles, 4-hydroxytamoxifen, trioxifene, keoxifene, LY117018, onapristone, and toremifene (Fareston); and antiandrogens such as flutamide, nilutamide, bicalutamide, leuprolide, and goserelin; and pharmaceutically acceptable salts, acids or derivatives of any of the above.

The term "cytokine" is a generic term for proteins released by one cell population which act on another cell as intercellular mediators. Examples of such cytokines are lymphokines, monokines, and traditional polypeptide hormones. Included among the cytokines are growth hormone such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen; tumor necrosis factor-a and -β; muflerian-inhibiting substance; mouse gonadotropin-associated peptide; inhibin; activin; vascular endothelial growth factor; integrin; thrombopoietin (TPO); nerve growth factors such as NGF-13; platelet-growth factor; transforming growth factors (TGFs) such as TGF-$\alpha$ and TGF-$\beta$; insulin-like growth factor-I and -II; erythropoietin (EPO); osteoinductive factors; interferons such as interferon-$\alpha$, -$\beta$, and -y; colony stimulating factors (CSFs) such as macrophage-CSF (M-CSF); granulocytemacrophage-CSF (GM-CSF); and granulocyte-CSF (G-CSF); interleukins (ILs) such as IL-1, IL-1a, IL-2, IL-g, IL-4, IL-5, IL-6, IL-7, IL-8, IL-9, IL-11, IL-12, IL-15; a tumor necrosis factor such as TNF-$\alpha$ or TNF-$\beta$; and other polypeptide factors including LIF and kit ligand (KL). As used herein, the term cytokine includes proteins from natural sources or from recombinant cell culture and biologically active equivalents of the native sequence cytokines.

The term "prodrug" as used in this application refers to a precursor or derivative form of a pharmaceutically active substance that is less cytotoxic to tumor cells compared to the parent drug and is capable of being enzymatically activated or converted into the more active parent form. See, e.g., Wilman, "Prodrugs in Cancer Chemotherapy" Biochemical Society Transactions, 14, pp. 375-382, 615th Meeting Belfast (1986) and Stella et al., "Prodrugs: A Chemical Approach to Targeted Drug Delivery," Directed Drug Delivery, Borchardt et al., (ed.), pp. 247-267, Humana Press (1985). The prodrugs of this invention include, but are not limited to, phosphate-containing prodrugs, thiophosphate-containing prodrugs, sulfate-containing prodrugs, peptide-containing prodrugs, D-amino acid-modified prodrugs, glycosylated prodrugs, 13-lactam-containing prodrugs, optionally substituted phenoxyacetamide-containing prodrugs or optionally substituted phenylacetamide-containing prodrugs, 5-fluorocytosine and other 5fluorouridine prodrugs which can be converted into the more active cytotoxic free drug. Examples of cytotoxic drugs that can be derivatized into a prodrug form for use in this invention include, but are not limited to, those chemotherapeutic agents described above.

A "liposome" is a small vesicle composed of various types of lipids, phospholipids and/or surfactant which is useful for delivery of a drug (such as the antagonists disclosed herein and, optionally, a chemotherapeutic agent) to a mammal. The components of the liposome are commonly arranged in a bilayer formation, similar to the lipid arrangement of biological membranes.

The term "package insert" is used to refer to instructions customarily included in commercial packages of therapeutic products, that contain information about the indications, usage, dosage, administration, contraindications and/or warnings concerning the use of such therapeutic products.

The methods and articles of manufacture of the present invention use, or incorporate, at least one antibody that has immunoregulatory activity, e.g. anti-B7, anti-CD23, anti-CD40L, anti-CD4 or anti-CD40 antibodies, and, optionally, at least one antibody that binds to a B cell surface marker having B depleting activity, e.g., ami-CD20, anti-CD22, anti-CD 19, or anti-CD37 antibody. Accordingly, methods for generating such antibodies will be described herein.

The molecule to be used for production of, or screening for, antigen(s) may be, e.g., a soluble form of the antigen or a portion thereof, containing the desired epitope. Alternatively, or additionally, cells expressing the antigen at their cell surface can be used to generate, or screen for, antagonist(s). Other forms of the B cell surface marker useful for generating antagonists will be apparent to those skilled in the art. Suitable antigen sources for CD40L, CD40, CD19, CD20, CD22, CD23, CD37, CD4 and B7 antigen (e.g., B7.1, B7.2) antigen for producing antibodies according to the invention are well known. Alternatively, peptides can be synthetically prepared based upon the amino acid sequence. For example, with respect to CD40L, this is disclosed in Armitage et al. (1992).

Preferably, the CD40L antibody or anti-CD40L antibody will be the humanized anti-CD40L antibody disclosed in U.S. Patent 6,001,358, issued on June 14,1999, and assigned to IDEC Pharmaceuticals Corporation.

While a preferred CD40L antagonist is an antibody, antagonists other than antibodies may also be administered. For example, the antagonist may comprise soluble CD40, a CD40 fusion protein or a small molecule antagonist optionally fused to, or conjugated with, a cytotoxic agent (such as those described herein). Libraries of small molecules may be screened against the B cell surface marker of interest herein in order to identify a small molecule which binds to that antigen. The small molecule may further be screened for its antagonistic properties and/or conjugated with a cytotoxic agent.

The antagonist may also be a peptide generated by rational design or by phage display (WO98/35036 published 13 August 1998), for example. In one embodiment, the molecule of choice may be a "CDR mimic" or antibody analogue designed based on the CDRs of an antibody, for example. While the peptide may be antagonistic by itself, the peptide may optionally be fused to a cytotoxic agent or to an immunoglobulin Fc region (e.g., so as to confer ADCC and/or CDC activity on the peptide).

Exemplary techniques for the production of the antibody antagonists used in accordance with the present invention are described.

Polyclonal antibodies are preferably raised in animals by multiple subcutaneous (sc) or intraperitoneal (ip) injections of the relevant antigen and an adjuvant. It may be useful to conjugate the relevant antigen to a protein that is immunogenic in the species to be immunized, e.g., keyhole limpet hemocyanin, serum albumin, bovine thyroglobulin, or soybean trypsin inhibitor using a bifunctional or derivatizing agent, for example, maleimidobenzoyl sulfosuccinimide ester (conjugation through cysteine residues), N-hydroxysuccinimide (through lysine residues), glutaraldehyde, succiic anhydride, $SOCl_2$, or $R^1N=C=NR$, where R and $R^1$ are different alkyl groups.

Animals are immunized against the antigen, immunogenic conjugates, or derivatives by combining, *e.g.* 100 $\mu$g or 5 $\mu$g of the protein or conjugate (for rabbits or mice, respectively) with 3 volumes of Freund's complete adjuvant and injecting the solution intradermally at multiple sites. One month later the animals are boosted with 1/5 to 1/10 the original amount of peptide or conjugate in Freund's complete adjuvant by subcutaneous injection at multiple sites. Seven to 14 days later the animals are bled and the serum is assayed for antibody titer. Animals are boosted until the titer plateaus. Preferably, the animal is boosted with the conjugate of the same antigen, but conjugated to a different protein and/or through a different cross-linking reagent. Conjugates also can be made in recombinant cell culture as protein fusions. Also, aggregating agents such as alum are suitably used to enhance the immune response.

Monoclonal antibodies are obtained from a population of substantially homogeneous antibodies, *i.e.,* the individual antibodies comprising the population are identical except for possible naturally occurring mutations that may be present in minor amounts. Thus, the modifier "monoclonal" indicates the character of the antibody as not being a mixture of discrete antibodies.

For example, the monoclonal antibodies may be made using the hybridoma method first described by Kohler et al., Nature, 256:495 (1975), or may be made by recombinant DNA methods (U.S. Patent No. 4,816,567).

In the hybridoma method, a mouse or other appropriate host animal, such as a hamster, is immunized as hereinabove described to elicit lymphocytes that produce or are capable of producing antibodies that will specifically bind to the protein used for immunization. Alternatively, lymphocytes may be immunized *in vitro.* Lymphocytes then are fused with myeloma cells using a suitable fusing agent, such as polyethylene glycol, to form a hybridoma cell (Goding, Monoclonal Antibodies: Principles and Practice, pp.59-103 (Academic Press, 1986)).

The hybridoma cells thus prepared are seeded and grown in a suitable culture medium that preferably contains one or more substances that inhibit the growth or survival of the unfused, parental myeloma cells. For example, if the parental myeloma cells lack the enzyme hypoxanthine guanine phosphoribosyl transferase (HGPRT or HPRT), the culture medium for the hybridomas typically will include hypoxanthine, aminopterin, and thymidine (HAT medium), which substances prevent the growth of HGPRT-deficient cells.

Preferred myeloma cells are those that fuse efficiently, support stable high-level production of antibody by the selected antibody-producing cells, and are sensitive to a medium such as HAT medium. Among these, preferred myeloma cell lines are murine myeloma lines, such as those derived from MOPC-21 and MPC-11 mouse tumors available from the Salk Institute Cell Distribution Center, San Diego, California USA, and SP-2 or X63-Ag8-653 cells available from the American Type Culture Collection, Manassas, Virginia, USA. Human myeloma and mouse-human heteromyeloma cell lines also have been described for the production of human monoclonal antibodies (Kozbor, J. ImmunoL, 133:300 1 (1984); Brodeur et al., Monoclonal Antibody Production Techniques and Applications, pp. 51-63 (Marcel Dekker, Inc., New York, 1987)).

Culture medium in which hybridoma cells are growing is assayed for production of monoclonal antibodies directed against the antigen. Preferably, the binding specificity of monoclonal antibodies produced by hybridoma cells is determined by immunoprecipitation or by an *in vitro* binding assay, such as radioimmunoassay (RIA) or enzyme-linked immunoabsorbent assay (ELISA).

The binding affinity of the monoclonal antibody can, for example, be determined by the 30 Scatchard analysis of Munson et al., Anal. Biochem., 107:220 (1980).

After hybridoma cells are identified that produce antibodies of the desired specificity, affinity, and/or activity, the clones may be subcloned by limiting dilution procedures and grown by standard methods (Goding, Monoclonal Antibodies: Principles and Practice, pp.59-103 (Academic Press, 1986)). Suitable culture media for this purpose include, for example, D-MEM or RPML-1640 medium. In addition, the hybridoma cells may be grown *in vivo* as ascites tumors in an animal.

The monoclonal antibodies secreted by the subclones are suitably separated from the culture medium, ascites fluid, or serum by conventional immunoglobulin purification procedures such as, for example, protein A-Sepharose, hydroxylapatite chromatography, gel electrophoresis, dialysis, or affinity chromatography.

DNA encoding the monoclonal antibodies is readily isolated and sequenced using conventional procedures (e.g., by using oligonucleotide probes that are capable of binding specifically to genes encoding the heavy and light chains of murine antibodies). The hybridoma cells serve as a preferred source of such DNA. Once isolated, the DNA may be placed into expression vectors, which are then transfected into host cells such as *E. coli* cells, simian COS cells, Chinese Hamster Ovary (CHO) cells, or myeloma cells that do not otherwise produce immunoglobulin protein, to obtain the synthesis of monoclonal antibodies in the recombinant host cells. Review articles on recombinant expression in bacteria of DNA encoding the antibody include Skerra et al., Curr. Opinion in Immunol., 5:256-262 (1993) and Pluckthun, Immunol. Revs., 130:151-188 (1992).

Another method of generating specific antibodies, or antibody fragments, reactive against a CD40L, CD 19, CD22, CD20, or CD40 protein or peptide (*e.g.,* such as the gp39 fusion protein described in U.S. Patent No. 5,945,513) is to screen expression libraries encoding immunoglobulin genes, or portions thereof, expressed in bacteria with a CD40L, CDC19, CD20, or CD22 protein or peptide. For example, complete Fab fragments, $V_H$ regions and Fv regions can be expressed in bacteria using phage expression libraries. See for example, Ward et al., Nature 341: 544-546 (1989); Huse et al., Science 246: 1275-1281 (1989); and McCafferty et al., Nature 348: 552-554 (1990). Screening such libraries with, for example, a CD40L, CD22, CD19, or CD20 peptide, can identify immunoglobulin fragments reactive with CD40L, CD22, CD19, or CD20. Alternatively, the SCID-hu mouse (available from Genpharm) can be used to produce antibodies or fragments thereof.

In a further embodiment, antibodies or antibody fragments can be isolated from antibody phage libraries generated using the techniques described in McCafferty et al., Nature, 348:552-554(1990). Clackson et al., Nature, 352: 624-628 (1991) and Marks al., J. Mol. Biol., 222:581-597 (1991) describe the isolation of murine and human antibodies, respectively, using phage libraries. Subsequent publications describe the production of high affinity (nM range) human antibodies by chain shuffling (Marks et al., Bio/Technology, 10:779-783 (1992)), as well as combinatorial infection and *in vivo.* recombination as a strategy for constructing very large phage libraries (Waterhouse et al., Nut. Acids. Res., 21: 2265-2266 (1993)). Thus, these techniques are viable alternatives to traditional monoclonal antibody hybridoma techniques for isolation of monoclonal antibodies.

Methodologies for producing monoclonal antibodies (MAb) directed against CD40L, including human CD40L and mouse CD40L, and suitable monoclonal antibodies for use in the methods of the invention, are described in PCT Patent Application No. WO 95/06666 entitled "Anti-gp39 Antibodies and Uses Therefor;" the teachings of which are incorporated herein by reference in their entirety. Particularly preferred anti-human CD40L antibodies of the invention are MAbs 24-31 and 89-76, produced respectively by hybridomas 24-31 and 89-76. The 89-76 and 24-31 hybridomas, producing the 89-76 and 24-31 antibodies, respectively, were deposited under the provisions of the Budapest Treaty with the American Type Culture Collection (ATCC), 10801 University Blvd., Manassas, VA 20110-2209, on Sept. 2,1994. The 89-76 hybridoma was assigned ATCC Accession Number HB11713 and the 24-31 hybridoma was assigned ATCC Accession Number HB11712.

The DNA also may be modified, for example, by substituting the coding sequence for human heavy- and light-chain constant domains in place of the homologous murine sequences (U.S. Patent No. 4,816,567; Morrison, et al., Proc. Natl Acad. ScL USA, 81:6851 (1984)), or by covalently joining to the immunoglobulin coding sequence all or part of the coding sequence for a non-immunoglobulin polypeptide.

Typically, such non-immunoglobulin polypeptides are substituted for the constant domains of an antibody, or they are substituted for the variable domains of one antigencombining site of an antibody to create a chimeric bivalent antibody comprising one antigen-combining site having specificity for an antigen and another antigen-combining site having specificity for a different antigen.

Methods for humanizing non-human antibodies have been described in the art. Preferably, a humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers (Jones et al., Nature, 321: 522-525 (1986); Reichmann et al., Nature, 332:323-327 (1988); Verhoeyen et al., Science, 239:1534-1536 (1988)), by substituting hypervariable region sequences for the corresponding sequences of a human antibody. Accordingly, such "humanized" antibodies are chimeric antibodies (U.S. Patent No. 4,816,567) wherein substantially less than an intact human variable domain has been substituted by the corresponding sequence from a non-human species. In practice, humanized antibodies are typically human antibodies in which some hypervariable region residues and possibly some FR residues are substituted by residues from analogous sites in rodent antibodies.

The choice of human variable domains, both light and heavy, to be used in making the humanized antibodies is very important to reduce antigenicity. According to the so called "best-fit" method, the sequence of the variable domain of a rodent antibody is screened against the entire library of known human variable-domain sequences. The human sequence which is closest to that of the rodent is then accepted as the human framework region (FR) for the humanized antibody (Suns et al., J Immunol., 151:2296 (1993); Chothia et al., J. Mol. Biol, 196:901 (1987)). Another method uses a particular framework region derived from the consensus sequence of all human antibodies of a particular subgroup of light or heavy chains. The same framework may be used for several different humanized antibodies (Carter et al., Proc. Natl. Acad Sci. USA, 89:4285 (1992); Presta et al., J. Immunol., 151:2623 (1993)).

It is further important that antibodies be humanized with retention of high affinity for the antigen and other favorable biological properties. To achieve this goal, according to a preferred method, humanized antibodies are prepared by a process of analysis of the parental sequences and various conceptual humanized products using three-dimensional models of the parental and humanized sequences. Three-dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, i. e., the analysis of residues that influence the ability of the candidate immunoglobulin to bind its antigen. In this way, FR residues can be selected and combined from the recipient and import sequences so that the desired antibody characteristic, such as increased affinity for the target antigen(s), is achieved. In general, the hypervariable region residues are directly and most substantially involved in influencing antigen binding.

Another highly efficient means for generating recombinant antibodies is disclosed by Newman, Biotechnology, 10: 1455-1460 (1992). More particularly, this technique results in the generation of primatized antibodies which contain monkey variable domains and human constant sequences. This reference is incorporated by reference in its entirety herein. Moreover, this technique is also described in commonly assigned U.S. Application No. 08/379,072, filed on January 25,1995, which is a continuation of U.S. Serial No. 07/912,292, filed July 10,1992, which is a continuation-in-part of U.S. Serial No. 07/856,281, filed March 23, 1992, which is finally a continuation-in-part of U.S. Serial No. 07/735,064, filed July 25, 1991. 08/379,072 and the parent application thereof all of which are incorporated by reference in their entirety herein.

This technique modifies antibodies such that they are not antigenically rejected upon administration in humans. This technique relies on immunization or cynomolgus monkeys with human antigens or receptors. This technique was developed to create high affinity monoclonal antibodies directed to human cell surface antigens.

Identification of macaque antibodies to human CD40L, CD20, CD22, CD40 or CD19 by screening of phage display libraries or monkey heterohybridomas obtained using B lymphocytes from CD40L, CD20, CD22, CD40, or CD 19 immunized monkeys can be performed using the methods described in commonly assigned U.S. Application No. 08/487,550, filed June 7,1995, incorporated by reference in its entirety herein.

Antibodies generated using the methods described in these applications have previously been reported to display human effector function, have reduced immunogenicity, and long serum half-life. The technology relies on the fact that despite the fact that cynomolgus monkeys are phylogenetically similar to humans, they still recognize many human proteins as foreign and therefore mount an immune response. Moreover, because the cynomolgus monkeys are phylogenetically close to humans, the antibodies generated in these monkeys have been discovered to have a high degree of amino acid homology to those produced in humans. Indeed, after sequencing macaque immunoglobulin light and heavy chain variable region genes, it was found that the sequence of each gene family was 85-98% homologous to its human counterpart (Newman *et al.,* 1992). The first antibody generated in this way, an anti-CD4 antibody, was 91-92% homologous to the consensus sequence of human immunoglobulin framework regions (Newman *et al.,* 1992).

As described above, the present invention relates, in part, to the use of monoclonal antibodies or primatized forms thereof which are specific to human CD40L antigen and which are capable of inhibiting CD40 signaling or inhibiting CD40/CD40L interaction. Blocking of the primary activation site between CD40 and CD40L with the identified antibodies (or therapeutically effective fragments thereof), while allowing the combined antagonistic effect on positive co-stimulation with an agnostic effect on negative signaling will be a useful therapeutic approach for intervening in relapsed forms of malignancy, especially B-cell lymphomas and leukemias. The functional activity of the identified antibodies is defined by blocking the signals of CD40 permitting it to survive and avoid IgM- or Fas-induced apoptosis.

Manufacture of monoclonal antibodies which specifically bind human CD40L, as well as primatized antibodies derived therefrom can be performed using the methods described in U.S. Patent Nos. 6,001,358 or 5,750,105, both assigned to IDEC Pharmaceuticals, Corporation, or other known methods. Preferably, such antibodies will possess high affinity to CD40L and therefore may be used as immunosuppressants which inhibit the CD40L/CD40 pathway. Similar techniques will yield monkey antibodies specific to CD20, CD19, CD22 or CD40.

Preparation of monkey monoclonal antibodies will preferably be effected by screening of phage display libraries or by preparation of monkey heterohybridomas using B lymphocytes obtained from CD40L (*e.g.* human CD40L) immunized monkeys. The human CD40 can also be from the fusion protein described in U.S. Patent No. 5,945,513.

As noted, the first method for generating anti-CD40L, CD 19, CD20, CD22 or CD40 antibodies involves recombinant phage display technology. Typically this will comprise synthesis of recombinant immunoglobulin libraries against the target, i.e., CD19. CD22, CD20, CD40, or CD40L antigen displayed on the surface of filamentous phage and selection of phage which secrete antibodies having high affinity to CD40L antigen. As noted *supra,* preferably antibodies will be selected which bind to both human CD40L and CD40. To effect such methodology, the present inventors have created a unique library for monkey libraries which reduces the possibility of recombination and improves stability.

Essentially, to adopt phage display for use with macaque libraries, this vector contains specific primers for PCR amplifying monkey immunoglobulin genes. These primers are based on macaque sequences obtained while developing the primatized technology and databases containing human sequences. Suitable primers are disclosed in commonly assigned 08/379,072, incorporated by reference herein.

The second method involves the immunization of monkeys, *i.e.,* macaques, against the desired antigen target, i.e., human CD19, CD20, CD22, CD40 or CD40L. The inherent advantage of macaques for generation of monoclonal antibodies is discussed *supra.* In particular, such monkeys, *i.e.,* cynomolgus monkeys, may be immunized against human antigens or receptors. Moreover, the resultant antibodies may be used to make primatized antibodies according to the methodology of Newman *et al.,* (1992), and Newman *et al,* commonly assigned U.S. Serial No. 08/379,072, filed January 25, 1995, which are incorporated by reference in their entirety.

The significant advantage of antibodies obtained from cynomolgus monkeys is that these monkeys recognize many human proteins as foreign and thereby provide for the formation of antibodies, some with high affinity to desired human antigens, e.g., human surface proteins and cell receptors. Moreover, because they are phylogenetically close to humans, the resultant antibodies exhibit a high degree of amino acid homology to those produced in humans. As noted above, after sequencing macaque immunoglobulin light and heavy variable region genes, it was found that the sequence of each gene family was 85-88% homologous to its human counterpart (Newman *et* al., 1992).

More particularly cynomolgus macaque monkeys are administered human, CD19, CD20, CD22, CD40, or CD40L antigen, B cells are isolated therefrom, e.g., lymph node biopsies are taken from the animals, and B lymphocytes are then fused with KH6/B5 (mouse x human) heteromyeloma cells using polyethylene glycol (PEG). Heterohybridomas secreting antibodies which bind human CD40L antigen are then identified.

In the case of antibodies which bind to CD40L or CD40, it is desirable that they do so in a manner which interrupts or regulates CD40 signaling because such antibodies potentially may be used to inhibit the interaction of CD40L with CD40, with their counter-receptors. If antibodies can be developed against more than one epitope on CD40L or CD40, and the antibodies are utilized together, their combined activity may potentially provide synergistic effects.

The disclosed invention involves the use of an animal which is primed to produce a particular antibody (*e.g.,* primates, such as organgutan, baboons, macaque, and cynomolgus monkeys). Other animals which may be used to raise antibodies to human CD40L include, but are not limited to, the following: mice, rats, guinea pigs, hamsters, monkeys, pigs, goats and rabbits.

Cell lines which express antibodies which specifically bind to human CD40L antigen are then used to clone variable domain sequences for the manufacture of primatized antibodies essentially as described in Newman *et al.,* (1992) and Newman et al., U.S. Serial No. 379,072, filed January 25,1995, both of which are incorporated by reference herein. Essentially, this entails extraction of RNA therefrom, conversion to cDNA, and amplification thereof by PCR using Ig specific primers. Suitable primers are described in Newman *et al.,* 1992, and in U.S. Serial No. 379,072. Similar techniques will yield cell lines that express antibodies specific to CD40, CD19, CD20, or CD22.

The cloned monkey variable genes are then inserted into an expression vector which contains human heavy and light chain constant region genes. Preferably, this is effected using a proprietary expression vector of IDEC, Inc., referred to as NEOSPLA. This vector contains the cytomegalovirus promoter/enhancer, the mouse beta globin major promoter, the SV40 origin of replication, the bovine growth hormone polyadenylation sequence, neomycin phosphotransferase exon 1 and exon 2, human immunoglobulin kappa or lambda constant region, the dihydrofolate reductase gene, the human immunoglobulin gamma 1 or gamma 4 PE constant region and leader sequence. This vector has been found to result in very high level expression or primatized antibodies upon incorporation of monkey variable region genes, transfection in CHO cells, followed by selection in G418 containing medium and methotrexate amplification.

For example, this expression system has been previously disclosed to result in primatized antibodies having high avidity (Kd $\leq$ 10$^{-10}$ M) against CD4 and other human cell surface receptors. Moreover, the antibodies have been found to exhibit the same affinity, specificity and functional activity as the original monkey antibody. This vector system is substantially disclosed in commonly assigned U.S. Serial No. 379,072, incorporated by reference herein as well as U.S. Serial No. 08/149,099, filed on November 3, 1993, also incorporated by reference in its entirety herein. This system provides for high expression levels, *i.e.,* > 30 pg/cell/day. Of course, the same methods can be used to produce cell lines that produce antibodies specific to CD19, CD20, CD22, or CD40.

As an alternative to humanization, human antibodies can be generated. For example, it is now possible to produce transgenic animals (e.g., mice) that are capable, upon immunization, of producing a full repertoire of human antibodies in the absence of endogenous immunoglobulin production. For example, it has been described that the homozygous deletion of the antibody heavy-chain joining region PH) gene in chimeric and germ-line mutant mice results in complete inhibition of endogenous antibody production. Transfer of the human germ-line immunoglobulin gene array in such germ line mutant mice will result in the production of human antibodies upon antigen challenge. See, e.g., Jakobovits et al., Proc. Mad. Acad. Ski. USA, 90:255 1 (1993); Jakobovits et al., Nature, 362:255-258 (1993); Bruggermann et al., Year in immuno., 7:33 (1993); and US Patent Nos. 5,591,669, 5,589,369 and 5,545,807.

Alternatively, phage display technology (McCafferty et al., Nature 348:552-553 (1990)) can be used to produce human antibodies and antibody fragments *in vitro,* from immunoglobulin variable (V) domain gene repertoires from unimmunized donors. According to this technique, antibody V domain genes are cloned in-frame into either a major or minor coat protein gene of a filamentous bacteriophage, such as M 13 or fd, and displayed as functional antibody fragments on the surface of the phage particle. Because the filamentous particle contains a single-stranded DNA copy of the phage genome, selections based on the functional properties of the antibody also result in selection of the gene encoding the antibody exhibiting those properties. Thus, the phage mimics some of the properties of the B cell. Phage display can be performed in a variety of formats; for their review see, e.g., Johnson, Kevin S. and Chiswell, David J., Current Opinion in Structural Biology 3:564-57 1 (1993). Several sources of V-gene segments can be used for phage display. Clackson et al., Nature, 352:624-628 (1991) isolated a diverse array of anti-oxazolone antibodies from a small random combinatorial library of V genes derived from the spleens of immunized mice. A repertoire of V genes from unimmunized human donors can be constructed and antibodies to a diverse array of antigens (including self antigens) can be isolated essentially following the techniques described by Marks et al., J.Mol. Biol, 222:581-597 (1991), or Griffith et al., EMBO J. 12:725-734 (1993). See, also, US Patent Nos. 5,565,332 and 5,573,905.

Human antibodies may also be generated by in *vitro* activated B cells (see US Patents 20 5,567,610 and 5,229,275). A preferred means of generating human antibodies using SCID mice is disclosed in commonly-owned, co-pending applications.

Various techniques have been developed for the production of antibody fragments. Traditionally, these fragments were derived via proteolytic digestion of intact antibodies (see, *e.g.,* Morimoto et al., Journal of Biochemical and Biophysical Methods 24:107-117 (1992) and Brennan et al., Science, 229:81 (1985)). However, these fragments can now be produced directly by recombinant host cells. For example, the antibody fragments can be isolated from the antibody phage libraries discussed above. Alternatively, Fab'-SH fragments can be directly recovered from *E. coli* and chemically coupled to form F(ab')2 fragments (Carter et al., BiolTechnology 10: 163-167 (1992)). According to another approach, F(ab')2 fragments can be isolated directly from recombinant host cell culture. Other techniques for the production of antibody fragments will be apparent to the skilled practitioner. In other embodiments, the antibody of choice is a single chain Fv fragment (scFv). See WO 93/16185; US Patent No. 5,571,894; and US Patent No. 5,587,458. The antibody fragment may also be a "linear antibody", *e.g.,* as described in US Patent 5,641,870 for example. Such linear antibody fragments may be monospecific or bispecific.

Bispecific antibodies are antibodies that have binding specificities for at least two different epitopes. Exemplary bispecific antibodies may bind to two different epitopes of the B cell surface marker. Other such antibodies may bind a first B cell marker and further bind a second B cell surface marker. Alternatively, an anti-B cell marker binding arm may be combined with an arm which binds to a triggering molecule on a leukocyte such as a T-cell receptor molecule *(e.g.* CD2 or CD3), or Fc receptors for IgG (FcyR), such as FcyRI (CD64), FcyRII (CD32) and FcyRIII (CD 16) so as to focus cellular defense mechanisms to the B cell. Bispecific antibodies may also be used to localize cytotoxic agents to the B cell. These antibodies possess a B cell marker-binding arm and an arm which binds the cytotoxic agent (*e.g.* saporin, anti-interfieron-$\alpha$, vinca alkaloid, ricin A chain, methotrexate or radioactive isotope hapten). Bispecific antibodies can be prepared as full length antibodies or antibody fragments (*e.g.* F(ab)2 bispecific antibodies).

Methods for making bi specific antibodies are known in the art. Traditional production of full length bispecific antibodies is based on the coexpression of two immunoglobulin heavy chain-light chain pairs, where the two chains have different specificities (Millstein et al., Nature, 305:537-539 (1983)). Because of the random assortment of immunoglobulin heavy and light chains, these hybridomas (quadromas) produce a potential mixture of 10 different antibody molecules, of which only one has the correct bispecific structure. Purification of the correct molecule, which is usually done by affinity chromatography steps, is rather cumbersome, and the product yields are low. Similar procedures are disclosed in WO 93/08829, and in Traunecker et al., EMBO J., 10:3655-3659(1991).

According to a different approach, antibody variable domains with the desired binding specificities (antibody-antigen combining sites) are fused to immunoglobulin constant domain sequences. The fusion preferably is with an immunoglobulin heavy chain constant domain, comprising at least part of the hinge, CH2, and CH3 regions. It is preferred to have the first heavy-chain constant region (CHI) containing the site necessary for light chain binding, present in at least one of the fusions. DNAs encoding the immunoglobulin heavy chain fusions and, if desired, the immunoglobulin light chain, are inserted into separate expression vectors, and are co-transfected into a suitable host organism. This provides for great flexibility in adjusting the mutual proportions of the three polypeptide fragments in embodiments when unequal ratios of the three polypeptide chains used in the construction provide the optimum yields. It is, however, possible to insert the coding sequences for two or all three polypeptide chains in one expression vector when the expression of at least two polypeptide chains in equal ratios results in high yields or when the ratios are of no particular significance.

In a preferred embodiment of this approach, the bispecific antibodies are composed of a hybrid immunoglobulin heavy chain with a first binding specificity in one arm, and a hybrid immunoglobulin heavy chain-light chain pair (providing a second binding specificity) in the other arm. It was found that this asymmetric structure facilitates the separation of the desired bispecific compound from unwanted immunoglobulin chain combinations, as the presence of an immunoglobulin light chain in only one half of the bispecific molecule provides for a facile way of separation. This approach is disclosed in WO 94/04690. For further details of generating bispecific antibodies see, for example, Suresh et al., Methods in Enzymology, 121:210 (1986).

According to another approach described in US Patent No. 5,731,168, the interface between a pair of antibody molecules can be engineered to maximize the percentage of heterodimers which are recovered from recombinant cell culture. The preferred interface comprises at least a part of the $C_H3$ domain of an antibody constant domain. In this method, one or more small amino acid side chains from the interface of the first antibody molecule are replaced with larger side chains (*e.g.* tyrosine or tryptophan). Compensatory "cavities" of identical or similar size to the large side chains) are created on the interface of the second antibody molecule by replacing large amino acid side chains with smaller ones (*e.g.* alanine or threonine). This provides a mechanism for increasing the yield of the heterodimer over other unwanted end-products such as homodimers.

Bispecific antibodies include cross-linked or "heteroconjugate" antibodies. For example, one of the antibodies in the heteroconjugate can be coupled to avidin, the other to biotin. Such antibodies have, for example, been proposed to target immune system cells to unwanted cells (US Patent No. 4,676,980), and for treatment of HIV infection (WO 91/00360, WO 92/200373, and EP 03089). Heteroconjugate antibodies may be made using any convenient cross-linking methods. Suitable cross-linking agents are well known in the art, and are disclosed in US Patent No. 4,676,980, along with a number of cross-linking techniques.

Techniques for generating bispecific antibodies from antibody fragments have also been described in the literature. For example, bispecific antibodies can be prepared using chemical linkage. Brennan et al., Science, 229:81 (1985) describe a procedure wherein intact antibodies are proteolytically cleaved to generate F(ab')2 fragments. These fragments are reduced in the presence of the dithiol complexing agent sodium arsenite to stabilize vicinal dithiols and prevent intermolecular disulfide formation. The Fab' fragments generated are then converted to thionitrobenzoate (TNB) derivatives. One of the Fab'-TNB derivatives is then reconverted to the Fab'-thiol by reduction with mercaptoethylamine and is mixed with an equimolar amount of the other Fab'-TNB derivative to form the bispecific antibody. The bispecific antibodies produced can be used as agents for the selective immobilization of enzymes.

Recent progress has facilitated the direct recovery of Fab'-SH fragments from *E. coli*, which can be chemically coupled to form bispecific antibodies. Shalaby et al., J.Exp. Med, 175:2 17-225 (1992) describe the production of a fully humanized bispecific antibody F(ab')2 molecule. Each Fab' fragment was separately secreted from *E. coli* and subjected to directed chemical coupling *in vitro* to form the bispecific antibody. The bispecific antibody thus formed was able to bind to cells overexpressing the ErbB2 receptor and normal human T cells, as well as trigger the lytic activity of human cytotoxic lymphocytes against human breast tumor targets.

Various techniques for making and isolating bispecific antibody fragments directly from recombinant cell culture have also been described. For example, bispecific antibodies have been produced using leucine zippers. Kostelny et al., J. Immunol.148(5):1547-1553 (1992). The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger et al., Proc.Natl. Acad. Sci. USA, 90:6444-6448 (1993) has provided an alternative mechanism for making bispecific antibody fragments. The fragments comprise a heavy-chain variable domain ($V_H$) connected to a light-chain variable domain ($V_L$)by a linker which is too short to allow pairing between the two domains on the same chain. Accordingly, the $V_H$ and $V_L$ domains of one fragment are forced to pair with the complementary $V_L$ and Van domains of another fragment, thereby forming two antigen-binding sites. Another strategy for making bispecific antibody fragments by the use of single-chain Fv (sFv) dimers has also been reported. See Gruber et al., J Immunol., 152:5368(1994).

Antibodies with more than two valencies are contemplated. For example, trispecific antibodies can be prepared. Tutt et al. J. Immunol. 147: 60(1991).

Other modifications of the antibody are contemplated herein. For example, the antibody may be linked to one of a variety of nonproteinaceous polymers, *e.g.,* polyethylene glycol, polypropylene glycol, polyoxyalkylenes, or copolymers of polyethylene glycol and polypropylene glycol.

The antibodies disclosed herein may also be formulated as liposomes. Liposomes containing the antagonist are prepared by methods known in the art, such as described in Epstein et al., Proc. Natl. Acad. Sci. USA, 82:3688 (1985); Hwang et al., Proc. Natl Acad Sci. USA, 77:4030 (1980); U.S. Pat. Nos. 4,485,045 and 4,544,545; and WO97/38731 published October 23, 1997. Liposomes with enhanced circulation time are disclosed in U.S. Patent No. 5,013,556.

Particularly useful liposomes can be generated by the reverse phase evaporation method with a lipid composition comprising phosphatidylcholine, cholesterol and PEG derivatized phosphatidylethanolamine (PEG-PE). Liposomes are extruded through filters of defined pore size to yield liposomes with the desired diameter. Fab' fragments of an antibody of the present invention can be conjugated to the liposomes as described in Martin et al. J. Biol. Chem. 257:286-288 (1982) via a disulfide interchange reaction. A chemotherapeutic agent is optionally contained within the liposome. See Gabizon et al. J.National Cancer Inst. 81(19)1484 (1989).

Amino acid sequence modification(s) of protein or peptide antagonists described herein are contemplated. For example, it may be desirable to improve the binding affinity and/or other biological properties of the antibody. Amino acid sequence variants of the antibody are prepared by introducing appropriate nucleotide changes into the antibody encoding nucleic acid, or by peptide synthesis. Such modifications include, for example, deletions from, and/or insertions into and/or substitutions of, residues within the amino acid sequences of the antagonist. Any combination of deletion, insertion, and substitution is made to arrive at the final construct, provided that the final construct possesses the desired characteristics. The amino acid changes also may alter post-translational processes of the antagonist, such as changing the number or position of glycosylation sites.

A useful method for identification of certain residues or regions of the antibody that are preferred locations for mutagenesis is called "alanine scanning mutagenesis" as described by Cunningham and Wells Science, 244:1081-1085 (1989*)*. Here, a residue or group of target residues are identified (*e.g.*, charged residues such as arg, asp, his, lys, and glu) and replaced by a neutral or negatively charged amino acid (most preferably alanine or polyalanine) to affect the interaction of the amino acids with antigen. Those amino acid locations demonstrating functional sensitivity to the substitutions then are refined by introducing further or other variants at, or for, the sites of substitution. Thus, while the site for introducing an amino acid sequence variation is predetermined, the nature of the mutation per se need not be predetermined. For example, to analyze the performance of a mutation at a given site, ala scanning or random mutagenesis is conducted at the target codon or region and the expressed antagonist variants are screened for the desired activity.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antagonist with an N-terminal methionyl residue or the antagonist fused to a cytotoxic polypeptide. Other insertional variants of the antagonist molecule include the fusion to the N- or C-terminus of the antagonist of an enzyme, or a polypeptide which increases the serum half-life of the antagonist.

Another type of variant is an amino acid substitution variant. These variants have at least one amino acid residue in the antagonist molecule replaced by different residue. The sites of greatest interest for substitutional mutagenesis of antibody antagonists include the hypervariable regions, but FR alterations are also contemplated. Conservative substitutions are shown in Table 1 under the heading of "preferred substitutions". If such substitutions result in a change in biological activity, then more substantial changes, denominated "exemplary substitutions" in Table 1, or as further described below in reference to amino acid classes, may be introduced and the products screened.

Table 1

| Original Residue | Exemplary Substitutions | Preferred Substitutions |
|---|---|---|
| Ala (A) | val; leu; ile | val |
| Arg (R) | lys; gin; asn | lys |

(continued)

| Original Residue | Exemplary Substitutions | Preferred Substitutions |
|---|---|---|
| Asn (N). | gln; his; asp, lys; arg | gln |
| Asp (D) | glu; asn | glu |
| Cys (C) | ser; ala | ser |
| Gln (Q | asn; glu | asn |
| Glu (E) | asp; gin | asp |
| Gly (G) | ala | ala |
| His (H) | asn; gin; lys; arg | arg |
| Ile (I) | leu; val; met; ala; phe; norleucine | leu |
| Leu (L) | norleucine; ile; val; met; ala; phe | ile |
| Lys (K) | arg; gln; asn | arg |
| Met(M) | leu; phe; ile | leu |
| Phe (F) | leu; val; ile; ala; tyr | tyr |
| Pro (P) | ala | ala |
| Ser (S) | thr | thr |
| Thr (T) | ser | ser |
| Trp (W) | tyr;phe | tyr |
| Tyr (Y) | trp; phe; thr; ser | phe |
| Val (V) | ile; leu; met; phe; ala; norleucine | leu |

Substantial modifications in the biological properties of the antibody are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Naturally occurring residues are divided into groups based on common side-chain properties:

    (1) hydrophobic: norleucine, met, ala, val, leu, ile;
    (2) neutral hydrophiuic: cys, ser, thr;
    (3) acidic: asp, glu;
    (4) basic: asn, gln, his, lys, arg;
    (5) residues that influence chain orientation: gly, pro; and
    (6) aromatic: trp, tyr, phe.

Non-conservative substitutions will entail exchanging a member of one of these classes for another class.

Any cysteine residue not involved in maintaining the proper conformation of the antagonist also may be substituted, generally with serine, to improve the oxidative stability of the molecule and prevent aberrant crosslinking, Conversely, cysteine bonds) may be added to the antagonist to improve its stability (particularly where the antagonist is an antibody fragment such as an Fv fragment).

A particularly preferred type of substitutional variant involves substituting one or more hypervariable region residues of a parent antibody (e.g. a humanized or human antibody). Generally, the resulting variants selected for further development will have improved biological properties relative to the parent antibody from which they are generated. A convenient way for generating such substitutional variants is affinity maturation using phage display. Briefly, several hypervariable region sites (e.g. 6-7 sites) are mutated to generate all possible amino substitutions at each site. The antibody variants thus generated are displayed in a monovalent fashion from filamentous phage particles as fusions to the gene III product of M13 packaged within each particle. The phage-displayed variants are then screened for their biological activity (e.g. binding affinity) as herein disclosed. In order to identify candidate hypervariable region sites for modification, alanine scanning mutagenesis can be performed to identified hypervariable region residues contributing significantly to antigen binding. Alternatively, or in addition, it may be beneficial to analyze a crystal structure of the antigen-antibody complex to identify contact points between the antibody and antigen. Such contact residues and neighboring residues are candidates for substitution according to the techniques elaborated herein. Once such variants are generated, the panel of variants is subjected to screening as described herein and antibodies with superior properties in one or more relevant assays may be selected for further development.

Another type of amino acid variant of the antibody alters the original glycosylation pattern of the antagonist. By altering is meant deleting one or more carbohydrate moieties found in the antagonist, and/or adding one or more glycosylation sites that are not present in the antagonist.

Glycosylation of polypeptides is typically either N-linked or O-linked. N-linked refers to the attachment of the carbohydrate moiety to the side chain of an asparagine residue. The tripeptide sequences asparagine-X-serine and asparagine- X-threonine, where X is any amino acid except proline, are the recognition sequences for enzymatic attachment of the carbohydrate moiety to the asparagine side chain. Thus, the presence of either of these tripeptide sequences in a polypeptide creates a potential glycosylation site. O-linked glycosylation refers to the attachment of one of the sugars N-aceylgalactosamine, galactose, or xylose to a hydroxyamino acid, most commonly seine or threonine, although 5-hydroxyproline or 5-hydroxylysine may also be used.

Addition of glycosylation sites to the antibody is conveniently accomplished by altering the amino acid sequence such that it contains one or more of the above-described tripeptide sequences (for N-linked glycosylation sites). The alteration may also be made by the addition of, or substitution by, one or more seine or threonine residues to the sequence of the original antagonist (for O-linked glycosylation sites).

Nucleic acid molecules encoding amino acid sequence variants of the antibody are prepared by a variety of methods known in the art. These methods include, but are not limited to, isolation from a natural source (in the case of naturally occurring amino acid sequence variants) or preparation by oligonucleotide-mediated (or site-directed) mutagenesis, PCR mutagenesis, and cassette mutagenesis of an earlier prepared variant or a non-variant version of the antagonist.

It may be desirable to modify the antibodies used in the invention to improve effector function, e.g. so as to enhance antigen-dependent cell-mediated cyotoxicity (ADCC) and/or complement dependent cytotoxicity (CDC) of the antagonist. This may be achieved by introducing one or more amino acid substitutions in an Fc region of an antibody antagonist. Alternatively or additionally, cysteine residue(s) may be introduced in the Fc region, thereby allowing interchain disulfide bond formation in this region. The homodimeric antibody thus generated may have improved internalization capability and/or increased complement- mediated cell killing and antibody-dependent cellular cytotoxicity (ADCC). See Caron et al., J Exp Med. 176:1191-1195 (1992) and Shopes, B. J. Immunol. 148:2918-2922 (1992). Homodimeric antibodies with enhanced anti-tumor activity may also be prepared using heterobifunctional cross-linkers as described in Wolff et al. Cancer Research 53:2560-2565 (1993). Alternatively, an antibody can be engineered which has dual Fc regions and may thereby have enhanced complement lysis and ADCC capabilities. See Stevenson et al. Anti-Cancer Drug Design 3:2 19-230 (1989).

To increase the serum half life of the antibody, one may incorporate a salvage receptor binding epitope into the antagonist (especially an antibody fragment) as described in US Patent 5,739,277, for example. As used herein, the term "salvage receptor binding epitope" refers to an epitope of the Fc region of an IgG molecule (e.g., IgGI, IgG2, IgG3, or IgG4) that is responsible for increasing the in vivo serum half-life of the IgG molecule.

Therapeutic formulations comprising antagonists used in accordance with the present invention are prepared for storage by mixing an antagonist having the desired degree of purity with optional pharmaceutically acceptable carriers, excipients or stabilizers *(Remington 's Pharmaceutical Sciences 16th edition, Osol, A. Ed. (1980))*, in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as TWEEN™, PLURONICS™ or polyethylene glycol (PEG).

The immunomodulatory antibody and the B cell depleting antibody may be in the same formulation or may be administered in difficult formulations. The composition may further include other non-antibody antagonists, e.g., CD40L or B7 antagonists. Examples there of include soluble CD40, B7 and fusions thereof. Administration can be concurrent or sequential, and may be effective in either order.

Exemplary anti-CD20 antibody formulations are described in W098/56418, expressly incorporated herein by reference. This publication describes a liquid multidose formulation comprising 40 mg/mL rituximab, 25 mM acetate, 150 mM trehalose, 0.9% benzyl alcohol, 0.02% polysorbate 20 at pH 5.0 that has a minimum shelf life of two years storage at 2-8°C. Another anti-CD20 formulation of interest comprises 1 Omg/mL rituximab in 9.0 mg/mL sodium chloride, 7.35 mg/mL sodium citrate dihydrate, 0.7mg/niL polysorbate 80, and Sterile Water for Injection, pH 6.5.

Lyophilized formulations adapted for subcutaneous administration are described in W097/04801 Such lyophilized formulations may be reconstituted with a suitable diluent to a high protein concentration and the reconstituted formulation may be administered subcutaneously to the mammal to be treated herein.

The formulation herein may also contain more than one active compound as necessary for the particular indication being treated, preferably those with complementary activities that do not adversely affect each other. For example, it may be desirable to further provide a chemotherapeutic agent, cytokine or immunosuppressive agent (e.g. one which acts on T cells, such as cyclosporin or an antibody that binds T cells, *e.g.* one which binds LFA-1). The effective amount of such other agents depends on the amount of antagonist present in the formulation, the type of disease or disorder or treatment, and other factors discussed above. These are generally used in the same dosages and with administration routes as used hereinbefore or about from 1 to 99% of the heretofore employed dosages.

The active ingredients may also be entrapped in microcapsules prepared, for example, by 30 coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

Sustained-release preparations may be prepared. Suitable examples of sustained release preparations include semipermeable matrices of solid hydrophobic polymers containing the antagonist, which matrices are in the form of shaped articles, *e.g.* films, or microcapsules. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and γ ethyl-L-glutamate, noir degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid. The formulations to be used for in vivo administration must be sterile. This is readily accomplished by filtration through sterile filtration membranes.

A composition comprising B cell depleting antibody and/or an immunoregulatory antibody will be formulated, dosed, and administered in a fashion consistent with good medical practice. Factors for consideration in this context include the particular malignancy or disorder being treated, the particular mammal being treated, the clinical condition of the individual patient, the cause of the disease or disorder, the site of delivery of the agent, the method of administration, the scheduling of administration, and other factors known to medical practitioners. The therapeutically effective amount of the antibodies to be administered will be governed by such considerations.

As discussed extensively above, selected embodiments of the invention comprise the administration of antibodies to patients or in combination or conjunction with one or more adjunct therapies such as radiotherapy or chemotherapy (i.e. a combined therapeutic regimen). As used herein, the administration of antibodies in conjunction or combination with another selected antibody or an adjunct therapy means the sequential, simultaneous, coextensive, concurrent, concomitant or contemporaneous administration or application of the the disclosed antibodies and/or therapy. Those skilled in the art will appreciate that the administration or application of the various components of the combined therapeutic regimen may be timed to enhance the overall effectiveness of the treatment. For example, an immunomodulatory antibody could be administered in standard, well known courses of treatment followed within a few weeks by a B cell depleting antibody of the present invention. Conversely, cytotoxin associated B cell depleting antibodies could be administered intravenously followed by tumor localized external beam radiation. In yet other embodiments, the immunoregulatory antibody or antibodies may be administered concurrently with one or more selected B cell depleting antibodies in a single office visit. A skilled artisan (e.g. an experienced oncologist) would be readily be able to discern effective combined therapeutic regimens without undue experimentation based on the selected antibodies and the teachings of the instant specification.

In this regard it will be appreciated that the selected combination of antibodies may be administered in any order and within any time frame that provides a therapeutic benefit to the patient. That is, the immunoregulatory antibodies and, optionally, the B cell depleting antibody may be administered in any order or concurrently. In selected embodiments the immunoregulatory antibodies of the present invention will be administered to patients that have previously undergone B cell depletion. In yet other embodiments selected immunoregulatory antibodies (e.g. anti-B7 and anti-CD40L) will be administered substantially simultaneously or concurrently. In preferred embodiments the selected antibodies (whether immunoregulatory or B cell depleting) will be administered within 1 year of each other. In other preferred embodiments the selected antibodies will be administered within 10, 8, 6, 4, or 2 months of each other. In still other preferred embodiments the selected antibodies will be administered within 4, 3, 2 or 1 week of each other. In yet other embodiments the selected antibodies will be administered within 5, 4, 3, 2 or 1 day of each other. It will further be appreciated that the selected agents or treatments may be administered to the patient within a matter of hours or minutes (i.e. substantially simultaneously).

As a general proposition, the therapeutically effective amount of an antibody administered parenterally per dose will typically be in the range of about 0.1 to 500 mg/kg of patient body weight per day, with the typical initial range of antagonist used being in the range of about 2 to 100 mg/kg.

The preferred B cell depleting antibody is RITUXANS®. Suitable dosages for such antibody are, for example, in the range from about 20mg/m2 to about 1000mg/m2. The dosage of the antibody may be the same or different from that presently recommended for RITUXAN® for the treatment of non-Hodgkin's lymphoma. For example, one may administer to the patient one or more doses of substantially less than 375mg/m2 of the antibody, e.g. where the dose is in the range from about $20mg/m^2$ to about $250mg/m^2$, for example from about $50mg/m^2$ to about $200mg/m^2$.

Moreover, one may administer one or more initial doses) of the antibody followed by one or more subsequent dose(s), wherein the mg/m$^2$ dose of the antibody in the subsequent doses) exceeds the mg/m$^2$ dose of the antibody in the initial dose(s). For example, the initial dose may be in the range from about $20mg/m^2$ to about $250mg/m^2$ *(e.g.* from about $50mg/m^2$ to about $200mg/m^2$) and the subsequent dose may be in the range from about $250mg/m^2$ to about $1000mg/m^2$.

As noted above, however, these suggested amounts of both immunoregulatory and B cell depleting antibody are subject to a great deal of therapeutic discretion. The key factor in selecting an appropriate dose and scheduling is the result obtained, as indicated above. For example, relatively higher doses may be needed initially for the treatment of ongoing and acute diseases. To obtain the most efficacious results, depending on the particular B cell malignancy, the antagonist is administered as close to the first sign, diagnosis, appearance, or occurrence of the disease or disorder as possible or during remissions of the disease or disorder.

The antibodies are administered by any suitable means, including parenteral, subcutaneous, intraperitoneal, intrapulmonary, and intranasal, and, if desired for local immunosuppressive treatment, intralesional administration. Parenteral infusions include intramuscular, intravenous, intraarterial, intraperitoneal, or subcutaneous administration. In addition, the antibody may suitably be administered by pulse infusion, e.g., with declining doses of the antibody. Preferably the dosing is given by injections, most preferably intravenous or subcutaneous injections, depending in part on whether the administration is brief or chronic.

One additionally may administer other compounds, such as chemotherapeutic agents, immunosuppressive agents and/or cytokines with the antibodies herein. The combined administration includes co-administration, using separate formulations or a single pharmaceutical formulation, and consecutive administration in either order, wherein preferably there is a time period while both (or all) active agents simultaneously exert their biological activities.

Aside from administration of antibodies to the patient the present application contemplates administration of antibodies by gene therapy. Such administration of nucleic acid encoding the antibodies is encompassed by the expression "administering a therapeutically effective amount of an antagonist". See, for example, W096/07321 published March 14, 1996 concerning the use of gene therapy to generate intracellular antibodies.

There are two major approaches to getting the nucleic acid (optionally contained in a vector) into the patient's cells; in vivo and ex vivo. For in vivo delivery the nucleic acid is injected directly into the patient, usually at the site where the antagonist is required. For ex vivo treatment, the patient's cells are removed, the nucleic acid is introduced into these isolated cells and the modified cells are administered to the patient either directly or, for example, encapsulated within porous membranes which are implanted into the patient (see, e.g. U.S. Patent Nos. 4,892,538 and 5,283,187). There are a variety of techniques available for introducing nucleic acids into viable cells. The techniques vary depending upon whether the nucleic acid is transferred into cultured cells *in vitro,* or in vivo in the cells of the intended host. Techniques suitable for the transfer of nucleic acid into mammalian cells *in vitro* include the use of liposomes, electroporation, microinjection, cell fusion, DEAF-dextran, the calcium phosphate precipitation method, etc. A commonly used vector for ex vivo delivery of the gene is a retrovirus.

The currently *preferred in vivo* nucleic acid transfer techniques include transfection with viral vectors (such as adenovirus, Herpes simplex I virus, or adenoassociated virus) and lipid-based systems (useful lipids for lipid-mediated transfer of the gene are DOTMA, DOPE and DC-Chol, for example). In some situations it is desirable to provide the nucleic acid source with an agent that targets the target cells, such as an antibody specific for a cell surface membrane protein or the target cell, a ligand for a receptor on the target cell, etc. Where liposomes are employed, proteins which bind to a cell surface membrane protein associated with endocytosis may be used for targeting and/or to facilitate uptake, e.g. capsid proteins or fragments thereof tropic for a particular cell type, antibodies for proteins which undergo internalization in cycling, and proteins that target intracellular localization and enhance intracellular half-life. The technique of receptor-mediated endocytosis is described, for example, by Wu et al., . .l. Biol. Chem 262:4429-4432 (1987); and Wagner et al., Pro. Natl. Acad. Sci. USA 87:3410-3414(1990). For review of the currently known gene marking and gene therapy protocols see Anderson et al., Science 256:808-8 13 (1992). See also WO 93/25673 and the references cited therein.

As previously discussed, the antibodies of the present invention, immunoreactive fragments or recombinants thereof may be administered in a pharmaceutically effective amount for the *in vivo* treatment of mammalian malignancies. In this regard, it will be appreciated that the disclosed antibodies will be formulated so as to facilitate administration and promote stability of the active agent. Preferably, pharmaceutical compositions in accordance with the present invention comprise a pharmaceutically acceptable, non-toxic, sterile carrier such as physiological saline, non-toxic buffers, preservatives and the like. For the purposes of the instant application, a pharmaceutically effective amount of the therapeutic antibody, immunoreactive fragment or recombinant thereof, conjugated or unconjugated to a cytotoxic agent, shall be held to mean an amount sufficient to achieve effective binding with selected immunoreactive antigens on neoplastic cells and provide for an increase in the death of those cells. Of course, the pharmaceutical compositions of the present invention may be administered in single or multiple doses to provide for a pharmaceutically effective amount of the modified antibody.

More specifically, they the disclosed antibodies and methods should be useful for reducing tumor size, inhibiting tumor growth and/or prolonging the survival time of tumor-bearing animals. Accordingly, this invention also relates to a method of treating tumors in a human or other animal by administering to such human or animal an effective, non-toxic amount of at least one immunregulatory antibody and, optionally, at least one B cell depleting antibody. One skilled in the art would be able, by routine experimentation, to determine what an effective, non-toxic amount of modified antibody would be for the purpose of treating malignancies. For example, a therapeutically active amount of a modified antibody may vary according to factors such as the disease stage (e.g., stage I versus stage IV), age, sex, medical complications (*e.g.,* immunosuppressed conditions or diseases) and weight of the subject, and the ability of the antibody to elicit a desired response in the subject. The dosage regimen may be adjusted to provide the optimum therapeutic response. For instance, several divided doses may be administered daily, or the dose may be proportionally reduced as indicated by the exigencies of the therapeutic situation. Generally, however, an effective dosage is expected to be in the range of about 0.05 to 100 milligrams per kilogram body weight per day and more preferably from about 0.5 to 10, milligrams per kilogram body weight per day.

In keeping with the scope of the present disclosure, the antibodies of the invention may be administered to a human or other animal in accordance with the aforementioned methods of treatment in an amount sufficient to produce such effect to a therapeutic or prophylactic degree. The antibodies of the invention can be administered to such human or other animal in a conventional dosage form prepared by combining the antibody of the invention with a conventional pharmaceutically acceptable carrier or diluent according to known techniques. It will be recognized by one of skill in the art that the form and character of the pharmaceutically acceptable carrier or diluent is dictated by the amount of active ingredient with which it is to be combined, the route of administration and other well-known variables. Those skilled in the art will further appreciate that a cocktail comprising one or more species of monoclonal antibodies according to the present invention may prove to be particularly effective.

Methods of preparing and administering conjugates of the antibody, immunoreactive fragments or recombinants thereof, and a therapeutic agent are well known to or readily determined by those skilled in the art. The route of administration of the antibody (or fragment thereof) of the invention may be oral, parenteral, by inhalation or topical. The term parenteral as used herein includes intravenous, intraarterial, intraperitoneal, intramuscular, subcutaneous, rectal or vaginal administration. The intravenous, intraarterial, subcutaneous and intramuscular forms of parenteral administration are generally preferred. While all these forms of administration are clearly contemplated as being within the scope of the invention, a preferred administration form would be a solution for injection, in particular for intravenous or intraarterial injection or drip. Usually, a suitable pharmaceutical composition for injection may comprise a buffer (e.g. acetate, phosphate or citrate buffer), a surfactant (e.g. polysorbate), optionally a stabilizer agent (e.g. human albumine), etc. However, in other methods compatible with the teachings herein, the antibodies can be delivered directly to the site of the malignancy site thereby increasing the exposure of the neoplastic tissue to the therapeutic agent.

Preparations for parenteral administration includes sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. In the subject invention, pharmaceutically acceptable carriers include, but are not limited to, 0.01-0.1M and preferably 0.05M phosphate buffer or 0.8% saline. Other common parenteral vehicles include sodium phosphate solutions, Ringer's dextrose, dextrose and sodium chloride, lactated Ringer's, or fixed oils. Intravenous vehicles include fluid and nutrient replenishers, electrolyte replenishers, such as those based on Ringer's dextrose, and the like. Preservatives and other additives may also be present such as for example, antimicrobials, antioxidants, chelating agents, and inert gases and the like.

More particularly, pharmaceutical compositions suitable for injectable use include sterile aqueous solutions (where water soluble) or dispersions and sterile powders for the extemporaneous preparation of sterile injectable solutions or dispersions. In such cases, the composition must be sterile and should be fluid to the extent that easy syringability exists. It should be stable under the conditions of manufacture and storage and will preferably be preserved against the contaminating action of microorganisms, such as bacteria and fungi. The carrier can be a solvent or dispersion medium containing, for example, water, ethanol, polyol (*e.g.,* glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants.

Prevention of the action of microorganisms can be achieved by various antibacterial and antifungal agents, for example, parabens, chlorobutanol, phenol, ascorbic acid, thimerosal and the like. In many cases, it will be preferable to include isotonic agents, for example, sugars, polyalcohols, such as mannitol, sorbitol, or sodium chloride in the composition. Prolonged absorption of the injectable compositions can be brought about by including in the composition an agent which delays absorption, for example, aluminum monostearate and gelatin.

In any case, sterile injectable solutions can be prepared by incorporating an active compound (*e.g.,* a modified antibody by itself or in combination with other active agents) in the required amount in an appropriate solvent with one or a combination of ingredients enumerated herein, as required, followed by filtered sterilization. Generally, dispersions are prepared by incorporating the active compound into a sterile vehicle, which contains a basic dispersion medium and the required other ingredients from those enumerated above. In the case of sterile powders for the preparation of sterile injectable solutions, the preferred methods of preparation are vacuum drying and freeze-drying, which yields a powder of an active ingredient plus any additional desired ingredient from a previously sterile-filtered solution thereof.

The preparations for injections are processed, filled into containers such as ampoules, bags, bottles, syringes or vials, and sealed under aseptic conditions according to methods known in the art. Further, the preparations may be packaged and sold in the form of a kit such as those described in co-pending U.S.S.N. 09/259,337 and U.S.S.N. 09/259,338 each of which is incorporated herein by reference. As whole, the article of manufacture or kit may comprise one or several compositions. At least one active agent in one of those compositions is an antibody having immunoregulatory activity such as an anti-CD40L, anti-CD40, anti-CD23, anti-CD4 or anti-B7 antibody. It may further include other materials desirable from a commercial and user standpoint, including other buffers, diluents, filters, needles, and syringes. Such articles of manufacture will preferably have labels, instructions or package inserts indicating that the associated compositions are useful for treating a subject suffering from, or predisposed to, a cancer, a malignancy or neoplastic disorders. In preferred embodiments the instructions or labels will indicate that the cancer or malignancy is a B cell neoplasm.

Further details of the invention are illustrated by the following non-limiting Examples. The disclosures of all citations in the specification are expressly incorporated herein by reference.

## EXAMPLES

*Example 1*

*Properties of B lymphoma cells. DHT-4 cells*

The concept that anti-CD40L antibody could block CD40L-CD40 mediated survival of malignant B-cells from chemotherapy induced toxicity/apoptosis was tested *in vitro* using IDEC-131, and the B-lymphoma cell line, DHL-4 (Roos et al., Leuk Res. 10: 195-202 (1986)) exposed to adriamycin (ADM). IDEC-131 is a humanized version of the murine, monoclonal anti-human CD40L antibody, 24-31.

Initially, the minimum concentration of ADM cytotoxic to DHL-4 cells was determined by exposing DHL-4 cells for 4 hours to different concentrations of ADM. The cell cytotoxicity of DHL-4 cells after 5 days in culture was measured by Alamar Blue, a dye-reduction assay by live cells (see Gazzano-Santoro et al., J. Immunol. Meth. 202: 163-171 (1997)). Briefly, $1 \times 10^5$ DHL-4 cells in growth medium

(RMPI- 1640 plus 10% Fetal Calf Serum) were incubated with varying concentrations of ADM ($1 \times 10^{-6}$ M to $1 \times 10^{-8}$ M) in cell culture tubes at 37 ° C. for 4 hours. After incubation, cells were washed, re-suspended in growth medium at $1 \times 10^5$ cells/ml concentration and 200 $\mu$l of cell suspension was added to each well of 96-well flat-bottom plate. Plates were incubated at 37 °C. and tested for cytotoxicity at different time points. During the last 18 hours of incubation, 50 $\mu$l of redox dye Alamar Blue (Biosource International, Cat. #DAL 1100) was added to each well. Following incubation, plates were cooled by incubating at room temperature for 10 minutes on a shaker, and the intracellular reduction of the dye was determined. Fluorescence was read using a 96-well fluorometer with excitation at 530 nm and emission at 590 nm. The results are expressed as relative fluorescence units (RFU). The percentage of cytotoxicity was calculated as follows:

$$[1- (\text{average RFU of test sample} \div \text{Average RFU of control cells})] \times 100\%.$$

Titration curve of ADM cytotoxicity was established and minimal concentrations of the drug for cytotoxicity was selected for subsequent assays.

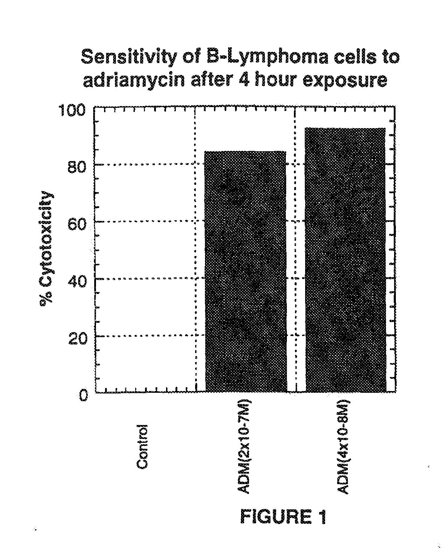

The results, as displayed in Fig. 1, shows cell cytotoxicity of DHL-4 cells cultured for 5 days after being exposed to ADM ($2 \times 10^{-7}$ M and $4 \times 10^{-8}$ M of ADM) for 4 hours prior to culture. Cells were washed once after exposure and cultured in growth medium for 5 days and cytotoxicity determined by Alamar Blue dye-uptake assay, as described above. Additionally, the DHL-4 cells were characterized for the membrane expression of selected CD molecules by flow cytometry. DHL-4 cells have been found to express CD19, CD20, CD40 molecules, but no expression of CD40L was detected.

*Example 2*

*Anti -CD40L antibody overrides CD40L mediated resistance to killing by to killing, by adriamy in of-lymphoma cells*

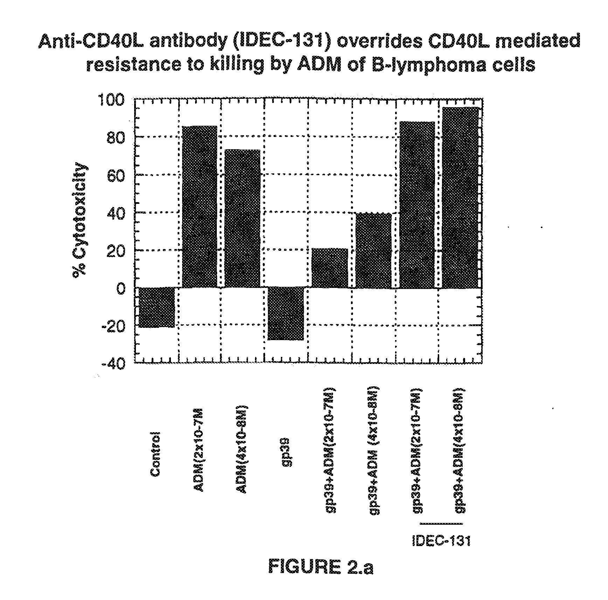

Fig. 2A shows the effect of an anti-CD40L antibody on CD40L-CD40 mediated resistance of DHL-4 cells to cell death induced by ADM. DHL-4 cells ($0.5 \times 10^6$ cells/ml) were incubated in the presence of 10 $\mu$g/ml of soluble CD40L (sCD40L, P. A. Brams, E. A. Padlan, K. Hariharan, K. Slater, J. Leonard, R. Noelle, and R. Newman, "A humanized anti-human CD 154 monoclonal antibody blocks CD 154-CD40 mediated human B cell activation," *(manuscript submitted)* for 1 hour at 37°C. After 1 hour of incubation, low concentrations of ADM ($2 \times 10^{-7}$M - $4 \times 10^{-8}$ M) were added and incubated for another 4 hours in the presence or absence of CD40L (10 $\mu$g/ml). Following exposure to ADM, cells were washed and resuspended in growth medium at $0.5 \times 10^6$ cells/ml concentration, and 100 $\mu$l of cell suspension added to each well of 96-well flat bottom plate, in duplicate, with or without sCD40L. sCD40L (10 $\mu$g/ml) was added to cultures that have been continuously exposed to sCD40L during ADM treatment and to cultures that had no sCD40L during ADM exposure. In addition, IDEC-131 at 10 $\mu$g/ml was added to cultures to determine its effect on DHL-4 cells incubated with sCD40L and ADM. After 5 days, the cytotoxicity was measured by Alamar Blue dye-uptake assay, as described.

Data show that sCD40L prolonged survival of DHL-4 cells after ADM treatment, whereas, as expected, increased cytotoxicity was observed in cells that were exposed to ADM in the absence of sCD40L. Furthermore, addition of anti-CD40L antibody (IDEC-131) reversed CD40L mediated cell survival, leading to increase in cell cytotoxicity (Fig. 2A).

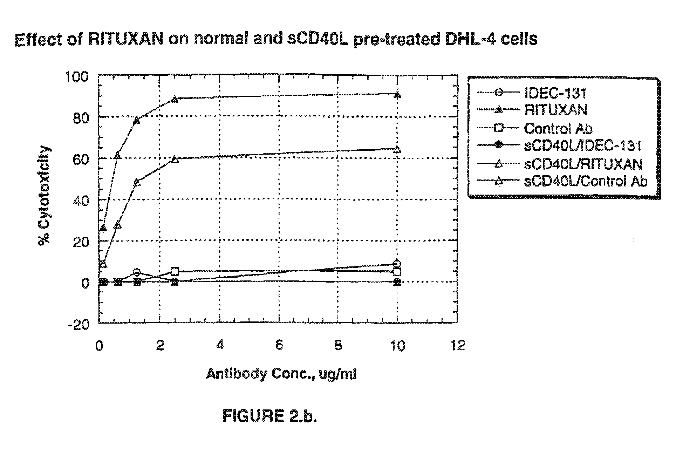

The addition of IDEC-131 alone had no effect on DHL-4 cells treated with sCD40L, which indicates that the antibody, by itself, does not have any direct inhibitory or cytotoxic activities on DHL-4 cells (Fig. 2B). DHL-4 cells pre-incubated with and without sCD40L were cultured in the presence of different concentrations of IDEC-131, RITUXAN®, the anti-CD20 antibody CE9.1, and anti-CD4 antibodies (Anderson et al., Clin. Immunol. & Immunopathol. 84: 73-84 (1997)). After 5 days, the cytotoxicity/proliferation of DHL-4 cells was determined by Alamar Blue assay, as described above. Fig. 2B shows no effect on the proliferation or the cytotoxicity of DHL-4 cells by IDEC-131, whereas RITUXAN®, as expected, inhibited cell proliferation and induced cytotoxicity. No effect was seen in the DHL-4 cells cultured with anti-CD4 antibodies.

*Example 3*

*CD40L-CD40 signaling prevents apoptosis of B-imphoma cells by anti-CD20 antibody. RITUXAN®*

The effect of CD40L-CD40 mediated signaling on anti-CD20 antibody induced apoptosis of B-lymphoma cells was determined using an *in vitro* system involving DHL-4 cells and the surface cross-linking of RITUXAN®. DHL-4 cells (0.5 to $1 \times 10^6$ cells/ml) were cultured with sCD40L (10 $\mu$g/ml) at 37° C. After overnight culture. cells were harvested and incubated with 10 $\mu$g/ml of RITUXAN® or the control antibody (CE9.1; an anti-CD4 antibody) with or without sCD40L (10 $\mu$g/ml) on ice. After 1 hour of incubation, cells were centrifuged to remove unbound antibodies, and resuspended at $1 \times 10^6$ cells/ml in growth medium (5% FCS-RPMI) and cultured in tissue culture tubes. The cells surface bound antibodies were cross-linked by spiking F(ab')$_2$ fragments of goat anti-human Ig-Fc$\gamma$ specific antibodies at 15 $\mu$g/ml, and the cultures were incubated at 37° C. until assayed for apoptosis. Apoptosis was detected using a flow cytometry caspase-3 assay. Cultured cells were harvested at 4 and 24 hours, washed and fixed at 4° C. using Cytofix (Cytofix/Cytoperm™ Kit, Pharmingen Cat. #2075KK). After 20 min of fixation, cells were washed and 15 $\mu$l of affinity purified PE-conjugated polyclonal rabbit anti-caspase-3 antibody (Pharmingen. Cat. # 67345) and 50 $\mu$l of cytoperm (Pharmingen; Cat. #2075KK) were added. Cells were incubated on ice in the dark for 30 min. After incubation cells were washed once and resuspended in cytoperm. Flow cytometry data was acquired on FACScan and analyzed using WinList software from Verity Software House.

Table I shows resistance of RITUXAN® induced apoptosis in DHL-4 lymphoma cells by exposure to sCD40L. In these studies, activation of caspase-3 was used as the surrogate marker since our previous studies revealed good correlation between caspase-3 and Tunel assay. Cross-linking of RITUXAN® on the DHL-4 cell surface in the presence of sCD40L decreased levels of apoptosis, whereas cells not exposed to sCD40L apoptosed. In comparison, cultures incubated in the presence of an antibody of the same isotype, control antibody (CE9.1), resulted in no apoptosis of the cells. Thus, the data suggests that sCD40L induced signaling of CD40 pathway can lead to development of RITUXAN® mediated killing of B-lymphoma cells.

**Table 1:**

| Resistance of RITUXAN® mediated apoptosis of DHL-4 cells by sCD40L | | |
|---|---|---|
| **Culture Conditions** | **% Apoptosis (IVHF)[a]** | |
| | **4 Hours** | **24 Hours** |
| DHT-4 cells exposed to sCD40L | | |
| Cells only | 3.35 (17.42) | 4.94 (7.62) |
| Cells + RITUXAN | 1.97 (1.97) | 4.54 (6.54) |
| Cells + RITTJXAN + anti-hu.IgG.F(ab')$_2$ | 21.17(17.39) | 9.62(13.44) |
| Cells + CE9.1 | 2.31 (13.25) | 4.15 (7.85) |
| Cells + CE9.1 + anti.hu.IgG.F(ab')$_2$ | 2.09 (22.14) | 4.14(9.57) |
| Cells + anti-hu.IgG.F(ab')$_2$ | 1.93 (12.57) | 5.13 (8.02) |
| DHL-4 cells not exposed to sCD40L | | |
| Cells only | 4.36(14.34) | 5.08(17.62) |
| Cells+RITUXAN | 5.67(10.66). | 1.08(17.92) |
| Cells + RITUXAN + anti-hu.IgG.F(ab')$_2$ | 74.82 (22.80) | 30.63 (26.84) |
| Cells + CE9.1 | 5.99(14.00) | 3.05(18.24) |
| Cells + CE9.1 + anti-hu.I-G.F(ab')$_2$ | 5.96 (12.11) | 2.24(18.19) |
| Cells+anti-hu.IgG.F(ab')$_2$ | 6.09 (12.27) | 1.85(17.27) |
| [a] Percent positive cells with caspase-3 activity and its mean fluorescent intensity in log scale. | | |

*Example 4*

*Effect of IDEC-131 on the survival of chronic lymphocytic leukemia (CLL) cells*

To determine the effect of IDEC-131 on the growth and survival of B-CLL cells *in vitro,* B-CLL cells were cultured with and without IDEC- 131 in the presence of CD40L *in vitro.* Peripheral blood mononuclear cells (PBMC) were isolated from a CLL patient's blood using a Ficoll-Hypaque gradient centrifugation. Viability was determined by Trypan blue dye exclusion and was >98%. Flow cytometric analysis revealed that 70% of the lymphocytes were CD 19+/CD20+. CLL cells (PBMC) were cultured in CLL growth medium (e.g., RPMI-1640 medium supplemented with 5% FCS or 2% of autologous donor plasma, supplemented with 2 mM L-Glutamine and 100 U/ml Penicillin-Streptomycin). In addition, for some experiments, CD19+ B-cells were purified using CD19+ Dynabeads™ as per manufacture's instructions (Dynal, Cat. #111.03/111.04) and cultured as above. CLL or purified B-CLL cells cultured in growth medium mostly under went spontaneous apoptotic cell death. However, culturing these cells in the presence of sCD40L extended their viability in cultures. **Table II** indicates the cell viability of CD 19+ B-CLL cells grown in the presence or absence of sCD40L (5 μg/ml) at different time points and indicates the longer survival of CLL cells. B-CLL cells from Patient #1 cultured with sCD40L had 60% viability for greater than 2 weeks, whereas cells grown in the absence of sCD40L had less than 10% viability.

**Table II:**

| Survival of B-CLL cells in the presence of sCD40L | | | |
|---|---|---|---|
| **B-CLL Sample** | **Time (Hours)** | **% Viability[a]** | |
| | | **(-) CD40L** | **(+)CD40L** |
| Patient #1 | 0 | ≥90 | ≥90 |
| | 48 | 88 | 90 |
| | 96 | 46 | 77 |
| | 144 | 30 | 72 |
| Patient #2 | 0 | ≥90 | ≥90 |
| | 72 | 40 | 72 |

(continued)

| Survival of B-CLL cells in the presence of sCD40L | | | |
|---|---|---|---|
| B-CLL Sample | Time (Hours) | % Viability[(a)] | |
| | | (-) CD40L | (+)CD40L |
| | 96 | 31 | 65 |
| | 144 | 17 | 51 |
| [(a)] equals the percent viability determined by Trypan blue dye exclusion. | | | |

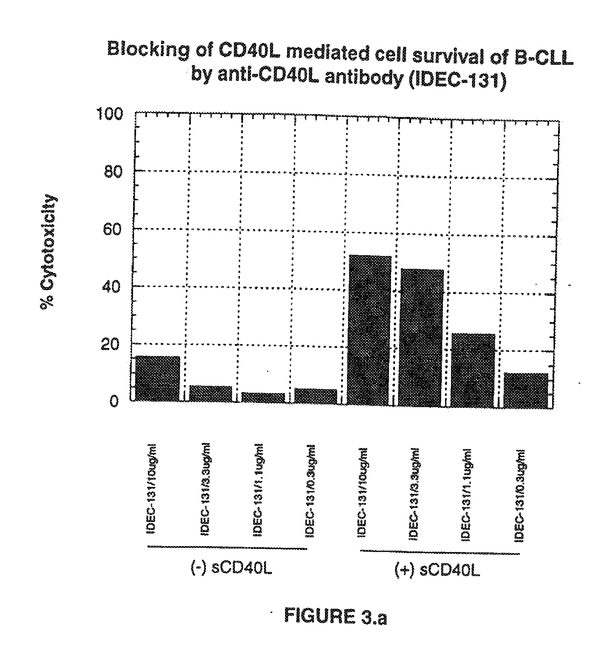

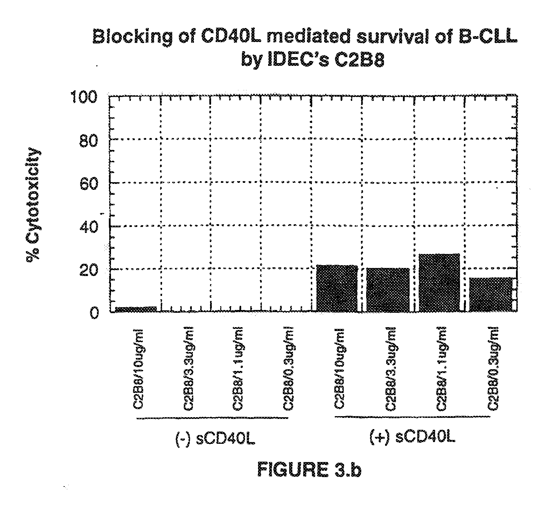

**Fig. 3A** shows the effect of IDEC- 131 on the growth and survival of B-CLL cells after 7 days in culture. Purified B-CLL cells from a CLL patient ($2 \times 10^6$ cells/ml) were divided into two culture tubes. Cells in one tube were mixed with sCD40L (5 $\mu$g/ml) in equal volume of growth medium, whereas the other tube was incubated with equal volume of growth medium as control. After 1 hour of incubation at 37° C., cells were gently mixed and 100 $\mu$l of cell suspension media added to each well of a 96-well flat bottom plate in duplicate with and without varying concentrations of IDEC- 131 ($10\mu$ g/ml to 0.3 $\mu$g/ml). Seven days later, cell survival/death in culture was determined by Alamar Blue assay, as described above. Data showed cell survival in cultures with sCD40L. The addition of IDEC-131 into culture resulted in increased cell death, which indicated a reversal of cell survival or a sensitization to cell death. Additionally, RITUXAN® administered at the same concentration as the IDEC-131 produced less of lower effect than IDEC-131 on cell death (Fig. 3B).

*Example 5*

*CD40L-CD40 mediated up-regulation of HLA-DR molecules in B-CLL*

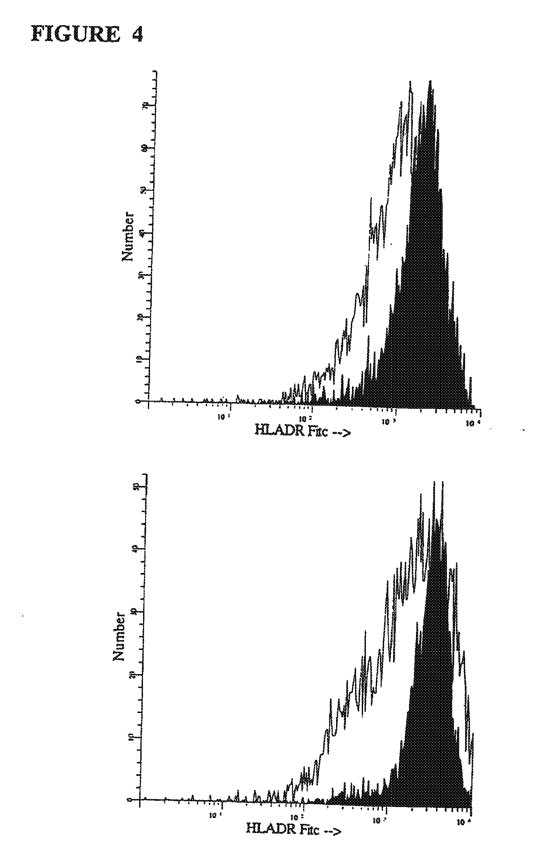

To determine whether the CD40L-CD40 signal transduction pathway is intact, CLL cells from CLL patients were cultured ($5 \times 10^5$ cells/ml) with and without 5 $\mu$g/ml of CD40L at 37 ° C. At 48 hours and 144 hours, the class II molecule, HLA-DR expression, was determined on CD 19$^+$ cells by flow cytometry using standard procedures. Briefly, cultured lymphocytes were harvested at different time points and analyzed for surface expression of molecules using antibodies coupled to either fluorescein (FITC) or phycoerythrin (PE) for single or double staining using a FACScan (Becton-Dickinson) flow cytometer. To stain for flow cytometry, $1 \times 10^6$ cells in culture tubes were incubated with appropriate antibodies as follows: anti-CD45-FITC to gate lymphocyte population on a scatter plot; anti-CD19-PE (Pharmingen, Cat. # 30655) or anti-CD20-FITC (Pharmingen; Cat. #33264) antibodies to determine the CD19$^+$ and/or CD20$^+$ B-cells; anti-CD3-FITC antibodies (Pharmingen; Cat. #30104) to gate-off the T cells; anti-CD 19-RPE and anti-HLA-DR-FITC antibodies (Pharmingen; Cat. #32384) to determine the Pclass II expression on CD 19$^+$ cells. Cells were washed once by centrifugation (at 200 x g, for 6 min.) with 2 ml cold PBS and incubated with antibody for 30 min. on ice, after which the cells washed once, fixed in 0.5% paraformaldehyde and stored at 4°C. until analyzed. Flow cytometry data was acquired on FACsan and analyzed using WinList software (Verity Software House). The machine was set to autogating to allow examination of quadrants containing cells that were single stained with either RPE or FITC, unstained or doubly stained. Fig. 4 shows the comparison of HLA-DR expression in CD 19$^+$ CLL cells cultured with sCD40L and those cells not cultured with sCD40L. A higher level of HLA-DR expression was detected on B-CLL cells cultured in the presence of sCD40L (Table III).

**Table III:**

| CD40L-CD40 mediated up-regulation ofHLA-DR molecule in B-CLL | | | |
|---|---|---|---|
| Sample | Time | HLA-DR$^{+(a)}$ | |
| | | %Positive | MFI |
| Control | 48 hrs | 81 | 92 |
| | 144 hrs | 88 | 1655 |
| Cells + sCD40L | 48 hrs | 88 | 101 |
| | 144 hrs | 95 | 2943 |
| (a) CD 19$^+$ B-cells that are positive for HLA-DR molecules and its mean fluorescent intensity (MIF). | | | |

*Example 6*

*Preparation of IDEC-131 and RITUXAN®*

For treatment of a CD40⁺ malignancy, IDEC-131 at about 10 to about 50 mg/ml in a formulation buffer 10 mM Na-citrate, 150 mM NaCl, 0.02% Polysorbate 80 at pH 6.5 is infused intravenously (iv) to a subject. IDEC-131 is administered before, after or in conjunction with RITUXAN®. The RITUXAN® dosage infused ranges from about 3 to about 10 mg/kg of subject weight.

*Example 7*

*Preparation of IDEC-131 and CHOP*

For treatment of CD40⁺ malignancies responsive to CHOP (e.g., Hodgkin's Disease, Non-Hodgkin's lymphoma and chronic lymphocytic leukemia, as well as salvage therapy for malignancies wherein cells are CD40⁺), IDEC- 131 is infused at a dosage ranging from about 3 to about 10 mg per kg of patient weight immediately prior to the initiation of the CHOP cycle. IDEC-131 administration will be repeated prior to each CHOP cycle for a total of 4 to 8 cycles.

*Example 8*

*Administration of anti- CD40L or anti-B7 in combination with RITUXAN® to treat B-cell lymphoma in a subject*

Combination therapies are particularly useful as salvage therapies or for treating relapsed or aggressive forms of CD40⁺ malignancies (e.g., Hodgkin's Disease, Non-Hodgkin's lymphoma and CLL). When IDEC-131 is to be administered in combination with CHOP and RITUXAN®, IDEC-131 is administered as discussed above in Example 6, followed by the schedule specified for CHOP-IDEC-131 administration in Example 7. Alternatively, the same regimen is effected wherein IDEC-131 (anti-CD40L) is substantially within an anti-B7 antibody.

*Examples 9*

*In vitro studies of Anti-CD80 and Anti-CD20 using Lymphoma Cell Lines*

In order to reinforce the scientific basis for employing anti-CD80 and anti-CD20 as a combination therapeutic regimen for treating lymphomas, the following in *vitro* experiments using lymphoma cell lines were conducted.

**Cell Lines Used:** Cell lines were obtained and maintained as follows. CD20-and B7-expressing B-lymphoma cell lines (SKW, SB, and Daudi cells) were cultured in complete medium. Complete medium is RPMI 1640 medium (Irvine Scientific, Santa Ana, CA) supplemented with 10% heat inactivated FBS (Hyclone), 2 mM 1-glutamine, 100 units/ml of penicillin, and 100 ug/ml of streptomycin. The SKW cell line is Epstein-Barr virus (EBV) positive and can be induced to secrete IgM (SKW 6.4, ADCC). The SB cell line originated from a patient with acute lymphoblastic leukemia and is positive for EBV (CCL-120, ATCC). The Daudi cell line was isolated from a patient with Burkitt's lymphoma (CCL-213, ATCC). Neomycin resistant CD80-expressing Chinese hamster ovary cells (CHO) were generated using IDEC Pharmaceuticals proprietary vector system.

**Antibodies Used:** The specific antibodies used in these studies are as follows. IDEC-114 is a PRIMATIZED® anti-human CD80 mAb that contains human gamma 1 heavy chain (Lot 114S004F, code 3002G710; Lot ZPPB-01) and rituximab is an anti-human CD20 specific mouse-human gamma 1 chimeric antibody (Lot E9107A1; Lot D9097A1). Other antibodies used include the murine anti-human CD80 mAb L307.4 (BD Pharmingen, San Diego, CA), the primatized anti-human CD4 mAb CE9.1, with human gamma 1 chain (Lot M2CD4156), and the murine isotype-matched (IgG1) control antibody 3C9 developed at IDEC Pharmaceuticals.

**Expression of CD80 and CD20 on certain lymphoma cell lines.**

In order to assay the cell surface expression of CD80 and CD20 on certain lymphoma cell lines, IDEC- 114 and Rituxan binding on those cell lines was determined by fluorescence-activated cell sorting (FACS) as follows. Varying concentrations of test or control antibodies diluted to a final volume of 200 $\mu$l in cold FACTS binding buffer were incubated in a cell-culture tube with $1 \times 10^6$ cells. IDEC- 114 and rituximab were used as test antibodies and CE9.1 was used as the isotype-matched negative control. The cells were incubated for 60 minutes on ice and washed once in FACS wash buffer following incubation. Cells were resuspended in 200 $\mu$l of FACTS binding buffer, and 2 $\mu$l of FITC-conjugated goat F(ab')$_2$ anti-human Ig gamma chain specific antibodies (Southern Biotechnology, Birmingham, AL) per $10^6$ cells

was added. Following further incubation of 30 minutes on ice, cells were washed once and resuspended in 200 $\mu$l cold HBSS, and fixed with 200 $\mu$l of 1% formaldehyde.

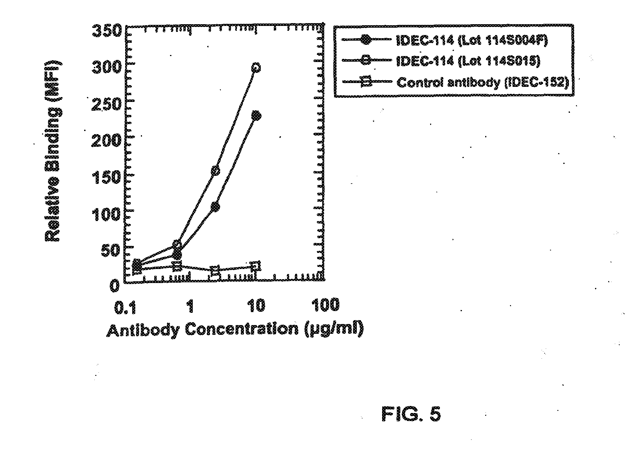

Fig. 5 shows the specific binding of IDEC-114 from two different lots (Lot 114S004F and Lot 114S015) to CD80-CHO cells in a concentration dependent fashion. As expected, isotype-matched control antibody of irrelevant specificity (IDEC-152) did not bind to CD80-CHO cells. Testing of IDEC-114 for binding to CD80 on SKW and SB lymphoma cell lines showed a lower binding than that of rituximab as demonstrated by a lower percentage of positive cells (Table IV) and lower mean fluorescence intensity (Table V).

**Table IV:**

| Binding of Antibodies to B-Lymphoma Cell Lines | | | |
|---|---|---|---|
| Antibody (10 $\mu$g/ml) | Intensity of Binding Activity* | | |
| | SKW | SB | Daudi |
| IDEC- 114 | 1.8 | 2 | 3 |
| Rituximab | 20 | 52 | 60 |
| CE9.1 (control mAb) | 1 | 1 | 1 |
| *Binding to cells was determined by flow cyometry at saturating concentrations of antibody. Intensity of Binding Activity = MFI of test antibody - MFI of control antibody. | | | |

**Table V:**

| Relative CD80 Antigen Density on CD80-CHO and SB Cells | |
|---|---|
| Cell | MFI* |
| CD80-CHO | 676 |
| SB (B-lymphoma line) | 189 |
| PBMC Control | 51 |
| PBMC Activated | 44 |
| *The relative CD80 antigen was measured by mean fluorescence intensity (MFI). Values are expressed in units after subtraction of background intrinsic fluorescence. | |

These results reinforce the suitability of anti-CD80 and anti-CD20 as therapeutic agents for lymphomas.

**Antibody-Dependent Cellular Cytotoxicity (ADCC)**

To further demonstrate the suitability of anti-CD80 and anti-CD20 as therapeutic agents for lymphomas, examples of each antibody were assayed for their ability to mediate ADCC. In the ADCC assay, SKW or SB cells and activated human peripheral monocytes (PBMC) were used as targets and effector cells, respectively. PBMC were isolated from whole blood of healthy donors using Histopaque (Sigma-Aldrich Corp., St. Louis, MO). The PBMC were cultured at a concentration of 5 x 10$^6$ cells/ml in complete medium with 20 U/ml recombinant human IL-2 (Invitrogen, Carlsbad, CA) in 75 cm$^2$ tissue culture flasks at 37°C and 5% $CO_2$. After overnight culture, 1 x 10$^6$ SKW or SB target cells were labeled with 150 $\mu$Ci of $^{51}$Cr (Amersham Pharmacia Biotech, Piscataway, NJ) for 1 hour at 37°C and 5% $CO_2$. The cells were washed four times and resuspended in 5 ml of complete medium; 50 $\mu$l of cell suspension was dispensed into each well containing equal volume of test or control antibodies.

Rituximab (Lot E9107A1) or IDEC- 114 (Lot 114S004F, code 3002G710) were used as test antibodies. Isotype matched CE9.1 (Lot NUCD4156) or L307.4 (BD Pharmingen), or a murine isotype-matched (IgG$_1$) antibody of irrelevant specificity, 3C9, were used. All wells were plated in triplicate into a 96 well, round bottom tissue culture plate. The effector cells were harvested, washed once with complete medium, and added at 1 x10$^6$ cells in 100 $\mu$l volume per well to obtain a 50:1 effector to target ratio. The following control wells were also included in triplicate: target cell incubated with 100 $\mu$l complete medium to determine spontaneous release and target cell incubated with 100 $\mu$l 0.5% Triton X-100 (Sigma-Aldrich Corp.) to determine maximum release. The culture was incubated for 4 hours at 37°C and 5% $CO_2$ and the $^{51}$Cr

released in the culture supernatant due to cell lysis was determined by a gamma counter (ISODATA). The cytotoxicity was expressed as the percentage of specific lysis and calculated as follows:

$$\frac{^{51}\text{Cr release of test samples - spontaneous } ^{51}\text{Cr release}}{\text{Maximum } ^{51}\text{Cr release - spontaneous } ^{51}\text{Cr release}} \times 100$$

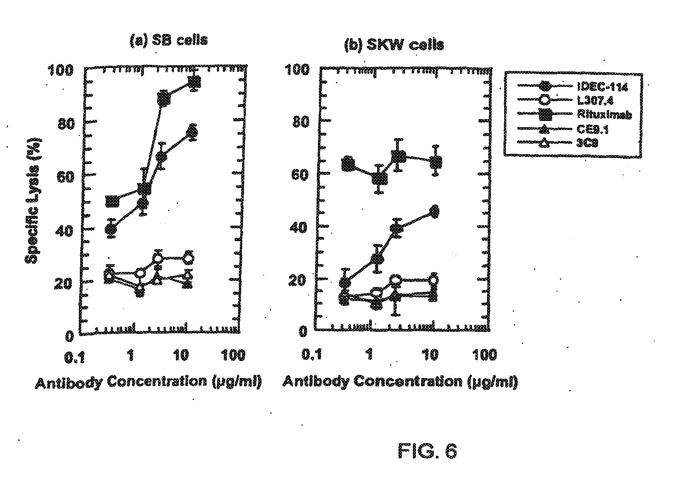

Fig. 6 shows the ADCC activity of IDEC-114 and rituximab on CD20+/CD80+ SB and SKW cells. Overall, higher levels of ADCC activity were observed with SB cells than with SKW cells. IDEC-1 14 showed a dose-dependent killing of SB and SKW cells with a maximum killing of 75% and 46%, respectively, at 10 $\mu$g/ml. Rituximab at comparable antibody concentrations showed higher ADCC activity (97% on SB cells and 65% on SKW cells) than IDEC-114, which correlated with higher cell-binding activity ofrituximab compared with IDEC-114. As expected, murine L307.4, which does not bind to the human Fc receptor, showed weak ADCC activity. Only background levels of ADCC were observed with isotype human and murine controls (CE9.1 and 3C9, respectively).

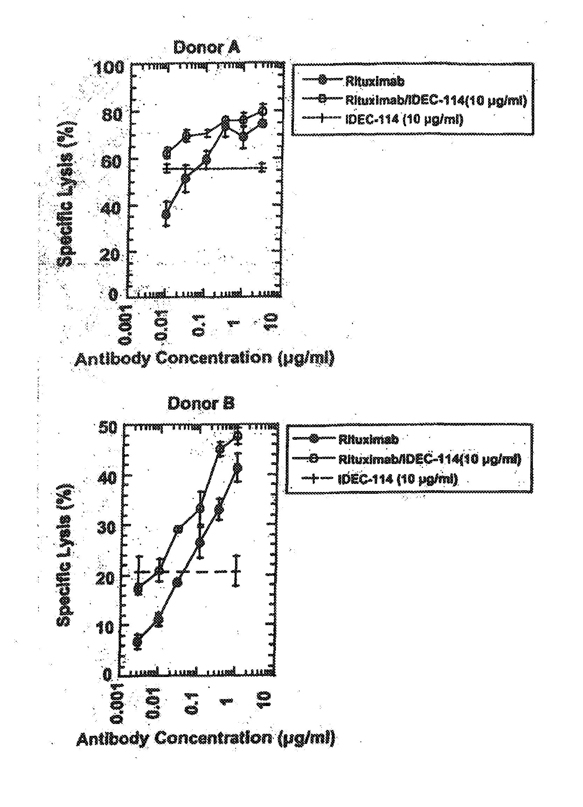

Experiments were performed to determine the effect of combining IDEC-114 with rituximab to increase host effector mediated killing of tumor cells. In these experiments, a fixed concentration of IDEC-114 was combined with varying concentrations of rituximab to reflect a scenario where low CD20 density with normal B7 expression on B-lymphoma cells could lead to elective tumor killing. Fig. 7 shows that the combination of IDEC-114 with rituximab leads to enhanced ADCC activity on SKW lymphoma cells. IDEC-114 at a fixed concentration of 10 $\mu$g/ml in combination with rituximab concentrations of 0.1 to 0.01 $\mu$g/ml mediated an enhanced killing of SKW cells. The results obtained using host effector cells from two donors showed the same trend in ADCC activity.

**Complement-Dependent Cytotoxicity (CDC)**

The CDC activity of IDEC-114 and rituximab was determined using B-cell lines and human complement (C). Dilutions of antibodies were made at 4x concentration and 50 $\mu$l was dispensed into each 96 well in triplicates. The SKW or Daudi cells were labeled with $^{51}$Cr (150 $\mu$Ci/10$^6$ cells) for 1 hour at 37°C and 5% $CO_2$. The cells were washed four times and resuspended in complete medium, and 1 x 10$^4$ cells in 50 $\mu$l were dispensed into each well. One hundred $\mu$l of normal human serum complement (Quidel, San Diego, CA) diluted 1:4 or 1:8 in complete medium was added. Methods for the spontaneous and maximum release control and set up are the same as described above for the ADCC experiments. The cultures were incubated 4 hours at 37°C and 5% $CO_2$. The radioactivity released into the culture supernatant was determined by a gamma counter. The formula for calculating the percentage of specific cell lysis is also as described above for the ADCC experiments.

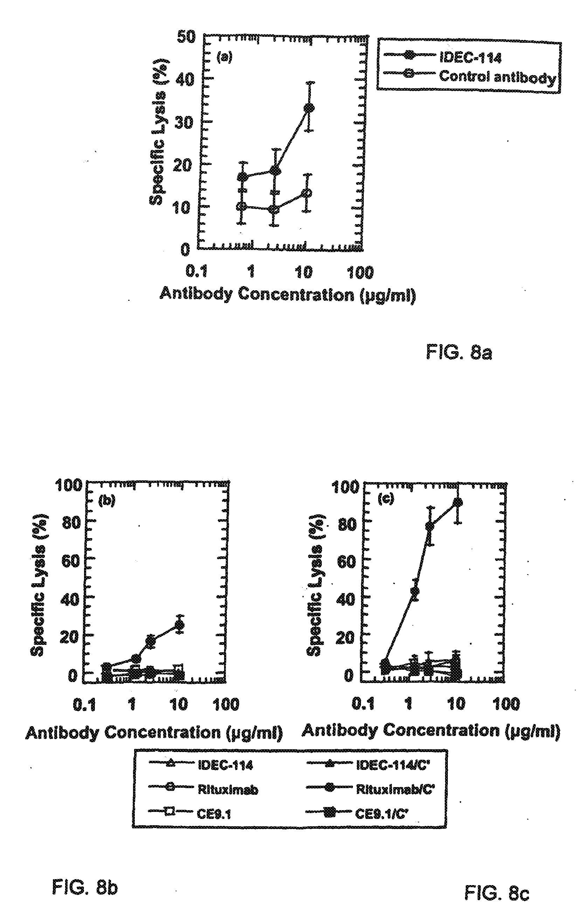

Activation of the complement cascade following binding of rituximab to the CD20 antigen results in efficient killing of B-lymphoma cells in vitro. Reft ME, Camer K, Chambers KS, Chinn PC, Leonard JE, Raab R, et al. Depletion of B cells in vivo by a chimeric mouse human monoclonal antibody to CD20. Blood 1994;83(2):435-45. Therefore, we evaluated the capacity of IDEC-114 to mediate complement-dependent killing of CD80+ target cells. Results showed that IDEC-114 mediates CDC of CD80-expressing CHO cells (Fig. 8a). However, binding of IDEC- 114 to CD80+ Daudi and SKW lymphoma cell lines showed no evidence of CDC (Fig. 8b and Fig. 8c, respectively). In contrast, rituximab showed CDC activity on both cell lines, although the Daudi cell line was more sensitive to CDC than the SKW cell line (Figs. 8b and Fig. 8c, respectively).

*Example 10*

*In vitro studies of Anti-CD80 and Anti-CD20 using Cells Isolated from Tumor Samples*

To further assess the scientific basis for employing anti-CD80 and anti-CD20 as a combination therapeutic regimen for treating lymphomas, the following in vitro experiments using cells isolated from tumor samples were conducted.

CD80 is transiently expressed on the surface of activated B cells and activated APCs, but is weakly expressed or not expressed on resting B-cells and resting APCs. Since CD80 is a B-cell activation marker, it is expressed primarily on the dividing and/or activated lymphoma cells. Reports suggest that CD80 is constitutively expressed on malignant B cells. To confirm these reports, the expression of CD80 was tested by flow cytometry in a panel of lymphoma and leukemia specimens obtained from 20 patients. Results indicate that CD80 is expressed in lymphomas and leukemias at different densities (Table VI).

**Table VI:**

| Expression of CD80 on Lymphoma/Leukemia Specimens | | |
|---|---|---|
| **Lymphoma** | **Positive Specimens*** | **Expression Level†** |
| Follicular, small-cleaved, low-grade lymphoma | 12/12 | Medium to High |
| Follicular, large-cell, intermediate-grade lymphoma | 1/1 | Low |
| Small, non-cleaved Burkitt's lymphoma | 2/2 | High |
| Small, non-cleaved, high-grade lymphoma | 1/1 | Weak |
| Chronic lymphocytic leukemia (CLL)/small lymphocytic lymphoma (SLL) | 2/2 | Weak to Medium |
| Mantle cell lymphoma (CLL variant) | 2/2 | Medium |
| * Positive samples/samples tested <br> †expression level is a subjective value estimated by analysis of flow cytometry data, and is the percentage of positive gated cells over the control antibody; <br> weak = < 10%, low = 10% to 25%, medium = 25% to 50%, and high = < 50% | | |

The highest CD80 expression was observed in follicular, small-cleaved, low-grade lymphoma and in small, non-cleaved Burkitt's lymphoma. The lowest expression was seen in one chronic lymphocytic lymphomas (CLL) sample and in one small, non-cleaved, high-grade lymphoma. CD80 expression on follicular, small-cleaved, low-grade lymphoma samples is presented in Table VII.

**Table VII:**

| Expression of CD80 on Follicular, Small-Cleaved, Low-Grade Lymphoma Specimens | |
|---|---|
| **Sample** | **Percentage of CD80 Expression** |
| 1 | 79% |
| 2 | 25% |
| 3 | 26 % (Small) |
| | 89% (Large) |
| 4 | 99% |
| 5 | 100% (Small) |
| | 99% (Large) |
| 6 | 67% (Small) |
| | 95% (Large) |
| 7 | 67% (Small) |
| | 89% (Large) |
| 8 | 64% (Small) |
| 9 | 100% |
| 10 | 70% |
| 11 | 88% |
| 12 | 84% |

The CD80 expression on these lymphoma cells ranged from 25% to 90% of the tumor cells in the samples. It is interesting, however, that within the same lymphoma the "large" cells were 90% to 100% positive, while the "small" cells were 25% to 100% positive. It is possible that CD80 is expressed on proliferating or activated malignant B cells, which may account for the variability of expression within lymphoma samples tested.

*Examples 11*

*In vivo studies of Anti-CD80 and Anti-CD20 using SCID Mouse Model*

In order to test the effectiveness of combination therapies, the following in vivo experiments were conducted using anti-CD80 (IDEC-114) and anti-CD20 (RITUXAN (Rituximab)).

**In Vivo Therapeutic Effect of IDEC-114 and Rituximab Single-Agent Therapies in Lymphoma**

A human lymphoma tumor model in severe immunodeficiency (SCID) mice was developed. Briefly, $3 \times 10^6$ to $4 \times 10^6$ human SKW lymphoma cells were inoculated intravenously (IV) into 6- to 8-week old female BALB/c SCID mice and their survival was monitored for 45 to 60 days. After inoculation, SKW cells disseminate throughout the mouse and grow primarily in the lungs and liver. Mice in the treatment groups (N = 8) were injected intraperitonealy with IDEC-114 (a) or rituximab (b) at 100 $\mu$g, 200 $\mu$g, or 400 $\mu$g on days 1,3, 5, 7, 9, and 11. All mice developed a paralytic form of the disease before circumventing to death. Mice that developed severe paralysis were sacrificed and scored as dead. Kaplan-Meier analysis was performed using the Statistical Analysis System (SAS) and *p*-values were generated by the Log-rank test.

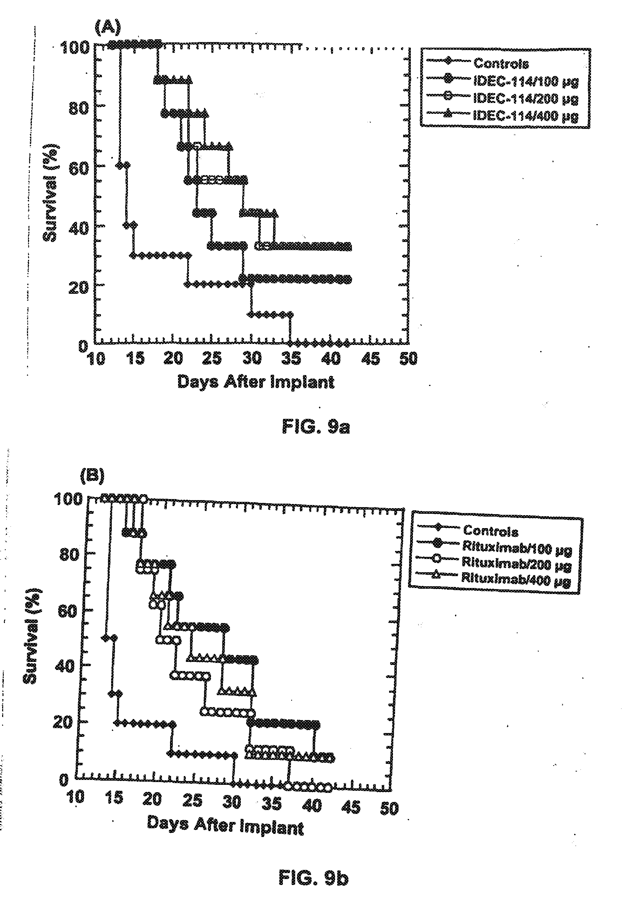

Figs 9A and 9B show the antitumor response of single-agent IDEC-114 (Fig. 9A) and single-agent rituximab (Fig. 9B) therapy using three doses (100, 200, and 400 $\mu$g) of the antibody. IDEC-114 and rituximab single-agent therapy showed inhibition of disease progression at all doses. The antitumor response observed with IDEC-114 was comparable to the antitumor response of rituximab at the same dose and treatment schedule.

*Example 12*

*In Viva Therapeutic Effect of IDEC-114/Rituximab Combination Therapy in Lymphoma*

Based on the antitumor activity of IDEC-114 as a single agent, a combination of IDEC-114 and rituximab was evaluated in the same tumor model at the same dosing schedule described above for the single agent studies.

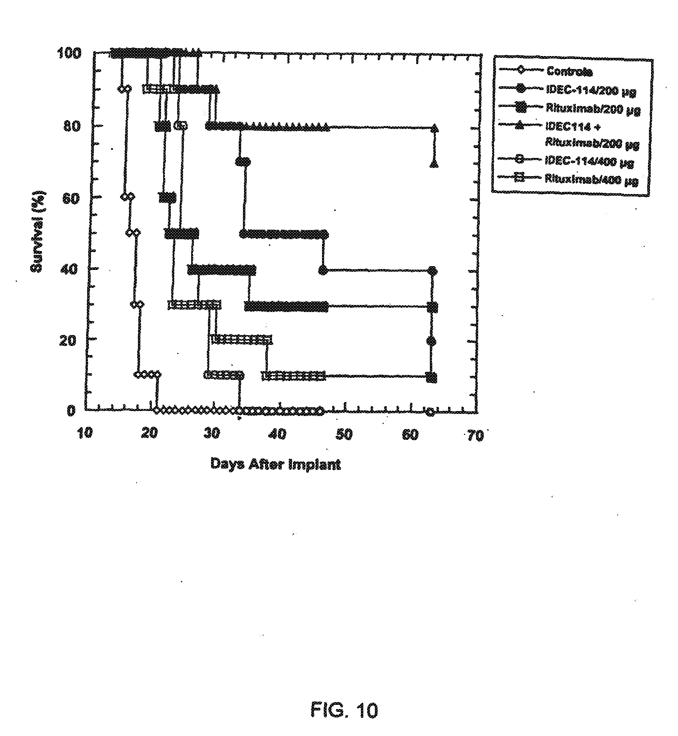

SKW/SCID mice were injected with 200 $\mu$g of IDEC-114 and 200 $\mu$g of rituximab, and compared with the mice injected with either 200 $\mu$g or 400 $\mu$g of IDEC-114 or 200 $\mu$g or 400 $\mu$g of rituximab. Fig. 10 shows the survival advantage of mice treated with a IDEC-114/rituximab combination therapy compared with mice treated with either IDEC-114 or rituximab as a single-agent therapy. Results show that the combination of IDEC-114 and rituximab leads to increased disease-free survival compared with either antibody alone. In the combination therapy group, 70% (7/10) of the mice survived for more than 50 days after the last antibody injection. In contrast, less than 10% of mice treated with IDEC-114 or rituximab alone were alive at the end of the study. Survival data were analyzed by Kaplan-Meier and Log-rank tests (Table VIII).

**Table VIII:**

| Comparison of IDEC-114/Rituximab Combination Therapy with IDEC-114 or Rituximab Single-Agent Therapy | |
|---|---|
| **Comparison** | **p-value*** |
| IDEC-114/tituximab combination vs. saline control group | 0.0001 |
| IDEC-114/rituximab combination vs. rituximab (200 $\mu$g and 400 $\mu$g) | 0.0008 |
| IDEC-114/rituximab combination vs. IDEC-114 (200 $\mu$g and 400 $\mu$g) | 0.0017 |
| IDEC-114/rituximab combination vs. IDEC-114 (200 $\mu$g) | 0.0403 |
| IDEC-114/rituximab combination vs. IDEC-114 (400 $\mu$g) | 0.001 |
| *p-value generated by Log-rank test | |

The IDEC-114/rituximab combination therapy produced a statistically greater response than 200 $\mu$g or 400 $\mu$g of IDEC-114 or rituximab single-agent therapy.

Those skilled in the art will further appreciate that the present invention may be embodied in other specific forms without departing from the spirit or central attributes thereof. In that the foregoing description of the present invention discloses only exemplary embodiments thereof, it is to be understood that other variations are contemplated as being within the scope of the present invention. Accordingly, the present invention is not limited to the particular embodiments that have been described in detail herein. Rather, reference should be made to the appended claims as indicative of the scope and content of the invention.

Preferred embodiments of the present invention are described and are reffered to as embodiments E1 to E50 .

E 1. A method of treating a mammal suffering from or predisposed to a neoplastic disorder comprising the steps of:

administering a therapeutically effective amount of a first immunoregulatory antibody to said mamma!; and administering a therapeutically effective amount of a second immunoregulatory antibody or a B cell depleting antibody to said mammal wherein said first and second immunoregulatory antibodies bind to different antigens and the first immunoreglatory antibody and the second immunoregulatory antibody or B cell depleting antibody may be administered in any order or concurrently.

E 2. The method of E 1 wherein said first immunoregulatory antibody reacts with or binds to B7 antigen.

E 3. The method of E 2 comprising the administration of a second immunoregulatory antibody wherein said second immunoregulatory antibody reacts with or binds to an antigen selected from the group consisting of CD40L, CD40, and CD23.

E 4. The method of E 3 wherein said second immunoregulatory antibody binds to CD40L antigen.

E 5. The method of E 1 wherein said first immunoregulatory antibody, said second immunoregulatory antibody and said B cell depleting antibody are monoclonal antibodies.

E 6. The method of E 5 wherein said monoclonal antibodies are selected from the group consisting of chimeric antibodies and humanized antibodies.

E 7. The method of E 6 wherein at least one of said monoclonal antibodies is a chimeric antibody and said chimeric antibody is primatized.

E 8 . The method of E 7 wherein said primatized antibody is IDEC-114.

E 9. The method of E 1 wherein said neoplastic disorder is selected from the group consisting of relapsed Hodgkin's disease, resistant Hodgkin's disease high grade, low grade and intermediate grade non-Hodgkin's lymphomas, B cell chronic lymphocytic leukemia (B-CLL), lymhoplasmacytoid lymphoma (LPL), mantle cell lymphoma (MCL), follicular lymphoma (FL), diffuse large cell lymphoma (DLCL), Burkitt's lymphoma (BL), AIDS- related lymphomas, monocytic B cell lymphoma, angioimmunoblastic lymphoadenopathy, small lymphocytic; follicular, diffuse large cell; diffuse small cleaved cell; large cell immunobiastic lymphoblastoma; small, non-cleaved; Burkitt's and non-Burkitt's; follicular, predominantly large cell; follicular, predominantly small cleaved cell; and follicular, mixed small cleaved and large cell lymphomas.

E 10. The method of E 1 comprising the administration of a first immunoregulatory antibody and a B cell depleting antibody.

E 11. The method of E 10 wherein said B cell depleting antibody reacts with or binds to an antigen selected from the group consisting of CD20, CD19, CD22 and CD37 antigens.

E 12. The method of E 11 wherein said B cell depleting antibody reacts with or binds to CD20.

E 13 . The method of E 12 wherein said B cell depleting antibody is associated with a radioisotope.

E 14. The method of E 12 wherein said B cell depleting antibody in RITUXAN®.

E 15. The method of E 14 wherein said first immunoregulatory antibody is IDEC-114.

E 16. The method of E 14 wherein said first immunoregulatory antibody is IDEC-131.

E 17. The method of E 1 further comprising the step of administering a chemotherapeutic agent.

E 18. A method of treating a mammal suffering from or predisposed to a neoplastic disorder comprising the steps of:

administering a therapeutically elective amount of at least one chemotherapeutic agent to said mammal; and administering a therapeutically effective amount of at least one immunoregulatory antibody to said mammal wherein said chemotherapeutic agent and
said immunoregulatory antibody may be administered in any order or concurrently.

E 19. The method of E 18 wherein said immunoregulatory antibody binds to an antigen selected from the group consisting of B7, CD40, CD40L and CD23 antigens.

E 20. The method of E. 19 wherein said immunoregulatory antibody comprises a monoclonal antibody.

E 21. The method of E 20 wherein said monoclonal antibody is selected from the group consisting of chimeric antibodies and humanized antibodies.

E 22. The method of E 21 wherein said immunoregulatory antibody is a chimeric antibody and said chimeric antibody is primatized.

E 23. The method of E 22 wherein said primatized antibody is IDEC-114.

E 24. The method of E 12 wherein said monoclonal antibody is humanized.

E 25. The method of E 24 wherein said humanized antibody comprises IDEC-131.

E 26. The method of E 18 wherein said neoplastic disorder is selected from the group consisting of relapsed Hodgkin's disease, resistant Hodgkin's disease high grade, low grade and intermediate grade non-Hodgkin's lymphomas, B cell chronic lymphocytic leukemia (B-CLL), lymhoplasmacytoid lymphoma (LPL), mantle cell lymphoma (MCL), follicular lymphoma (FL), diffuse large cell lymphoma (DLCL), Burkitt's lymphoma (BL), AIDS- related lymphomas, monocytic B cell lymphoma, angioimmunoblastic lymphoadenopathy, small lymphocytic; follicular, diffuse large cell; diffuse small cleaved cell; large cell immunoblastic lymphoblastoma; small, non-cleaved; Burkitt's and non-Burkitt's; follicular, predominantly large cell; follicular, predominantly small cleaved cell; and follicular, mixed small cleaved and large cell lymphomas.

E 27. The method of E 18 further comprising the step of administering a B cell depleting antibody.

E 28. The method of E 27 wherein said B cell depleting antibody reacts with or binds to CD20 antigen.

E 29. A method of treating a mammal suffering from or predisposed to a neoplastic disorder comprising the steps of:

administering a therapeutically effective amount of a first immunoregulatory antibody to said mammal; and administering a therapeutically effective amount of a second immunoregulatory antibody to said mammal wherein said first and second immunoregulatory antibodies bind to different antigens and the first immunoreglatory antibody and the second immunoregulatory antibody may be administered in any order or concurrently.

E 30. The method of E 29 wherein said first and second immunoregulatory antibodies bind to an antigen selected from the group consisting of B7, CD40, CD40L and CD23 antigens.

E 31. The method of E 30 wherein said first immunoregulatory antibody reacts with or binds to B7 antigen and said second immunoregulatory antibody reacts with or binds to CD40L antigen.

E 32. The method of E 31 wherein said first immunoregulatory antibody comprises IDEC- 114 and said second immunoregulatory antibody comprises IDEC-131.

E 33. The method of E 32 wherein said neoplastic disorder is selected from the group consisting of relapsed Hodgkin's disease, resistant Hodgkin's disease high grade, low grade and intermediate grade non-Hodgkin's lymphomas, B cell chronic lymphocytic leukemia (B-CLL), lymhoplasmacytoid lymphoma (LPL), mantle cell lymphoma (MCL), follicular lymphoma (FL), diffuse large cell lymphoma (DLCL), Burkitt's lymphoma (BL), AIDS- related lymphomas, monocytic B cell lymphoma, angioimmunoblastic lymphoadenopathy, small lymphocytic; follicular, diffuse large cell; diffuse small cleaved cell; large cell immunoblastic lymphoblastoma; small, non-cleaved; Burkitt's and non-Burkitt's; follicular, predominantly large cell; follicular, predominantly small cleaved cell; and follicular, mixed small cleaved and large cell lymphomas.

E 34. The method of E 32 further comprising the step of administering a therapeutically effective amount of at least one chemotherapeutic agent.

E 35. The method of E 32 further comprising the step of administering at least one B cell depleting antibody.

E 36. The method of E 35 wherein said B cell depleting antibody reacts with or binds to an antigen selected from the group consisting of CD20, CD19, CD22 and CD37 antigens.

E 37. The method of E 36 wherein said B cell depleting antibody reacts with or binds to CD20.

E 38. The method of E 37 wherein said B cell depleting antibody in RITUXAN®.

E 39. A kit useful tor the treatment of a mammal suffering from or predisposed to a neoplastic disorder comprising at least one container having an immunoregulatory antibody deposited therein and a label, instructions or an insert indicating that said immunoregulatory antibody may be used to treat said neoplastic disorder.

E 40. The kit of E 39 wherein said immunoregulatory antibody binds to an antigen selected from the group consisting of B7, CD40, CD40L and CD23 antigens.

41. The kit of E 40 wherein said immunoregulatory antibody comprises a monoclonal antibody.

42. The kit of E 41 wherein said monoclonal antibody is selected from the group consisting of chimeric antibodies and humanized antibodies.

E 43. The kit of E 42 wherein said immunoregulatory antibody is a chimeric antibody and said chimeric antibody is primatized.

E 44. The kit of E 43 wherein said primatized antibody is IDEC-114.

E 45. The kit of E 42 wherein said monoclonal antibody is humanized.

46. The kit of E 45 wherein said humanized antibody comprises IDEC-131.

E 47. The kit of E 39 further comprising a container comprising a second immunoregulatory antibody or a B cell depleting antibody wherein said second immunoregulatory antibody binds to a different antigen than said immunoregulatory antibody.

E 48. The kit of E 47 comprising an immunoregulatory antibody and a second immunoregulatory antibody wherein said immunoregulatory antibody binds to B7 antigen and said second immunoregulatory antibody binds to CD40L antigen.

E 49. The kit of E 48 wherein said immunoregulatory antibody comprises IDEC-114 and said second immunoregulatory antibody comprises IDEC-131.

50. The kit of E 47 comprising an immunoregulatory antibody and a B cell depleting antibody wherein said immunoregulatory antibody reacts with or binds to an antigen selected from the group consisting of CD40L and B7 antigens and said B cell depleting antigen reacts with or binds to CD20 antigen.

**Claims**

1. Use of an anti-CD80 antibody for the manufacture of a medicament for simultaneous or sequential use in combination with one or more chemotherapeutic agents for treating a B cell malignancy in a human subject.

2. The use according to claim 1, wherein the B cell malignancy is a B cell lymphoma, and is preferably Hodgkin's lymphoma, non-Hodgkin's lymphoma (NHL), low grade/follicular NHL, follicular center cell (FCC) lymphoma, mantle cell lymphoma (MCL), diffuse large cell lymphoma (DLCL), small lymphocytic (SL) NHL, intermediate grade/follicular NHL, intermediate grade diffuse NHL, high grade immunoblastic NHL, high grade lymphoblastic NHL, high grade small non-cleaved cell NHL, bulky disease NHL, or Waldenstrom's macroglobulinemia, and is more preferably non-Hodgkin's lymphoma (NHL).

3. The use according to claim 1, wherein the B cell malignancy is a B cell leukemia, and is preferably ALL-L3 (Burkitt's type leukemia), chronic lymphocytic leukemia (CLL), or a monocytic cell leukemia.

4. The use according to claim 1, wherein the anti-CD80 antibody is a human, humanized, or chimeric antibody, and wherein the antibody preferably comprises constant regions derived from human $IgG_1$ or $IgG_3$ constant regions.

5. The use according to claim 1, wherein the anti-CD80 antibody is IDEC-114.

6. The use according to claim 1, wherein the anti-CD80 antibody induces antibody-dependent cellular cytotoxicity (ADCC) and/or complement-dependent cytotoxicity (CDC) of B lymphoma cells.

7. The use according to claim 1, wherein the anti-CD80 antibody is conjugated to a cytotoxin, and wherein the cytotoxin is preferably a radionuclide, a cytostatic agent, a biotoxin, or a prodrug, and is more preferably a radionuclide, and even more preferably $^{131}I$ or $^{90}Y$.

8. The use according to claim 1, wherein the medicament is formulated for administration to the human subject parenterally, subcutaneously, intraperitoneally, intrapulmonary, intranasally, intralesionally, intraarterially, intravenously, intramuscularly, rectally, vaginally, topically, via inhalation, or orally.

9. The use according to claim 1, wherein the anti-CD80 antibody is to be administered at a dosage of from about 0.05 mg/kg body weight to about 100 mg/kg body weight, and more preferably at a dosage of from about 0.5 mg/kg body weight to about 10 mg/kg body weight.

10. Use of an anti-CD80 antibody for the manufacture of a medicament for simultaneous or sequential use in combination with one or more chemotherapeutic agents for enhancing antibody-dependent cellular cytotoxicity (ADCC) or complement-dependent cytotoxicity (CDC) against CD80-positive B cells for the treatment of a neoplastic disorder.

11. The use according to claim 10, wherein the CD80-positive B cells are lymphoma or leukemia cells.

12. The use according to claim 10, wherein the anti-CD80 antibody is a human, humanized, or chimeric antibody.

13. The use according to claim 10, wherein the anti-CD80 antibody is IDEC-114.

14. The use according to any one of the preceding claims, wherein the one or more chemotherapeutic agents are selected from the group consisting of alkylating substances, mechlorethamine, triethylenephosphoramide, cyclophosphamide, ifosfamide, chlorambucil, busulfan, melphalan, triaziquone, nitrosourea compounds, carmustine, lomustine, semustine, anthracyclins, vinca drugs, mitomycins, bleomycins, cytotoxic nucleosides, pteridines, diynenes, podophyllotoxins, adriamycin, carminomycin, danuorubicin, doxorubicin, aminopterin, methotrexate, methopterin, mithramycin, streptonigrin, dichloromethotrexate, mitomycin C, actinomycin-D, porfiromycin, 5-fluorouracil, floxuridine, ftorafur, 6-mercaptopurine, cytarabine, cytosine arabinoside, podophyllotoxin, etoposide, etoposide phosphate, vinblastine, vincristine, leurosidine, vindesine, and leurosine.

Sensitivity of B-Lymphoma cells to adriamycin after 4 hour exposure

FIGURE 1

Anti-CD40L antibody (IDEC-131) overrides CD40L mediated
resistance to killing by ADM of B-lymphoma cells

FIGURE 2.a

Effect of RITUXAN on normal and sCD40L pre-treated DHL-4 cells

FIGURE 2.b.

## Blocking of CD40L mediated cell survival of B-CLL by anti-CD40L antibody (IDEC-131)

**FIGURE 3.a**

Blocking of CD40L mediated survival of B-CLL
by IDEC's C2B8

FIGURE 3.b

# FIGURE 4

FIG. 5

FIG. 6

FIG. 8a

FIG. 8b

FIG. 8c

FIG. 9a

FIG. 9b

FIG. 10

Europäisches
Patentamt

European
Patent Office

Office européen
des brevets

## EUROPEAN SEARCH REPORT

Application Number

EP 10 18 2916

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,P | WO 01/89567 A1 (IDEC PHARMA CORP [US]; ANDERSON DARRELL R [US]; HANNA NABIL [US]; BRAM) 29 November 2001 (2001-11-29) | 1-6,8-14 | INV.<br>A61K45/00<br>A61K45/06 |
| Y,P | * page 11, line 16 *<br>* page 14, line 30 *<br>* page 28, lines 19,20 *<br>* page 29 - page 31 *<br>* page 35, lines 13-27 *<br>* claims 1,7,8,20-22 * | 1-14 | A61K39/395<br>A61P35/00<br>A61P35/02 |
| Y | BOLOGNESI A ET AL: "In vitro anti-tumor activity of anti-CD80 and anti-CD86 immunotoxins containing type 1 ribosome-inactivating proteins",<br>BRITISH JOURNAL OF HAEMATOLOGY,<br>vol. 110, 2000, pages 351-361,<br>XP002626473,<br>* page 351, column 1 *<br>* page 352, columns 2,8 *<br>* page 353, columns 1,5 *<br>* figure 1 *<br>* page 356, column 2 *<br>* page 357, columns 2,4 *<br>* page 358, column 2 *<br>* page 359, column 2 *<br>* page 360, column 6 * | 1-14 | |
| Y,P | HARIHARAN K ET AL: "THERAPEUTIC ACTIVITY OF IDEC-114 (ANTI.CD80) AND RITUXIMAB (RITUXAN) IN B-CELL LYMPHOMA",<br>BLOOD, AMERICAN SOCIETY OF HEMATOLOGY, US,<br>vol. 98, no. 11, PART 01,<br>16 November 2001 (2001-11-16), page 608A,<br>XP001079527,<br>ISSN: 0006-4971<br>* abstract *<br>-/-- | 1-14 | TECHNICAL FIELDS SEARCHED (IPC)<br><br>A61K<br>A61P |

The present search report has been drawn up for all claims

| Place of search | Date of completion of the search | Examiner |
|---|---|---|
| Munich | 10 March 2011 | Weisser, Dagmar |

EPO FORM 1503 03.82 (P04C01)

Europäisches
Patentamt

European
Patent Office

Office européen
des brevets

## EUROPEAN SEARCH REPORT

Application Number

EP 10 18 2916

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | CZUCZMAN M S ET AL: "TREATMENT OF PATIENTS WITH LOW-GRADE B-CELL LYMPHOMA WITH THE COMBINATION OF CHIMERIC ANTI-CD20 MONOCLONAL ANTIBODY AND CHOP CHEMOTHERAPY", JOURNAL OF CLINICAL ONCOLOGY, AMERICAN SOCIETY OF CLINICAL ONCOLOGY, US, vol. 17, no. 1, 1 January 1999 (1999-01-01), pages 268-276, XP000952705, ISSN: 0732-183X * page 269, columns 2,4 * * figure 1 * * page 272, column 6 * * page 274, column 4 * * page 275, column 3 * ----- | 1-14 | |
| Y | WO 00/27428 A1 (IDEC PHARMA CORP [US]; GRILLO LOPEZ ANTONIO J [US]; WHITE CHRISTINE A) 18 May 2000 (2000-05-18) * abstract * * page 2, lines 9,20-24 * * page 4, lines 3-10 * * page 6, lines 13,14 * * page 14, lines 6-13 * -----<br><br>-/-- | 1-14 | TECHNICAL FIELDS SEARCHED (IPC) |

The present search report has been drawn up for all claims

| Place of search | Date of completion of the search | Examiner |
|---|---|---|
| Munich | 10 March 2011 | Weisser, Dagmar |

EPO FORM 1503 03.82 (P04C01)

Europäisches
Patentamt

European
Patent Office

Office européen
des brevets

# EUROPEAN SEARCH REPORT

Application Number

EP 10 18 2916

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | COIFFIER B ET AL: "Rituximab (anti-CD20 monoclonal antibody) for the treatment of patients with relapsing or refractory aggressive lymphoma: A multicenter phase II study", BLOOD, AMERICAN SOCIETY OF HEMATOLOGY, US, vol. 92, no. 6, 15 September 1998 (1998-09-15), pages 1927-1932, XP002216671, ISSN: 0006-4971 * page 1927, column 1 * * page 1929, column 4 * * page 1931, column 3 * * page 1932, columns 4,5 * ----- | 1-14 | |
| Y | BUSKE C ET AL: "MONOCLONAL ANTIBODY THERAPY FOR B CELL NON-HODGKIN'S LYMPHOMAS: EMERGING CONCEPTS OF A TUMOUR-TARGETED STRATEGY", EUROPEAN JOURNAL OF CANCER, PERGAMON PRESS, OXFORD, GB, vol. 35, no. 4, 1 January 1999 (1999-01-01), pages 549-557, XP001010018, ISSN: 0959-8049, DOI: DOI:10.1016/S0959-8049(98)00420-1 * page 549, column 2 * * page 550, columns 4,7; figure 1 * * page 551, columns 2,4,5 * * page 552, column 3; table 2 * * page 553, column 3 - page 554 * ----- | 1-14 | TECHNICAL FIELDS SEARCHED (IPC) |

-/--

The present search report has been drawn up for all claims

| Place of search | Date of completion of the search | Examiner |
|---|---|---|
| Munich | 10 March 2011 | Weisser, Dagmar |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number

EP 10 18 2916

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | DELABIE JAN ET AL: "The B7/BB1 antigen is expressed by Reed-Sternberg cells of Hodgkin's disease and contributes to the stimulating capacity of Hodgkin's disease-derived cell lines", BLOOD, AMERICAN SOCIETY OF HEMATOLOGY, US, vol. 82, no. 9, 1 January 1993 (1993-01-01), pages 2845-2852, XP002489909, ISSN: 0006-4971 * page 2845, line 4 * * page 2846, line 2; figure 1 * * page 2848, column 4 * * page 2849, column 3 * * page 2851, column 3 * ----- | 1-14 | |

TECHNICAL FIELDS SEARCHED (IPC)

The present search report has been drawn up for all claims

| Place of search | Date of completion of the search | Examiner |
|---|---|---|
| Munich | 10 March 2011 | Weisser, Dagmar |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EP 2 314 318 A1

## ANNEX TO THE EUROPEAN SEARCH REPORT
## ON EUROPEAN PATENT APPLICATION NO.

EP 10 18 2916

10-03-2011

| Patent document cited in search report | | Publication date | Patent family member(s) | | Publication date |
|---|---|---|---|---|---|
| WO 0189567 | A1 | 29-11-2001 | AU | 6474701 A | 03-12-2001 |
| WO 0027428 | A1 | 18-05-2000 | AT | 479443 T | 15-09-2010 |
| | | | AU | 761844 B2 | 12-06-2003 |
| | | | BR | 9915164 A | 07-01-2003 |
| | | | CA | 2350058 A1 | 18-05-2000 |
| | | | CN | 1356909 A | 03-07-2002 |
| | | | CN | 1679934 A | 12-10-2005 |
| | | | DK | 1616572 T3 | 06-12-2010 |
| | | | EP | 1616572 A1 | 18-01-2006 |
| | | | EP | 1949910 A1 | 30-07-2008 |
| | | | EP | 1949911 A2 | 30-07-2008 |
| | | | EP | 1949912 A2 | 30-07-2008 |
| | | | EP | 2055313 A1 | 06-05-2009 |
| | | | EP | 2289543 A1 | 02-03-2011 |
| | | | EP | 1131093 A1 | 12-09-2001 |
| | | | ES | 2351149 T3 | 01-02-2011 |
| | | | HK | 1047043 A1 | 02-09-2005 |
| | | | JP | 2002529426 T | 10-09-2002 |
| | | | JP | 2010285439 A | 24-12-2010 |
| | | | MX | PA01004648 A | 06-05-2002 |
| | | | PT | 1616572 E | 11-11-2010 |
| | | | TR | 200101299 T2 | 21-01-2002 |
| | | | US | 7682612 B1 | 23-03-2010 |
| | | | US | 2010080769 A1 | 01-04-2010 |
| | | | ZA | 200103720 A | 08-08-2002 |

EPO FORM P0459

## REFERENCES CITED IN THE DESCRIPTION

### Patent documents cited in the description

- US 09772938 B
- US 09855717 B
- US 09985646 B
- US 33118701 P
- US 5843398 A
- US 5843439 A
- US 5776456 A
- US 5736137 A
- US 6113198 A
- US 5874082 A
- US 5667165 A
- US 5674492 A
- US 1997 A
- US 5874085 A
- US 5801227 A
- US 5945513 A
- US 1999 A
- EP 555880 A
- EP 1993 A
- US 57474037 B
- US 1998 A
- US 5876718 A
- US 5484892 A, Tedder
- US 5789557 A, Leung
- US 5789554 A, Leung
- WO 9842378 A
- WO 0020864 A
- WO 9841641 A
- US 5443953 A, Hansen
- US 5686072 A, Uhr
- US 6113898 A
- US 6001358 A
- US 6011138 A
- EP 404097 A
- WO 9311161 A
- US PN6001358 A
- US 5500362 A
- US 5821337 A
- US 5885577 A, Linsley
- US 5869050 A, DeBoer
- US 6011358 A
- US 5674442 A
- US 5736B7 A
- US 5595721 A
- US 5677180 A
- US 4665077 A
- US 4120649 A
- WO 9008187 A
- US 5114721 A, Cohen
- WO 9011294 A
- WO 9101133 A
- EP 340109 A
- US 4831175 A
- US 5099069 A
- US 5246692 A
- US 5286850 A
- US 5434287 A
- US 5124471 A
- US 08475813 B
- US 08475815 B
- US 08478967 B
- US 5460785 A
- WO 9835036 A
- US 4816567 A
- WO 9506666 A
- US 37907295 A
- US 07912292 B
- US 07856281 B
- US 07735064 B
- US 08379072 B
- US 48755095 A
- US 5750105 A
- US 379072 A, Newman
- US 08149099 B
- US 5591669 A
- US 5589369 A
- US 5545807 A
- US 5565332 A
- US 5573905 A
- US 205567610 B
- US 5229275 A
- WO 9316185 A
- US 5571894 A
- US 5587458 A
- US 5641870 A
- WO 9308829 A
- WO 9404690 A
- US 5731168 A
- US 4676980 A
- WO 9100360 A
- WO 92200373 A
- EP 03089 A
- US 4485045 A
- US 4544545 A
- WO 9738731 A
- US 5013556 A
- US 5739277 A
- WO 9856418 A

- WO 9704801 A
- US 3773919 A
- WO 9607321 A
- US 4892538 A

- US 5283187 A
- WO 9325673 A
- US SN09259337 A
- US SN09259338 A

**Non-patent literature cited in the description**

- **PRESR et al.** *J. Clin. Oncol.,* August 1989, vol. 7 (8), 1027-1038
- **GROSSBARD et al.** *Blood,* 15 August 1992, vol. 8 (4), 863-876
- **MCLAUGHLIN et al.** *J. Clan. Oncol.,* 1998, vol. 16, 2825-2833
- **CLARK et al.** *Adv. Cancer Res.,* 1989, vol. 52, 81-149
- **TEDDER et al.** *Immunology Today,* 1994, vol. 15, 450-454
- **ENDO.** *Gan To Kagaku Ryoho,* 1999, vol. 26, 744-748
- **PRESS et al.** *Blood,* 1987, vol. 69, 584-591
- **REFF et al.** *Blood,* 1994, vol. 83, 435-445
- **DEMIDEM et al.** *Cancer Biotherapy & Radiopharmaceuticals,* 1997, vol. 12, 177
- **MCLAUGHLIN et al.** *Oncology (Huntingt),* 1998, vol. 12, 1763-1777
- **MALONEY et al.** *Oncology,* 1998, vol. 12, 63-76
- **LEGET et al.** *Curr. Opin. Oncol.,* 1998, vol. 10, 548-551
- **WHITE et al.** *Pharm. Sci. Technol. Today,* 1999, vol. 2, 95-101
- **NGUYEN et al.** *Eur. J. Haematol,* 1999, vol. 62, 76-82
- **OHNISHI.** *Gan To Kagaku Ryoho,* 1998, vol. 25, 2223-8
- **BERINSTEIN et al.** *Ann. Oncol.,* 1998, vol. 9, 995-1001
- **PIRO.** *Ann. Oncol.,* 1999, vol. 10, 655-61
- **COIFFIER et al.** *Blood,* 1998, vol. 92, 1927-1932
- **MALONEY et al.** *Blood,* 1997, vol. 90, 2188-2195
- **CZUCZMAN et al.** *J. Clin. Oncol.,* 1999, vol. 17, 268-76
- **VALLE.** *Eur. J.Immunol.,* 1989, vol. 19, 1463-1467
- **GRUSS.** *Leuk. Lymphoma,* 1997, vol. 24, 393-422
- **JOHNSON et al.** *Blood,* 1993, vol. 82, 1848-1857
- **METKAR.** *Cancer Immunol. Immunother.,* 1998, vol. 47, 104
- **WANG.** *J. Immunology,* 1995, vol. 155, 3722-3725
- **CLODI.** *Brit. J. Haematol.,* 1998, vol. 103, 217-219

- **FUNAKOSHI.** *Blood,* 1994, vol. 83, 2787-2794
- **MURPHY.** *Blood,* 1995, vol. 86, 1946-1953
- **TUTT.** *J. Immunol.,* 1998, vol. 161, 3176-3185
- **LEDBETTER et al.** *Circ. Shock,* 1994, vol. 44, 67-72
- **SPIGGS et al.** *Exp. Med,* 1992, vol. 176, 1543-1550
- **LANE.** *Eur. J. Immunol.,* 1992, vol. 22, 2573-2578
- **ROY et al.** *J. Immunol.,* 1993, vol. 151, 1-14
- **STAMENKOVI.** *EMBO J.,* 1989, vol. 8, 1403-1410
- **ARMITAGE.** *Nature,* 1992, vol. 357, 80-82
- **LEDERMAN.** *J. Exp. Med,* 1992, vol. 175, 1091-1101
- **HOLLENBAUGH.** *EMBO J.,* 1992, vol. 11, 4313-4321
- **CARBONE.** *Am. J. Pathol.,* 1995, vol. 147, 912-922
- **GREEN.** *Virology,* 1998, vol. 241, 260-268
- **RIBEIRO et al.** *British J. Haematol.,* 1998, vol. 103, 684-689
- **LAYTRAGOON-LEWIN.** *Eur. J Haematol.,* 1998, vol. 61, 266-271
- **WORIN et al.** *International Immunol.,* 1994, vol. 6, 1883-1890
- **PLUMAS et al.** *Blood,* 1998, vol. 91, 2875-2885
- **BENOIT.** *Immunopharmacology,* 1996, vol. 35, 129-139
- **FUNAKOSHI.** *J.Immunother. Emphasis Tumor Immunol.,* 1996, vol. 19, 93-101
- **STAMENKOVIC et al.** *Nature,* 1990, vol. 344, 74
- **WILSON et al.** *J. Exp. Med.,* 1991, vol. 173, 137
- **STAMENKOVIC et al.** *Cell,* 1991, vol. 66, 1133
- **DORKEN et al.** *J. Immunol.,* 1986, vol. 136, 4470
- Expression of cytoplasmic CD22 in B-cell ontogeny. In Leukocyte Typing III. **DORKEN et al.** White Cell Differentiation Antigens. Oxford University Press, 1987, 474
- **SCHWARTING et al.** *Blood,* 1985, vol. 65, 974
- **MASON et al.** *Blood,* 1987, vol. 69, 836

- B-cell and plasma antigens: new and previously defined clusters. **LING et al.** Leukocyte Typing III. White Cell Differentiation Antigens. Oxford University Press, 1987, 302
- **WILSON et al.** *J. Exp. Med,* 1991, vol. 173, 137
- **PEZZUTTO et al.** *J. Immunol.,* 1987, vol. 138, 98
- **PEZZUTTO et al.** *J. Immunol,* 1988, vol. 140, 1791
- **BOUE et al.** *J. Immunol.,* 1988, vol. 140, 192
- **CAMPANA, D. et al.** *J. Immunol.,* 1985, vol. 134, 1524
- **DORKEN et al.** *J. Immunol.,* 1993, vol. 150, 4719
- **ENGEL et al.** *J. Immunol.,* 1993, vol. 150, 4519
- **KABAT.** Sequences of Proteins of Immunologicαl Interest. Public Health Service, National Institutes of Health, 1991
- **PLUCKTHUN.** The Phαrmαcology of Monoclonαl Antibodies. Springer-Verlag, 1994, vol. 113, 269-315
- **HOLLINGER.** *Proc. Natl. Acad Sci. USA,* 1993, vol. 90, 6444-6448
- **MORRISON.** *Proc. Natl. Acαd Sci.,* 1984, vol. 81, 6851-5
- **MORRISON.** *Adv. Immunol.,* 1988, vol. 44, 65-92
- **VERHOEYEN et al.** *Science,* 1988, vol. 239, 1534-1536
- **PADLAN.** *Molec. Immun.,* 1991, vol. 28, 489-498
- **PADLAN.** *Molec. Immun.,* 1994, vol. 31, 169-217
- **NEWMAN.** *Biotechnology,* 1992, vol. 10, 1458-1460
- **CLARK.** *PNAS,* 1985, vol. 82, 1766
- **KIESEL.** *Leukemiα Reseαrch,* 1987, vol. 11 (12), 1119
- **NITSCHKE.** *Curr. Biol.,* 1997, vol. 7, 133
- **STAMENKOVIC.** *Nαture,* 1990, vol. 345, 74
- **ENGEL.** *J. Etyp. Med,* 1995, vol. 181 (4), 1521-1586
- **RAVETCH ; KINET.** *Annu, Rev. Immunol,* 1991, vol. 9, 457-92
- **CLYNES.** *PNAS,* 1998, vol. 95, 652-656
- **DAEON.** *Annu. Rev. Immunol.,* 1997, vol. 15, 203-234
- **RAVETCH ; KINET.** *Annu. Rev. Immunol.,* 1991, vol. 9, 457-92
- **CAPEL et al.** *Immunomethods,* 1994, vol. 4, 25-34
- **DE HAAS.** *J. Lαb. Clin. Med.,* 1995, vol. 126, 330-41
- **GUYER.** *J. Immunol.,* 1976, vol. 117, 587
- **KIM et al.** *J. Immunol.,* 1994, vol. 24, 249
- **GAZZANO-SANTORO et al.** *J. Immunol. Methods,* 1996, vol. 202, 163
- **CHOTHIA ; LESK.** *1. Mol. Biol.,* 1987, vol. 196, 901-917
- **PRESS.** *Blood,* 1987, vol. 69 (2), 584-591
- **RECTOR.** *J. Immunol.,* 1985, vol. 55, 481-488
- **FLORES-RUMEO.** *Science,* 1993, vol. 241, 1038-1046
- **SHERR.** *J. Immunol.,* 1989, vol. 142, 481-489
- **PENE.** *PNAS,* 1988, vol. 85, 6820-6824
- Lymphomas. **GAIDONO et al.** CANCER: PRINCIPLES & PRACTICE OF ONCOLOGY. 1997, vol. 2, 2131-2145
- **OFFNER et al.** *Science,* 1991, vol. 251, 430-432
- **LANEWAY.** *Nature,* 1989, vol. 341, 482
- **MURRAY.** *J. Nut. Med.,* 1985, vol. 26, 3328
- **CARRAGUILLO et al.** *J Nuc. Med.,* 1985, vol. 26, 67
- **PEIERERSZ et al.** *Immunol. Cell Biol.,* 1987, vol. 65, 111-125
- **WILMAN.** Prodrugs in Cancer Chemotherapy. *Biochemical Society Transactions,* 1986, vol. 14, 375-382
- Prodrugs: A Chemical Approach to Targeted Drug Delivery. **STELLA et al.** Directed Drug Delivery. Humana Press, 1985, 247-267
- **KOHLER et al.** *Nature,* 1975, vol. 256, 495
- **GODING.** Monoclonal Antibodies: Principles and Practice. Academic Press, 1986, 59-103
- **KOZBOR.** *J. ImmunoL,* 1984, vol. 133, 300-1
- **BRODEUR et al.** Monoclonal Antibody Production Techniques and Applications. Marcel Dekker, Inc, 1987, 51-63
- **MUNSON et al.** Anal. Biochem. 1980, vol. 107, 220
- **SKERRA et al.** *Curr. Opinion in Immunol.,* 1993, vol. 5, 256-262
- **PLUCKTHUN.** *Immunol. Revs.,* 1992, vol. 130, 151-188
- **WARD et al.** *Nature,* 1989, vol. 341, 544-546
- **HUSE et al.** *Science,* 1989, vol. 246, 1275-1281
- **MCCAFFERTY et al.** *Nature,* 1990, vol. 348, 552-554
- **CLACKSON et al.** *Nature,* 1991, vol. 352, 624-628
- **MARKS.** *J. Mol. Biol.,* 1991, vol. 222, 581-597
- **MARKS et al.** *Bio/Technology,* 1992, vol. 10, 779-783
- **WATERHOUSE et al.** *Nut. Acids. Res.,* 1993, vol. 21, 2265-2266
- **MORRISON et al.** *Proc. Natl Acad. ScL USA,* 1984, vol. 81, 6851
- **JONES et al.** *Nature,* 1986, vol. 321, 522-525
- **REICHMANN et al.** *Nature,* 1988, vol. 332, 323-327

- **SUNS et al.** *J Immunol.,* 1993, vol. 151, 2296
- **CHOTHIA et al.** *J. Mol. Biol,* 1987, vol. 196, 901
- **CARTER et al.** *Proc. Natl. Acad Sci. USA,* 1992, vol. 89, 4285
- **PRESTA et al.** *J. Immunol.,* 1993, vol. 151, 2623
- **NEWMAN.** *Biotechnology,* 1992, vol. 10, 1455-1460
- **JAKOBOVITS et al.** *Proc. Mad. Acad. Ski. USA,* 1993, vol. 90, 255-1
- **JAKOBOVITS et al.** *Nature,* 1993, vol. 362, 255-258
- **BRUGGERMANN et al.** *Year in immuno.,* 1993, vol. 7, 33
- **MCCAFFERTY et al.** *Nature,* 1990, vol. 348, 552-553
- **JOHNSON, KEVIN S. ; CHISWELL, DAVID J.** *Current Opinion in Structural Biology,* 1993, vol. 3, 564-57 1
- **MARKS et al.** *J.Mol. Biol,* 1991, vol. 222, 581-597
- **GRIFFITH et al.** *EMBO J.,* 1993, vol. 12, 725-734
- **MORIMOTO et al.** *Journal of Biochemical and Biophysical Methods,* 1992, vol. 24, 107-117
- **BRENNAN et al.** *Science,* 1985, vol. 229, 81
- **CARTER et al.** *BiolTechnology,* 1992, vol. 10, 163-167
- **MILLSTEIN et al.** *Nature,* 1983, vol. 305, 537-539
- **TRAUNECKER et al.** *EMBO J.,* 1991, vol. 10, 3655-3659
- **SURESH et al.** *Methods in Enzymology,* 1986, vol. 121, 210
- **SHALABY et al.** *J.Exp. Med,* 1992, vol. 175, 2 17-225
- **KOSTELNY et al.** *J. Immunol.,* 1992, vol. 148 (5), 1547-1553
- **HOLLINGER et al.** *Proc.Natl. Acad. Sci. USA,* 1993, vol. 90, 6444-6448
- **GRUBER et al.** *J Immunol.,* 1994, vol. 152, 5368
- **TUTT et al.** *J. Immunol.,* 1991, vol. 147, 60
- **EPSTEIN et al.** *Proc. Natl. Acad. Sci. USA,* 1985, vol. 82, 3688
- **HWANG et al.** *Proc. Natl Acad Sci. USA,* 1980, vol. 77, 4030
- **MARTIN et al.** *J. Biol. Chem.,* 1982, vol. 257, 286-288
- **GABIZON et al.** *J.National Cancer Inst.,* 1989, vol. 81 (19), 1484
- **CUNNINGHAM ; WELLS.** *Science,* 1989, vol. 244, 1081-1085
- **CARON et al.** *J Exp Med.,* 1992, vol. 176, 1191-1195
- **SHOPES.** *B. J. Immunol.,* 1992, vol. 148, 2918-2922
- **WOLFF et al.** *Cancer Research,* 1993, vol. 53, 2560-2565
- **STEVENSON et al.** *Anti-Cancer Drug Design,* 1989, vol. 3, 2 19-230
- Remington's Pharmaceutical Sciences. 1980
- **WU et al.** *.l. Biol. Chem,* 1987, vol. 262, 4429-4432
- **WAGNER et al.** *Pro. Natl. Acad. Sci. USA,* 1990, vol. 87, 3410-3414
- **ANDERSON et al.** *Science,* 1992, vol. 256, 808-8 13
- **ROOS et al.** *Leuk Res.,* 1986, vol. 10, 195-202
- **GAZZANO-SANTORO et al.** *J. Immunol. Meth.,* 1997, vol. 202, 163-171
- **ANDERSON et al.** *Clin. Immunol. & Immunopathol.,* 1997, vol. 84, 73-84
- **REFT ME ; CAMER K ; CHAMBERS KS ; CHINN PC ; LEONARD JE ; RAAB R et al.** Depletion of B cells in vivo by a chimeric mouse human monoclonal antibody to CD20. *Blood,* 1994, vol. 83 (2), 435-45